# EUROPÄISCHE PATENTANMELDUNG

(11) **EP 2 468 882 A1**
(43) Veröffentlichungstag der Anmeldung: **27.06.2012**
(21) Anmeldenummer: 10197084.6
(22) Anmeldetag: 27.12.2010
(51) Int. Cl.: C12Q 1/18, G01N 33/569

(54) **Aurorakinaseaktivatorpolypeptide zum Identifizieren von fungizid wirksamen Verbindungen**

(71) Anmelder: Bayer CropScience AG, 40789 Monheim (DE); Max-Planck-Gesellschaft zur Förderung der Wissenschaften e.V., 80539 München (DE)
(72) Erfinder: Braun, Christoph Andreas, 40627 Düsseldorf (DE); Schreier, Peter, 50674 Köln (DE); Tietjen, Klaus, 40764 Langenfeld (DE); Beck, Michael Edmund, 40789 Monheim (DE); Mattes, Amos, 40674 Langenfeld (DE); Greul, Jörg, 51381 Leverkusen (DE); Gutbrod, Oliver, 40764 Langenfeld (DE); Rauh, Daniel, 67549 Worms (DE); Tückmantel, Sandra, 51379 Leverkusen (DE)
(74) Vertreter: Vossius & Partner

(57) **Zusammenfassung**

Die Erfindung bezieht sich auf eine Kombination aus Polypeptiden aus pflanzenpathogenen Pilzen mit der biologischen Aktivität einer Aurorakinase und der Funktion eines Aurorakinaseaktivators, dafür kodierende Nukleinsäuren, die Verwendung der Polypeptide und Nukleinsäuren zum Identifizieren von Modulatoren einer Aurorakinase, Verfahren zum Identifizieren solcher Modulatoren und die Verwendung dieser Modulatoren als Fungizide.

SEQUENCE LISTING

<110>  Bayer Cropscience

<120>  Polypeptide zum Identifizieren von fungizid wirksamen
       Verbindungen

<130>  BCS10-3121

<160>  16

<170>  PatentIn version 3.5

<210>  1
<211>  1428
<212>  DNA
<213>  Ustilago maydis

<220>
<223>  Aurorakinase

<400>  1
atggagtcgc agctcgccaa cttgcacctg gatggcggct cgacggcaga tctcaagcaa      60

ggatccagac cgtcacatgc acaggcatcg cacggaaaca gacatgttca tcctacaaac     120

tcgtcactcc gacctctggc gccttccagc tcaattgcgt ccaacatggg tatcaatatg     180

ggtgtcaaca tgcagcactt caaccgtcta gctgcctcct caacggggct cacgaacccc     240

aacagcattg cacaaggcat gagccgagct gcgcagcagc cgctcaacgg atcgagacct     300

agcacacaac aacagcctac gcaggcacat atcaagagtc gacctcctag cgttgctggc     360

ggactgaatg ctgcggctgg tccttcgtat cgtccagcgg cgcctgctgc tcaatctacc     420

aaacaaccag ctagtgctgg atcagccgta gcatcggttg atttgggacg atatgacggt     480

ggtctggaac gcgatgaagc acgcggacgt cgaggaacga tgcaattcga tccgctcacg     540

ctttcctctg ccgatgctgg gaaacaacat ccaccaaccc gcgtgtggtc gctcaaagac     600

ttcgaaatgg gtcgacctct tggcaaaggc aaattcggtc gcgtgtacat ggttcgcact     660

cgcgctgcac ccaacaaagg ctacattatc gccctcaagt gcatgtacaa aaacgagcta     720

gtcgagaaca aggtggaaaa acaacttcga agagagatcg agatccagat gaacctccgc     780

catcctcata tccttcgact tcacggatac ttccacgatg agggccgagt cttcctcatg     840

atcgaatttg ctggtcgtgg ggagctctac aaattgatga acaaactaca cgatcgaagg     900

ttcgaagaaa aggttgcagc aacctacatc gcgcagatgg cagacgctct atcatacctg     960

cacagcaagc acgtcattca tcgcgacatc aaaccagaaa accttctatt gggtatcaaa    1020

ggcgatctca agattggtga ctttggatgg agcgttcacg cccctggaaa ccgtcgtcag    1080

actctgtgtg gtaccttgga ctacttgccc cccgagatgg taaatggaga gcaacacgat    1140

aaggcagtag acctgtgggc attgggcgtg ctgtgttacg aatttttgga aggtgttcct    1200

ccgtttgaag agctggaaaa cgctcccgca ggcacgtaca agagaatcaa caacatcgac    1260

ttcaagatcc ctcgacattt cagccccgaa gctgccgatc tggtaaaggc actgctaaag    1320

aagaaaccag acgacaggct acccttgacc aaagtgctga ggcatccgtg gatcatgaag    1380

tacgatccag atgcttgtcg tcgtgcgtct cgtggcaaaa ttcagtaa                 1428

<210>  2
<211>  475
<212>  PRT
<213>  Ustilago maydis

<220>
<223>  Aurorakinase

<400>  2

Met Glu Ser Gln Leu Ala Asn Leu His Leu Asp Gly Gly Ser Thr Ala
1               5                   10                  15

Asp Leu Lys Gln Gly Ser Arg Pro Ser His Ala Gln Ala Ser His Gly
            20                  25                  30

Asn Arg His Val His Pro Thr Asn Ser Ser Leu Arg Pro Leu Ala Pro
        35                  40                  45

Ser Ser Ser Ile Ala Ser Asn Met Gly Ile Asn Met Gly Val Asn Met
    50                  55                  60

Gln His Phe Asn Arg Leu Ala Ala Ser Ser Thr Gly Leu Thr Asn Pro
65                  70                  75                  80

Asn Ser Ile Ala Gln Gly Met Ser Arg Ala Ala Gln Gln Pro Leu Asn
                85                  90                  95

Gly Ser Arg Pro Ser Thr Gln Gln Gln Pro Thr Gln Ala His Ile Lys
            100                 105                 110

Ser Arg Pro Pro Ser Val Ala Gly Gly Leu Asn Ala Ala Ala Gly Pro
        115                 120                 125

Ser Tyr Arg Pro Ala Ala Pro Ala Ala Gln Ser Thr Lys Gln Pro Ala
    130                 135                 140

Ser Ala Gly Ser Ala Val Ala Ser Val Asp Leu Gly Arg Tyr Asp Gly
145                 150                 155                 160

Gly Leu Glu Arg Asp Glu Ala Arg Gly Arg Arg Gly Thr Met Gln Phe
                165                 170                 175

Asp Pro Leu Thr Leu Ser Ser Ala Asp Ala Gly Lys Gln His Pro Pro
            180                 185                 190

Thr Arg Val Trp Ser Leu Lys Asp Phe Glu Met Gly Arg Pro Leu Gly
        195                 200                 205

Lys Gly Lys Phe Gly Arg Val Tyr Met Val Arg Thr Arg Ala Ala Pro
    210                 215                 220

Asn Lys Gly Tyr Ile Ile Ala Leu Lys Cys Met Tyr Lys Asn Glu Leu

225                 230                 235                 240

Val Glu Asn Lys Val Glu Lys Gln Leu Arg Arg Glu Ile Glu Ile Gln
                245                 250                 255

Met Asn Leu Arg His Pro His Ile Leu Arg Leu His Gly Tyr Phe His
            260                 265                 270

Asp Glu Gly Arg Val Phe Leu Met Ile Glu Phe Ala Gly Arg Gly Glu
        275                 280                 285

Leu Tyr Lys Leu Met Asn Lys Leu His Asp Arg Arg Phe Glu Glu Lys
    290                 295                 300

Val Ala Ala Thr Tyr Ile Ala Gln Met Ala Asp Ala Leu Ser Tyr Leu
305                 310                 315                 320

His Ser Lys His Val Ile His Arg Asp Ile Lys Pro Glu Asn Leu Leu
                325                 330                 335

Leu Gly Ile Lys Gly Asp Leu Lys Ile Gly Asp Phe Gly Trp Ser Val
            340                 345                 350

His Ala Pro Gly Asn Arg Arg Gln Thr Leu Cys Gly Thr Leu Asp Tyr
        355                 360                 365

Leu Pro Pro Glu Met Val Asn Gly Glu Gln His Asp Lys Ala Val Asp
    370                 375                 380

Leu Trp Ala Leu Gly Val Leu Cys Tyr Glu Phe Leu Glu Gly Val Pro
385                 390                 395                 400

Pro Phe Glu Glu Leu Glu Asn Ala Pro Ala Gly Thr Tyr Lys Arg Ile
                405                 410                 415

Asn Asn Ile Asp Phe Lys Ile Pro Arg His Phe Ser Pro Glu Ala Ala
            420                 425                 430

Asp Leu Val Lys Ala Leu Leu Lys Lys Lys Pro Asp Asp Arg Leu Pro
        435                 440                 445

Leu Thr Lys Val Leu Arg His Pro Trp Ile Met Lys Tyr Asp Pro Asp
    450                 455                 460

Ala Cys Arg Arg Ala Ser Arg Gly Lys Ile Gln
465                 470                 475

<210>  3
<211>  5193
<212>  DNA
<213>  Ustilago maydis

<220>

<223>  Aurorakinaseaktivator

<400>  3
atgctccgcg ttcggtttgg tgcctgtccg cttgcattca tcctgaccat catcactctc      60

cggatcacac ccccacctgt cggctcttcc agattccaat tgatgcactt tgtgctagta     120

ctcgcgcatc ttgccagcag acgattagat tcacgaccag agaaacgctg cctacacttg     180

catccgcctc aaatcggctc acgccatcca tcctggtatc cacgctcata cagcgactcg     240

gacaagctcc gaccaacctc gtccgccgat tcatctgtct cttttcttgg tagcaaatca     300

atcacactca gcagccatgc cgacagctcg aaccgcttct cgcaccgtgt cccggccttg     360

gactcctcgg caagcacctc ggctgcgagt agtgactcaa gctcaccact gaccccagaa     420

gagcgtctgc tcagccacgt caacaacatg cgcaggtgct gcgacatgtc tctcttccac     480

aagaacctcc aagagcaagg tatcgatttt attgcagata gccttttgca tgccaacgac     540

atctttgcca agtacgcaac aacgtcgcgc accgttgcct cgccgtccaa gctggctggc     600

gaactcctca agtctcccac ccgccctcgc acccgcgctc gtgatgcgcc agccgcatca     660

ggctatcctc gtggggcatt caaagtatcg attgatccct tcgtcgatgc ggccgacgtg     720

ttcgacaagg aaaatgcacc cttgcaaact agatcttcac gatcacgtac cgcttccact     780

gtcgagcctc agaaaggcaa gggcaaggct agagccaaag ctattcatgc tgtcgaaaga     840

gaagcatcgt cacctctttc tcgcccgacc cgtagagtgc ttggcgatgt caacacgcaa     900

aagaatgaga caaagcaacc cactccacag cccaagccca ccagcaaaga agcaacgctc     960

aagaacattg ccacagatga cgacagcatg gtcattgatt cttcgctcca agcgcacctc    1020

tcacccgatg ataccatgcc aagagcttcc acaccaccac cgcctcaagc gtcgcctccg    1080

agaatacttg cccttcacaa gccttccaag gccactcagt ccgcagcgtc actctcgggc    1140

gccatttcca tcactgattc ggacgaggac ggctcagacg gagattcgga cgccgatttg    1200

cacgaagaag agacaggagg gctaaacgat accgttcaac atgaagttgc cgagcctaca    1260

gtgacaggaa atgtcgtgtc tcggagctcc cgcagcgatg acaacacagc atccttccaa    1320

cctgacataa acgacacgat gccagccaac gacaacaccg tgtctggtac tttgattgat    1380

ccgaaactcg aaagtgcccg tcaagcagcc tctgtactgc ttacaagcaa aattcagcag    1440

cagaaggatg caaacgccat gaagccggtc aaacggacgc ttgcatcgta cggcgcaaaa    1500

aagagcctgc cagctgctgc caacaactcc ggtcccagca acgtaaccac cacgatcgtt    1560

cccacctcga cccccgctgg tcctggcttc cgttccagct tcctcaacaa gagtctgcgc    1620

aaacagattg ccgaccagga agctctcgaa agcggccaag actctgactc tgactcggac    1680

gacaacgaaa acgacacagg tcttgtcgcc ggcgccacct ttgctgctat gtacaaaaag    1740

gcagccgcca acacgcaagc caatactcgc gcaaatgctg taccagcgtc aaacagcacc    1800

actagctcca agaagcgcaa gagtgacgag gttcttccag ctgcgattga ggccagcttg    1860

agcgctgccg ctccaccgca caaggtgtcc aagctgggtg ccgcgatgat ggcggccaag    1920

acacccacgt ccgctcaacc atctactgcc aagaaagcac cagctgccgg ccctgcgtcc    1980

aaactggagc aattccgcaa caacctcgct catgttgctc gcagcaccgg aagcggtgcg 2040

agtgccagct cggcttctca gtcagcgtcg tccgtcacgt cgcccaccat ggaacaagca 2100

ccatccgcgc cgctgcccgt cgtcaagcct tcggatgcgg ccgtcgtgcc attcgctcaa 2160

gaagcgtcgg cactgccttc cattgaaagc ttgaatgctt cgcattcgaa atcctcggat 2220

tctggctccg actcgacgga aaaacttacg gcaggtacca agagtggcga gtcaaccatc 2280

aaggcttctg ccatcccacg atcgcccact cgctctcctc gcgccgccgc cacttcaccg 2340

atgcggcctc cgcctcgcac cggcagtgtg cgtaaatctc cacgcaaagt ggccgagcag 2400

atcaacacaa gcggacaaga gtcggccaga tcggcttcgc cggggcccaa ggtgaaagaa 2460

agtatagctc ttttccagtc gagcagaaac ggcggtctga ctgataagca cacacgtagc 2520

cctaccacgc agccccgaag tctcgcagca ctctttggca gtcccaagca agcgaccaaa 2580

ctaccgacgc tgtcggccac tgccggcagc agcaccaccc cggctcactc tccacccaag 2640

aactggcaag gaagtcgttt accgtttgca caaaccttcc agttgcaaca gcaagcgctg 2700

cgtgaggtgg acaagtcttt gccgcagaca ccggccgagg agacggccga cagccagcgt 2760

gtcgtctcgg cagcctcgac ggtcttgtcc acgtccaagg ctgaaacagc ggagagccaa 2820

ttcttcgacg cccgctcgag ggagaacagt gccgagccag ccgatgcgcc agctccagcg 2880

ccattggcgg cgatgaaagg cgcgacggca cctgggacca gtgcacctca agtggtcgcc 2940

gaaaaggagt gcgtcatgct tgctgaggca cccatcgttg ccaacaaggc atccagcatt 3000

gtgaagacta gctcgctttc accaaagaaa gtggcggcca aggcgcttgc gaaaactgga 3060

ccgacctcgc cgaccaaggc gagcagtctc cgagctgcag ccaacgccac gtcgccttcc 3120

aagatcaagg cgtcggcgcg cacagcacag cagcatggcg gaccgggact gggaccggga 3180

ccggcaccgt ctggatcgcc ttcgagggct tggggcattg gtgaaaagat caaagggttc 3240

ctcggattgc atgcctcgac gacgatccac ccatctacag cagttgctca gaccaaggta 3300

tcggcgtctg gcacattcca gccgcgtgcc aacgggtttg ccacaaacac ggctcatgcg 3360

agcgcttccg aacacaagac tgcggctaca gctacaccgg caccttgcat tcctggcgct 3420

ctgcttgcta gccctccgcc aacggccaag tctgcgcagg tcaactcaac accctcaagc 3480

ggtgctgctt cggcgacggc caacggaaag ctcaccagca ttgcgcgcgc cgaaatgctg 3540

cgcaagaagc aggcagagga ggaggagcgc aaggctaagg acaagcaaga gcgtcgaaga 3600

atgctgatgg ccaagaagga agagcgtgcc aaggcggtcg cggaggagga aatcgaaaag 3660

gagaaccgca aacgagcgcg tgagcagaac gaccgcctcg gccgttccgg catcgaagac 3720

gccagcatcg cttcgtctaa cggtgcaccg gctgcgattc ctgctgcgct cctgggcaat 3780

attgtcgtcg gtggcggtgg ctccgcaatt tctggcagtg ttcacagcgg ctcagctagc 3840

tcaaggacaa gcacagtcag caccgttagc agcggaccgc ccacgcgacc atcaagtgct 3900

ctcaatggaa gctatgctgg tgtcaagagt cgctacatgc agggagcggt ccagcagcag 3960

cagcagcagc cgccgccgca gcaacaaggc gaggaaagta gtaagaagcg tcgcgtcaca 4020

aatgacaagg aaggtatagc ggcaggcttg caagtgccat tgaagcagca gccggcgcct 4080

cagcaatcac aaggtgcacc tagcgctcgt cctggtgctt cggtggcgac gtcgcagatc 4140

ggtgcaccca agatggttcg cacagtcagc cagtcgtcga tacgatctgg tgctgcctca 4200

caagctgctg cccagcaaca gcaacagcaa cagcgcactc agccgccgcc gcagatcaag 4260

ctgggcatga agccgagtgc ggcgacgaca gcagcagcag gagcaggatt tggagcagcg 4320

ccacgcatag gcggagctgg tgctacagcc ggcaacggcg cggctactgc gtcaatgatg 4380

gcgaatgctg gcaaagctgt ttcgtcggcg caacccgcgg cagccaaggc tatggcgaat 4440

gccaactttt gctcttcgaa tccgttccag caagccaagc agcagcaact gcagctgcag 4500

ttgcaaaagc agaagcagct tcaaatgcag caacagcagc aacagcaaca gcaagttgca 4560

tccaacgcga tgcaggcgca ggcggctgct agtgcggcag cggtgcgaga attagagttg 4620

catcgtgcgg cgcaattgca aatgcagatg cagaatgcag ctggtggacg gtatcaagag 4680

atggacgacg atgttgcagg cgatggcgac gaggtgctgc cggagattgc atcggaatac 4740

tcggactcgg aagacgaaga gacaattcag aagcgagcgg cgatgccgtt gtggacgcag 4800

ggcgatatgc tggatgcggc gctgttggcg caatccacgg tggatgccga tgagatcttt 4860

ggtattccac atggtcctgt tgagctagaa aaaatcttgc ctggcgagcg cacagcgaac 4920

cggatccgac gagcgcgaac ttcgtcggcc aactggtccg ggcccgacgg cctcgcgcag 4980

tgggagatcg acagatacaa caaacgcatg ggcatcaaga gtgcaggtgt ccagttacat 5040

cgcgacccat ctcagccgtc tcggccagcc gctatgccat ccctctcgat ccaccgacac 5100

tcgatctcgc acaacccact cgctcccacc tcgtccacct cgtccactgc tccacacaca 5160

gccatcaacg acgacgctcg tacaaatgct tag 5193

<210> 4
<211> 1730
<212> PRT
<213> Ustilago maydis

<220>
<223> Aurorakinaseaktivator

<400> 4

Met Leu Arg Val Arg Phe Gly Ala Cys Pro Leu Ala Phe Ile Leu Thr
1               5                   10                  15

Ile Ile Thr Leu Arg Ile Thr Pro Pro Pro Val Gly Ser Ser Arg Phe
            20                  25                  30

Gln Leu Met His Phe Val Leu Val Leu Ala His Leu Ala Ser Arg Arg
        35                  40                  45

Leu Asp Ser Arg Pro Glu Lys Arg Cys Leu His Leu His Pro Pro Gln
    50                  55                  60

Ile Gly Ser Arg His Pro Ser Trp Tyr Pro Arg Ser Tyr Ser Asp Ser
65                  70                  75                  80

Asp Lys Leu Arg Pro Thr Ser Ser Ala Asp Ser Ser Val Ser Phe Leu
                85                  90                  95

Gly Ser Lys Ser Ile Thr Leu Ser Ser His Ala Asp Ser Ser Asn Arg
            100                 105                 110

Phe Ser His Arg Val Pro Ala Leu Asp Ser Ser Ala Ser Thr Ser Ala
        115                 120                 125

Ala Ser Ser Asp Ser Ser Ser Pro Leu Thr Pro Glu Glu Arg Leu Leu
    130                 135                 140

Ser His Val Asn Asn Met Arg Arg Cys Cys Asp Met Ser Leu Phe His
145                 150                 155                 160

Lys Asn Leu Gln Glu Gln Gly Ile Asp Phe Ile Ala Asp Ser Leu Leu
                165                 170                 175

His Ala Asn Asp Ile Phe Ala Lys Tyr Ala Thr Thr Ser Arg Thr Val
            180                 185                 190

Ala Ser Pro Ser Lys Leu Ala Gly Glu Leu Leu Lys Ser Pro Thr Arg
        195                 200                 205

Pro Arg Thr Arg Ala Arg Asp Ala Pro Ala Ala Ser Gly Tyr Pro Arg
    210                 215                 220

Gly Ala Phe Lys Val Ser Ile Asp Pro Phe Val Asp Ala Ala Asp Val
225                 230                 235                 240

Phe Asp Lys Glu Asn Ala Pro Leu Gln Thr Arg Ser Ser Arg Ser Arg
                245                 250                 255

Thr Ala Ser Thr Val Glu Pro Gln Lys Gly Lys Gly Lys Ala Arg Ala
            260                 265                 270

Lys Ala Ile His Ala Val Glu Arg Glu Ala Ser Ser Pro Leu Ser Arg
        275                 280                 285

Pro Thr Arg Arg Val Leu Gly Asp Val Asn Thr Gln Lys Asn Glu Thr
    290                 295                 300

Lys Gln Pro Thr Pro Gln Pro Lys Pro Thr Ser Lys Glu Ala Thr Leu
305                 310                 315                 320

Lys Asn Ile Ala Thr Asp Asp Asp Ser Met Val Ile Asp Ser Ser Leu
                325                 330                 335

Gln Ala His Leu Ser Pro Asp Asp Thr Met Pro Arg Ala Ser Thr Pro
            340                 345                 350

Pro Pro Pro Gln Ala Ser Pro Pro Arg Ile Leu Ala Leu His Lys Pro
        355                 360                 365

Ser Lys Ala Thr Gln Ser Ala Ala Ser Leu Ser Gly Ala Ile Ser Ile
    370                 375                 380

Thr Asp Ser Asp Glu Asp Gly Ser Asp Gly Asp Ser Asp Ala Asp Leu
385                 390                 395                 400

His Glu Glu Glu Thr Gly Gly Leu Asn Asp Thr Val Gln His Glu Val
                405                 410                 415

Ala Glu Pro Thr Val Thr Gly Asn Val Val Ser Arg Ser Ser Arg Ser
            420                 425                 430

Asp Asp Asn Thr Ala Ser Phe Gln Pro Asp Ile Asn Asp Thr Met Pro
        435                 440                 445

Ala Asn Asp Asn Thr Val Ser Gly Thr Leu Ile Asp Pro Lys Leu Glu
    450                 455                 460

Ser Ala Arg Gln Ala Ala Ser Val Leu Leu Thr Ser Lys Ile Gln Gln
465                 470                 475                 480

Gln Lys Asp Ala Asn Ala Met Lys Pro Val Lys Arg Thr Leu Ala Ser
                485                 490                 495

Tyr Gly Ala Lys Lys Ser Leu Pro Ala Ala Ala Asn Asn Ser Gly Pro
            500                 505                 510

Ser Asn Val Thr Thr Thr Ile Val Pro Thr Ser Thr Pro Ala Gly Pro
        515                 520                 525

Gly Phe Arg Ser Ser Phe Leu Asn Lys Ser Leu Arg Lys Gln Ile Ala
    530                 535                 540

Asp Gln Glu Ala Leu Glu Ser Gly Gln Asp Ser Asp Ser Asp Ser Asp
545                 550                 555                 560

Asp Asn Glu Asn Asp Thr Gly Leu Val Ala Gly Ala Thr Phe Ala Ala
                565                 570                 575

Met Tyr Lys Lys Ala Ala Ala Asn Thr Gln Ala Asn Thr Arg Ala Asn
            580                 585                 590

Ala Val Pro Ala Ser Asn Ser Thr Thr Ser Ser Lys Lys Arg Lys Ser
        595                 600                 605

Asp Glu Val Leu Pro Ala Ala Ile Glu Ala Ser Leu Ser Ala Ala Ala
    610                 615                 620

Pro Pro His Lys Val Ser Lys Leu Gly Ala Ala Met Met Ala Ala Lys

625                 630                 635                 640

Thr Pro Thr Ser Ala Gln Pro Ser Thr Ala Lys Lys Ala Pro Ala Ala
                645                 650                 655

Gly Pro Ala Ser Lys Leu Glu Gln Phe Arg Asn Asn Leu Ala His Val
            660                 665                 670

Ala Arg Ser Thr Gly Ser Gly Ala Ser Ala Ser Ser Ala Ser Gln Ser
        675                 680                 685

Ala Ser Ser Val Thr Ser Pro Thr Met Glu Gln Ala Pro Ser Ala Pro
    690                 695                 700

Leu Pro Val Val Lys Pro Ser Asp Ala Ala Val Val Pro Phe Ala Gln
705                 710                 715                 720

Glu Ala Ser Ala Leu Pro Ser Ile Glu Ser Leu Asn Ala Ser His Ser
                725                 730                 735

Lys Ser Ser Asp Ser Gly Ser Asp Ser Thr Glu Lys Leu Thr Ala Gly
            740                 745                 750

Thr Lys Ser Gly Glu Ser Thr Ile Lys Ala Ser Ala Ile Pro Arg Ser
        755                 760                 765

Pro Thr Arg Ser Pro Arg Ala Ala Ala Thr Ser Pro Met Arg Pro Pro
    770                 775                 780

Pro Arg Thr Gly Ser Val Arg Lys Ser Pro Arg Lys Val Ala Glu Gln
785                 790                 795                 800

Ile Asn Thr Ser Gly Gln Glu Ser Ala Arg Ser Ala Ser Pro Gly Pro
                805                 810                 815

Lys Val Lys Glu Ser Ile Ala Leu Phe Gln Ser Ser Arg Asn Gly Gly
            820                 825                 830

Leu Thr Asp Lys His Thr Arg Ser Pro Thr Thr Gln Pro Arg Ser Leu
        835                 840                 845

Ala Ala Leu Phe Gly Ser Pro Lys Gln Ala Thr Lys Leu Pro Thr Leu
    850                 855                 860

Ser Ala Thr Ala Gly Ser Ser Thr Thr Pro Ala His Ser Pro Pro Lys
865                 870                 875                 880

Asn Trp Gln Gly Ser Arg Leu Pro Phe Ala Gln Thr Phe Gln Leu Gln
                885                 890                 895

Gln Gln Ala Leu Arg Glu Val Asp Lys Ser Leu Pro Gln Thr Pro Ala
            900                 905                 910

Glu Glu Thr Ala Asp Ser Gln Arg Val Val Ser Ala Ala Ser Thr Val
        915                 920                 925

Leu Ser Thr Ser Lys Ala Glu Thr Ala Glu Ser Gln Phe Phe Asp Ala
    930                 935                 940

Arg Ser Arg Glu Asn Ser Ala Glu Pro Ala Asp Ala Pro Ala Pro Ala
945                 950                 955                 960

Pro Leu Ala Ala Met Lys Gly Ala Thr Ala Pro Gly Thr Ser Ala Pro
                965                 970                 975

Gln Val Val Ala Glu Lys Glu Cys Val Met Leu Ala Glu Ala Pro Ile
            980                 985                 990

Val Ala Asn Lys Ala Ser Ser Ile  Val Lys Thr Ser Ser  Leu Ser Pro
        995                 1000                1005

Lys Lys  Val Ala Ala Lys Ala  Leu Ala Lys Thr Gly  Pro Thr Ser
    1010                1015                1020

Pro Thr  Lys Ala Ser Ser Leu  Arg Ala Ala Ala Asn  Ala Thr Ser
    1025                1030                1035

Pro Ser  Lys Ile Lys Ala Ser  Ala Arg Thr Ala Gln  Gln His Gly
    1040                1045                1050

Gly Pro  Gly Leu Gly Pro Gly  Pro Ala Pro Ser Gly  Ser Pro Ser
    1055                1060                1065

Arg Ala  Trp Gly Ile Gly Glu  Lys Ile Lys Gly Phe  Leu Gly Leu
    1070                1075                1080

His Ala  Ser Thr Thr Ile His  Pro Ser Thr Ala Val  Ala Gln Thr
    1085                1090                1095

Lys Val  Ser Ala Ser Gly Thr  Phe Gln Pro Arg Ala  Asn Gly Phe
    1100                1105                1110

Ala Thr  Asn Thr Ala His Ala  Ser Ala Ser Glu His  Lys Thr Ala
    1115                1120                1125

Ala Thr  Ala Thr Pro Ala Pro  Cys Ile Pro Gly Ala  Leu Leu Ala
    1130                1135                1140

Ser Pro  Pro Pro Thr Ala Lys  Ser Ala Gln Val Asn  Ser Thr Pro
    1145                1150                1155

Ser Ser  Gly Ala Ala Ser Ala  Thr Ala Asn Gly Lys  Leu Thr Ser
    1160                1165                1170

Ile Ala Arg Ala Glu Met Leu Arg Lys Lys Gln Ala Glu Glu Glu
    1175                1180                1185

Glu Arg Lys Ala Lys Asp Lys Gln Glu Arg Arg Arg Met Leu Met
    1190                1195                1200

Ala Lys Lys Glu Glu Arg Ala Lys Ala Val Ala Glu Glu Glu Ile
    1205                1210                1215

Glu Lys Glu Asn Arg Lys Arg Ala Arg Glu Gln Asn Asp Arg Leu
    1220                1225                1230

Gly Arg Ser Gly Ile Glu Asp Ala Ser Ile Ala Ser Ser Asn Gly
    1235                1240                1245

Ala Pro Ala Ala Ile Pro Ala Ala Leu Leu Gly Asn Ile Val Val
    1250                1255                1260

Gly Gly Gly Gly Ser Ala Ile Ser Gly Ser Val His Ser Gly Ser
    1265                1270                1275

Ala Ser Ser Arg Thr Ser Thr Val Ser Thr Val Ser Ser Gly Pro
    1280                1285                1290

Pro Thr Arg Pro Ser Ser Ala Leu Asn Gly Ser Tyr Ala Gly Val
    1295                1300                1305

Lys Ser Arg Tyr Met Gln Gly Ala Val Gln Gln Gln Gln Gln Gln
    1310                1315                1320

Pro Pro Pro Gln Gln Gln Gly Glu Glu Ser Ser Lys Lys Arg Arg
    1325                1330                1335

Val Thr Asn Asp Lys Glu Gly Ile Ala Ala Gly Leu Gln Val Pro
    1340                1345                1350

Leu Lys Gln Gln Pro Ala Pro Gln Gln Ser Gln Gly Ala Pro Ser
    1355                1360                1365

Ala Arg Pro Gly Ala Ser Val Ala Thr Ser Gln Ile Gly Ala Pro
    1370                1375                1380

Lys Met Val Arg Thr Val Ser Gln Ser Ser Ile Arg Ser Gly Ala
    1385                1390                1395

Ala Ser Gln Ala Ala Ala Gln Gln Gln Gln Gln Gln Gln Arg Thr
    1400                1405                1410

Gln Pro Pro Pro Gln Ile Lys Leu Gly Met Lys Pro Ser Ala Ala
    1415                1420                1425

Thr Thr Ala Ala Ala Gly Ala Gly Phe Gly Ala Ala Pro Arg Ile
    1430                1435                1440

Gly Gly Ala Gly Ala Thr Ala Gly Asn Gly Ala Ala Thr Ala Ser
    1445                1450                1455

Met Met Ala Asn Ala Gly Lys Ala Val Ser Ser Ala Gln Pro Ala
    1460                1465                1470

Ala Ala Lys Ala Met Ala Asn Ala Asn Phe Cys Ser Ser Asn Pro
    1475                1480                1485

Phe Gln Gln Ala Lys Gln Gln Gln Leu Gln Leu Gln Leu Gln Lys
    1490                1495                1500

Gln Lys Gln Leu Gln Met Gln Gln Gln Gln Gln Gln Gln Gln Gln
    1505                1510                1515

Val Ala Ser Asn Ala Met Gln Ala Gln Ala Ala Ala Ser Ala Ala
    1520                1525                1530

Ala Val Arg Glu Leu Glu Leu His Arg Ala Ala Gln Leu Gln Met
    1535                1540                1545

Gln Met Gln Asn Ala Ala Gly Gly Arg Tyr Gln Glu Met Asp Asp
    1550                1555                1560

Asp Val Ala Gly Asp Gly Asp Glu Val Leu Pro Glu Ile Ala Ser
    1565                1570                1575

Glu Tyr Ser Asp Ser Glu Asp Glu Glu Thr Ile Gln Lys Arg Ala
    1580                1585                1590

Ala Met Pro Leu Trp Thr Gln Gly Asp Met Leu Asp Ala Ala Leu
    1595                1600                1605

Leu Ala Gln Ser Thr Val Asp Ala Asp Glu Ile Phe Gly Ile Pro
    1610                1615                1620

His Gly Pro Val Glu Leu Glu Lys Ile Leu Pro Gly Glu Arg Thr
    1625                1630                1635

Ala Asn Arg Ile Arg Arg Ala Arg Thr Ser Ser Ala Asn Trp Ser
    1640                1645                1650

Gly Pro Asp Gly Leu Ala Gln Trp Glu Ile Asp Arg Tyr Asn Lys
    1655                1660                1665

Arg Met Gly Ile Lys Ser Ala Gly Val Gln Leu His Arg Asp Pro
    1670                1675                1680

Ser Gln Pro Ser Arg Pro Ala Ala Met Pro Ser Leu Ser Ile His

1685                    1690                    1695

Arg His  Ser Ile Ser His Asn  Pro Leu Ala Pro Thr  Ser Ser Thr
    1700                    1705                    1710

Ser Ser  Thr Ala Pro His Thr  Ala Ile Asn Asp Asp  Ala Arg Thr
    1715                    1720                    1725

Asn Ala
    1730

<210>  5
<211>  456
<212>  DNA
<213>  Ustilago maydis

<220>
<223>  Aurorakinaseaktivator (C-Terminus)

<400>  5
tactcggact cggaagacga agagacaatt cagaagcgag cggcgatgcc gttgtggacg        60

cagggcgata tgctggatgc ggcgctgttg gcgcaatcca cggtggatgc cgatgagatc       120

tttggtattc cacatggtcc tgttgagcta gaaaaaatct tgcctggcga gcgcacagcg       180

aaccggatcc gacgagcgcg aacttcgtcg gccaactggt ccgggcccga cggcctcgcg       240

cagtgggaga tcgacagata caacaaacgc atgggcatca agagtgcagg tgtccagtta       300

catcgcgacc catctcagcc gtctcggcca gccgctatgc catccctctc gatccaccga       360

cactcgatct cgcacaaccc actcgctccc acctcgtcca cctcgtccac tgctccacac       420

acagccatca acgacgacgc tcgtacaaat gcttag                                 456

<210>  6
<211>  151
<212>  PRT
<213>  Ustilago maydis

<220>
<223>  Aurorakinaseaktivator (C-Terminus)

<400>  6

Tyr Ser Asp Ser Glu Asp Glu Glu Thr Ile Gln Lys Arg Ala Ala Met
1               5                   10                  15

Pro Leu Trp Thr Gln Gly Asp Met Leu Asp Ala Ala Leu Leu Ala Gln
            20                  25                  30

Ser Thr Val Asp Ala Asp Glu Ile Phe Gly Ile Pro His Gly Pro Val
        35                  40                  45

Glu Leu Glu Lys Ile Leu Pro Gly Glu Arg Thr Ala Asn Arg Ile Arg
    50                  55                  60

Arg Ala Arg Thr Ser Ser Ala Asn Trp Ser Gly Pro Asp Gly Leu Ala
65                  70                  75                  80

Gln Trp Glu Ile Asp Arg Tyr Asn Lys Arg Met Gly Ile Lys Ser Ala
                85                  90                  95

Gly Val Gln Leu His Arg Asp Pro Ser Gln Pro Ser Arg Pro Ala Ala
            100                 105                 110

Met Pro Ser Leu Ser Ile His Arg His Ser Ile Ser His Asn Pro Leu
        115                 120                 125

Ala Pro Thr Ser Ser Thr Ser Ser Thr Ala Pro His Thr Ala Ile Asn
    130                 135                 140

Asp Asp Ala Arg Thr Asn Ala
145                 150

<210>  7
<211>  26
<212>  DNA
<213>  Artificial Sequence

<220>
<223>  Nukleinsäuresequenz Primer AK_forward

<400>  7
caccatggag tcgcagctcg ccaact                                          26

<210>  8
<211>  29
<212>  DNA
<213>  Artificial Sequence

<220>
<223>  Nukleinsäuresequenz Primer AKA_forward

<400>  8
cacctactcg gactcggaag acgaagaga                                       29

<210>  9
<211>  23
<212>  DNA
<213>  Artificial Sequence

<220>
<223>  Nukleinsäuresequenz Primer AK_reverse

<400>  9
ttactgaatt ttgccacgag acg                                             23

<210>  10
<211>  22
<212>  DNA
<213>  Artificial Sequence

<220>
<223>  Nukleinsäuresequenz Primer AKA_reverse

<400>  10
ctaagcattt gtacgagcgt cg                                              22

<210> 11
<211> 2679
<212> DNA
<213> Artificial Sequence

<220>
<223> Aurorakinase mit MBP-TAG

<400> 11
atgaaaactg aagaaggtaa actggtaatc tggattaacg gcgataaagg ctataacggt 60

ctcgctgaag tcggtaagaa attcgagaaa gataccggaa ttaaagtcac cgttgagcat 120

ccggataaac tggaagagaa attcccacag gttgcggcaa ctggcgatgg ccctgacatt 180

atcttctggg cacacgaccg ctttggtggc tacgctcaat ctggcctgtt ggctgaaatc 240

accccggaca aagcgttcca ggacaagctg tatccgttta cctgggatgc cgtacgttac 300

aacggcaagc tgattgctta cccgatcgct gttgaagcgt tatcgctgat ttataacaaa 360

gatctgctgc cgaacccgcc aaaaacctgg gaagagatcc cggcgctgga taaagaactg 420

aaagcgaaag gtaagagcgc gctgatgttc aacctgcaag aaccgtactt cacctggccg 480

ctgattgctg ctgacggggg ttatgcgttc aagtatgaaa acggcaagta cgacattaaa 540

gacgtgggcg tggataacgc tggcgcgaaa gcgggtctga ccttcctggt tgacctgatt 600

aaaaacaaac acatgaatgc agacaccgat tactccatcg cagaagctgc ctttaataaa 660

ggcgaaacag cgatgaccat caacggcccg tgggcatggt ccaacatcga caccagcaaa 720

gtgaattatg gtgtaacggt actgccgacc ttcaagggtc aaccatccaa accgttcgtt 780

ggcgtgctga gcgcaggtat taacgccgcc agtccgaaca aagagctggc aaaagagttc 840

ctcgaaaact atctgctgac tgatgaaggt ctggaagcgg ttaataaaga caaaccgctg 900

ggtgccgtag cgctgaagtc ttacgaggaa gagttggcga aagatccacg tattgccgcc 960

accatggaaa acgcccagaa aggtgaaatc atgccgaaca tcccgcagat gtccgctttc 1020

tggtatgccg tgcgtactgc ggtgatcaac gccgccagcg gtcgtcagac tgtcgatgaa 1080

gccctgaaag acgcgcagac taattcgagc tcgaacaaca acaacaataa caataacaac 1140

aacctcggga tcgagggaag gatttcagaa ttatcaacaa gtttgtacaa aaaagcaggc 1200

tccgcggccg ccgtactcga ggaaaacctg tattttcagg gctccttcac catggagtcg 1260

cagctcgcca acttgcacct ggatggcggc tcgacggcag atctcaagca aggatccaga 1320

ccgtcacatg cacaggcatc gcacggaaac agacatgttc atcctacaaa ctcgtcactc 1380

cgacctctgg cgccttccag ctcaattgcg tccaacatgg gtatcaatat gggtgtcaac 1440

atgcagcact tcaaccgtct agctgcctcc tcaacggggc tcacgaaccc caacagcatt 1500

gcacaaggca tgagccgagc tgcgcagcag ccgctcaacg gatcgagacc tagcacacaa 1560

caacagccta cgcaggcaca tatcaagagt cgacctccta gcgttgctgg cggactgaat 1620

gctgcggctg gtccttcgta tcgtccagcg gcgcctgctg ctcaatctac caaacaacca 1680

gctagtgctg gatcagccgt agcatcggtt gatttgggac gatatgacgg tggtctggaa 1740

cgcgatgaag cacgcggacg tcgaggaacg atgcaattcg atccgctcac gctttcctct 1800

gccgatgctg ggaaacaaca tccaccaacc cgcgtgtggt cgctcaaaga cttcgaaatg 1860

ggtcgacctc ttggcaaagg caaattcggt cgcgtgtaca tggttcgcac tcgcgctgca 1920

cccaacaaag gctacattat cgccctcaag tgcatgtaca aaaacgagct agtcgagaac 1980

aaggtggaaa aacaacttcg aagagagatc gagatccaga tgaacctccg ccatcctcat 2040

atccttcgac ttcacggata cttccacgat gagggccgag tcttcctcat gatcgaattt 2100

gctggtcgtg gggagctcta caaattgatg aacaaactac acgatcgaag gttcgaagaa 2160

aaggttgcag caacctacat cgcgcagatg gcagacgctc tatcatacct gcacagcaag 2220

cacgtcattc atcgcgacat caaaccagaa aaccttctat tgggtatcaa aggcgatctc 2280

aagattggtg actttggatg gagcgttcac gcccctggaa accgtcgtca gactctgtgt 2340

ggtaccttgg actacttgcc ccccgagatg gtaaatggag agcaacacga taaggcagta 2400

gacctgtggg cattgggcgt gctgtgttac gaatttttgg aaggtgttcc tccgtttgaa 2460

gagctggaaa acgctcccgc aggcacgtac aagagaatca acaacatcga cttcaagatc 2520

cctcgacatt tcagccccga agctgccgat ctggtaaagg cactgctaaa gaagaaacca 2580

gacgacaggc tacccttgac caaagtgctg aggcatccgt ggatcatgaa gtacgatcca 2640

gatgcttgtc gtcgtgcgtc tcgtggcaaa attcagtaa 2679

<210> 12
<211> 892
<212> PRT
<213> Artificial Sequence

<220>
<223> Aurorakinase mit MBP-TAG

<400> 12
   Met Lys Thr Glu Glu Gly Lys Leu Val Ile Trp Ile Asn Gly Asp Lys
   1 5 10 15
   Gly Tyr Asn Gly Leu Ala Glu Val Gly Lys Lys Phe Glu Lys Asp Thr
   20 25 30
   Gly Ile Lys Val Thr Val Glu His Pro Asp Lys Leu Glu Glu Lys Phe
   35 40 45
   Pro Gln Val Ala Ala Thr Gly Asp Gly Pro Asp Ile Ile Phe Trp Ala
   50 55 60
   His Asp Arg Phe Gly Gly Tyr Ala Gln Ser Gly Leu Leu Ala Glu Ile
   65 70 75 80
   Thr Pro Asp Lys Ala Phe Gln Asp Lys Leu Tyr Pro Phe Thr Trp Asp
   85 90 95
   Ala Val Arg Tyr Asn Gly Lys Leu Ile Ala Tyr Pro Ile Ala Val Glu
   100 105 110
   Ala Leu Ser Leu Ile Tyr Asn Lys Asp Leu Leu Pro Asn Pro Pro Lys
           115                 120                 125
   Thr Trp Glu Glu Ile Pro Ala Leu Asp Lys Glu Leu Lys Ala Lys Gly
       130                 135                 140
   Lys Ser Ala Leu Met Phe Asn Leu Gln Glu Pro Tyr Phe Thr Trp Pro
   145                 150                 155                 160
   Leu Ile Ala Ala Asp Gly Gly Tyr Ala Phe Lys Tyr Glu Asn Gly Lys
                   165                 170                 175
   Tyr Asp Ile Lys Asp Val Gly Val Asp Asn Ala Gly Ala Lys Ala Gly
               180                 185                 190
   Leu Thr Phe Leu Val Asp Leu Ile Lys Asn Lys His Met Asn Ala Asp
           195                 200                 205
   Thr Asp Tyr Ser Ile Ala Glu Ala Ala Phe Asn Lys Gly Glu Thr Ala
       210                 215                 220
   Met Thr Ile Asn Gly Pro Trp Ala Trp Ser Asn Ile Asp Thr Ser Lys
   225                 230                 235                 240
   Val Asn Tyr Gly Val Thr Val Leu Pro Thr Phe Lys Gly Gln Pro Ser
                   245                 250                 255
   Lys Pro Phe Val Gly Val Leu Ser Ala Gly Ile Asn Ala Ala Ser Pro
               260                 265                 270
   Asn Lys Glu Leu Ala Lys Glu Phe Leu Glu Asn Tyr Leu Leu Thr Asp
           275                 280                 285
   Glu Gly Leu Glu Ala Val Asn Lys Asp Lys Pro Leu Gly Ala Val Ala
       290                 295                 300
   Leu Lys Ser Tyr Glu Glu Glu Leu Ala Lys Asp Pro Arg Ile Ala Ala
   305                 310                 315                 320
   Thr Met Glu Asn Ala Gln Lys Gly Glu Ile Met Pro Asn Ile Pro Gln
                   325                 330                 335
   Met Ser Ala Phe Trp Tyr Ala Val Arg Thr Ala Val Ile Asn Ala Ala
               340                 345                 350
   Ser Gly Arg Gln Thr Val Asp Glu Ala Leu Lys Asp Ala Gln Thr Asn
           355                 360                 365
   Ser Ser Ser Asn Asn Asn Asn Asn Asn Asn Asn Asn Asn Leu Gly Ile
       370                 375                 380
   Glu Gly Arg Ile Ser Glu Leu Ser Thr Ser Leu Tyr Lys Lys Ala Gly
   385                 390                 395                 400
   Ser Ala Ala Ala Val Leu Glu Glu Asn Leu Tyr Phe Gln Gly Ser Phe
                   405                 410                 415
   Thr Met Glu Ser Gln Leu Ala Asn Leu His Leu Asp Gly Gly Ser Thr
               420                 425                 430
   Ala Asp Leu Lys Gln Gly Ser Arg Pro Ser His Ala Gln Ala Ser His
           435                 440                 445
   Gly Asn Arg His Val His Pro Thr Asn Ser Ser Leu Arg Pro Leu Ala
       450                 455                 460
   Pro Ser Ser Ser Ile Ala Ser Asn Met Gly Ile Asn Met Gly Val Asn
   465                 470                 475                 480
   Met Gln His Phe Asn Arg Leu Ala Ala Ser Ser Thr Gly Leu Thr Asn
                   485                 490                 495
   Pro Asn Ser Ile Ala Gln Gly Met Ser Arg Ala Ala Gln Gln Pro Leu
               500                 505                 510
   Asn Gly Ser Arg Pro Ser Thr Gln Gln Gln Pro Thr Gln Ala His Ile
           515                 520                 525
   Lys Ser Arg Pro Pro Ser Val Ala Gly Gly Leu Asn Ala Ala Ala Gly
       530                 535                 540
   Pro Ser Tyr Arg Pro Ala Ala Pro Ala Ala Gln Ser Thr Lys Gln Pro
   545                 550                 555                 560
   Ala Ser Ala Gly Ser Ala Val Ala Ser Val Asp Leu Gly Arg Tyr Asp
                   565                 570                 575
   Gly Gly Leu Glu Arg Asp Glu Ala Arg Gly Arg Arg Gly Thr Met Gln
               580                 585                 590
   Phe Asp Pro Leu Thr Leu Ser Ser Ala Asp Ala Gly Lys Gln His Pro
           595                 600                 605
   Pro Thr Arg Val Trp Ser Leu Lys Asp Phe Glu Met Gly Arg Pro Leu
       610                 615                 620
   Gly Lys Gly Lys Phe Gly Arg Val Tyr Met Val Arg Thr Arg Ala Ala
   625                 630                 635                 640
   Pro Asn Lys Gly Tyr Ile Ile Ala Leu Lys Cys Met Tyr Lys Asn Glu
                   645                 650                 655
   Leu Val Glu Asn Lys Val Glu Lys Gln Leu Arg Arg Glu Ile Glu Ile
   660                     665                     670
   Gln Met Asn Leu Arg His Pro His Ile Leu Arg Leu His Gly Tyr Phe
           675                     680                     685
   His Asp Glu Gly Arg Val Phe Leu Met Ile Glu Phe Ala Gly Arg Gly
       690                     695                     700
   Glu Leu Tyr Lys Leu Met Asn Lys Leu His Asp Arg Arg Phe Glu Glu
   705                     710                     715                     720
   Lys Val Ala Ala Thr Tyr Ile Ala Gln Met Ala Asp Ala Leu Ser Tyr
                   725                     730                     735
   Leu His Ser Lys His Val Ile His Arg Asp Ile Lys Pro Glu Asn Leu
               740                     745                     750
   Leu Leu Gly Ile Lys Gly Asp Leu Lys Ile Gly Asp Phe Gly Trp Ser
           755                     760                     765
   Val His Ala Pro Gly Asn Arg Arg Gln Thr Leu Cys Gly Thr Leu Asp
       770                     775                     780
   Tyr Leu Pro Pro Glu Met Val Asn Gly Glu Gln His Asp Lys Ala Val
   785                     790                     795                     800
   Asp Leu Trp Ala Leu Gly Val Leu Cys Tyr Glu Phe Leu Glu Gly Val
                   805                     810                     815
   Pro Pro Phe Glu Glu Leu Glu Asn Ala Pro Ala Gly Thr Tyr Lys Arg
               820                     825                     830
   Ile Asn Asn Ile Asp Phe Lys Ile Pro Arg His Phe Ser Pro Glu Ala
           835                     840                     845
   Ala Asp Leu Val Lys Ala Leu Leu Lys Lys Lys Pro Asp Asp Arg Leu
       850                     855                     860
   Pro Leu Thr Lys Val Leu Arg His Pro Trp Ile Met Lys Tyr Asp Pro
   865                     870                     875                     880
   Asp Ala Cys Arg Arg Ala Ser Arg Gly Lys Ile Gln
                   885                     890

<210>  13
<211>  6444
<212>  DNA
<213>  Artificial Sequence

<220>
<223>  Aurorakinaseaktivator mit MBP-TAG

<400>  13
atgaaaactg aagaaggtaa actggtaatc tggattaacg gcgataaagg ctataacggt        60

ctcgctgaag tcggtaagaa attcgagaaa gataccggaa ttaaagtcac cgttgagcat 120
ccggataaac tggaagagaa attcccacag gttgcggcaa ctggcgatgg ccctgacatt 180
atcttctggg cacacgaccg ctttggtggc tacgctcaat ctggcctgtt ggctgaaatc 240
accccggaca aagcgttcca ggacaagctg tatccgttta cctgggatgc cgtacgttac 300
aacggcaagc tgattgctta cccgatcgct gttgaagcgt tatcgctgat ttataacaaa 360
gatctgctgc cgaacccgcc aaaaacctgg gaagagatcc cggcgctgga taaagaactg 420
aaagcgaaag gtaagagcgc gctgatgttc aacctgcaag aaccgtactt cacctggccg 480
ctgattgctg ctgacggggg ttatgcgttc aagtatgaaa acggcaagta cgacattaaa 540
gacgtgggcg tggataacgc tggcgcgaaa gcgggtctga ccttcctggt tgacctgatt 600
aaaaacaaac acatgaatgc agacaccgat tactccatcg cagaagctgc ctttaataaa 660
ggcgaaacag cgatgaccat caacggcccg tgggcatggt ccaacatcga caccagcaaa 720
gtgaattatg gtgtaacggt actgccgacc ttcaagggtc aaccatccaa accgttcgtt 780
ggcgtgctga gcgcaggtat taacgccgcc agtccgaaca aagagctggc aaaagagttc 840
ctcgaaaact atctgctgac tgatgaaggt ctggaagcgg ttaataaaga caaaccgctg 900
ggtgccgtag cgctgaagtc ttacgaggaa gagttggcga aagatccacg tattgccgcc 960
accatggaaa acgcccagaa aggtgaaatc atgccgaaca tcccgcagat gtccgctttc 1020
tggtatgccg tgcgtactgc ggtgatcaac gccgccagcg gtcgtcagac tgtcgatgaa 1080
gccctgaaag acgcgcagac taattcgagc tcgaacaaca acaacaataa caataacaac 1140
aacctcggga tcgagggaag gatttcagaa ttatcaacaa gtttgtacaa aaaagcaggc 1200
tccgcggccg ccgtactcga ggaaaacctg tattttcagg gctccttcac catgctccgc 1260
gttcggtttg gtgcctgtcc gcttgcattc atcctgacca tcatcactct ccggatcaca 1320
cccccacctg tcggctcttc cagattccaa ttgatgcact ttgtgctagt actcgcgcat 1380
cttgccagca gacgattaga ttcacgacca gagaaacgct gcctacactt gcatccgcct 1440
caaatcggct cacgccatcc atcctggtat ccacgctcat acagcgactc ggacaagctc 1500
cgaccaacct cgtccgccga ttcatctgtc tcttttcttg gtagcaaatc aatcacactc 1560
agcagccatg ccgacagctc gaaccgcttc tcgcaccgtg tcccggcctt ggactcctcg 1620
gcaagcacct cggctgcgag tagtgactca agctcaccac tgaccccaga agagcgtctg 1680
ctcagccacg tcaacaacat gcgcaggtgc tgcgacatgt ctctcttcca caagaacctc 1740
caagagcaag gtatcgattt tattgcagat agccttttgc atgccaacga catctttgcc 1800
aagtacgcaa caacgtcgcg caccgttgcc tcgccgtcca agctggctgg cgaactcctc 1860
aagtctccca cccgccctcg cacccgcgct cgtgatgcgc cagccgcatc aggctatcct 1920
cgtggggcat tcaaagtatc gattgatccc ttcgtcgatg cggccgacgt gttcgacaag 1980
gaaaatgcac ccttgcaaac tagatcttca cgatcacgta ccgcttccac tgtcgagcct 2040
cagaaaggca agggcaaggc tagagccaaa gctattcatg ctgtcgaaag agaagcatcg 2100

tcacctcttt ctcgcccgac ccgtagagtg cttggcgatg tcaacacgca aaagaatgag 2160
acaaagcaac ccactccaca gcccaagccc accagcaaag aagcaacgct caagaacatt 2220
gccacagatg acgacagcat ggtcattgat tcttcgctcc aagcgcacct ctcacccgat 2280
gataccatgc caagagcttc cacaccacca ccgcctcaag cgtcgcctcc gagaatactt 2340
gcccttcaca agccttccaa ggccactcag tccgcagcgt cactctcggg cgccatttcc 2400
atcactgatt cggacgagga cggctcagac ggagattcgg acgccgattt gcacgaagaa 2460
gagacaggag ggctaaacga taccgttcaa catgaagttg ccgagcctac agtgacagga 2520
aatgtcgtgt ctcggagctc ccgcagcgat gacaacacag catccttcca acctgacata 2580
aacgacacga tgccagccaa cgacaacacc gtgtctggta ctttgattga tccgaaactc 2640
gaaagtgccc gtcaagcagc ctctgtactg cttacaagca aaattcagca gcagaaggat 2700
gcaaacgcca tgaagccggt caaacggacg cttgcatcgt acggcgcaaa aaagagcctg 2760
ccagctgctg ccaacaactc cggtcccagc aacgtaacca ccacgatcgt tcccacctcg 2820
acccccgctg gtcctggctt ccgttccagc ttcctcaaca agagtctgcg caaacagatt 2880
gccgaccagg aagctctcga aagcggccaa gactctgact ctgactcgga cgacaacgaa 2940
aacgacacag gtcttgtcgc cggcgccacc tttgctgcta tgtacaaaaa ggcagccgcc 3000
aacacgcaag ccaatactcg cgcaaatgct gtaccagcgt caaacagcac cactagctcc 3060
aagaagcgca agagtgacga ggttcttcca gctgcgattg aggccagctt gagcgctgcc 3120
gctccaccgc acaaggtgtc caagctgggt gccgcgatga tggcggccaa gacacccacg 3180
tccgctcaac catctactgc caagaaagca ccagctgccg gccctgcgtc caaactggag 3240
caattccgca acaacctcgc tcatgttgct cgcagcaccg gaagcggtgc gagtgccagc 3300
tcggcttctc agtcagcgtc gtccgtcacg tcgcccacca tggaacaagc accatccgcg 3360
ccgctgcccg tcgtcaagcc ttcggatgcg gccgtcgtgc cattcgctca agaagcgtcg 3420
gcactgcctt ccattgaaag cttgaatgct tcgcattcga aatcctcgga ttctggctcc 3480
gactcgacgg aaaaacttac ggcaggtacc aagagtggcg agtcaaccat caaggcttct 3540
gccatcccac gatcgcccac tcgctctcct cgcgccgccg ccacttcacc gatgcggcct 3600
ccgcctcgca ccggcagtgt gcgtaaatct ccacgcaaag tggccgagca gatcaacaca 3660
agcggacaag agtcggccag atcggcttcg ccggggccca aggtgaaaga aagtatagct 3720
cttttccagt cgagcagaaa cggcggtctg actgataagc acacacgtag ccctaccacg 3780
cagccccgaa gtctcgcagc actctttggc agtcccaagc aagcgaccaa actaccgacg 3840
ctgtcggcca ctgccggcag cagcaccacc ccggctcact ctccacccaa gaactggcaa 3900
ggaagtcgtt taccgtttgc acaaaccttc cagttgcaac agcaagcgct gcgtgaggtg 3960
gacaagtctt tgccgcagac accggccgag gagacggccg acagccagcg tgtcgtctcg 4020
gcagcctcga cggtcttgtc cacgtccaag gctgaaacag cggagagcca attcttcgac 4080
gcccgctcga gggagaacag tgccgagcca gccgatgcgc cagctccagc gccattggcg 4140
gcgatgaaag gcgcgacggc acctgggacc agtgcacctc aagtggtcgc cgaaaaggag 4200

tgcgtcatgc ttgctgaggc acccatcgtt gccaacaagg catccagcat tgtgaagact 4260
agctcgcttt caccaaagaa agtggcggcc aaggcgcttg cgaaaactgg accgacctcg 4320
ccgaccaagg cgagcagtct ccgagctgca gccaacgcca cgtcgccttc caagatcaag 4380
gcgtcggcgc gcacagcaca gcagcatggc ggaccgggac tgggaccggg accggcaccg 4440
tctggatcgc cttcgagggc ttggggcatt ggtgaaaaga tcaaagggtt cctcggattg 4500
catgcctcga cgacgatcca cccatctaca gcagttgctc agaccaaggt atcggcgtct 4560
ggcacattcc agccgcgtgc caacgggttt gccacaaaca cggctcatgc gagcgcttcc 4620
gaacacaaga ctgcggctac agctacaccg gcaccttgca ttcctggcgc tctgcttgct 4680
agccctccgc caacggccaa gtctgcgcag gtcaactcaa caccctcaag cggtgctgct 4740
tcggcgacgg ccaacggaaa gctcaccagc attgcgcgcg ccgaaatgct gcgcaagaag 4800
caggcagagg aggaggagcg caaggctaag gacaagcaag agcgtcgaag aatgctgatg 4860
gccaagaagg aagagcgtgc caaggcggtc gcggaggagg aaatcgaaaa ggagaaccgc 4920
aaacgagcgc gtgagcagaa cgaccgcctc ggccgttccg gcatcgaaga cgccagcatc 4980
gcttcgtcta acggtgcacc ggctgcgatt cctgctgcgc tcctgggcaa tattgtcgtc 5040
ggtggcggtg gctccgcaat ttctggcagt gttcacagcg gctcagctag ctcaaggaca 5100
agcacagtca gcaccgttag cagcggaccg cccacgcgac catcaagtgc tctcaatgga 5160
agctatgctg gtgtcaagag tcgctacatg cagggagcgg tccagcagca gcagcagcag 5220
ccgccgccgc agcaacaagg cgaggaaagt agtaagaagc gtcgcgtcac aaatgacaag 5280
gaaggtatag cggcaggctt gcaagtgcca ttgaagcagc agccggcgcc tcagcaatca 5340
caaggtgcac ctagcgctcg tcctggtgct tcggtggcga cgtcgcagat cggtgcaccc 5400
aagatggttc gcacagtcag ccagtcgtcg atacgatctg gtgctgcctc acaagctgct 5460
gcccagcaac agcaacagca acagcgcact cagccgccgc cgcagatcaa gctgggcatg 5520
aagccgagtg cggcgacgac agcagcagca ggagcaggat ttggagcagc gccacgcata 5580
ggcggagctg gtgctacagc cggcaacggc gcggctactg cgtcaatgat ggcgaatgct 5640
ggcaaagctg tttcgtcggc gcaacccgcg gcagccaagg ctatggcgaa tgccaacttt 5700
tgctcttcga atccgttcca gcaagccaag cagcagcaac tgcagctgca gttgcaaaag 5760
cagaagcagc ttcaaatgca gcaacagcag caacagcaac agcaagttgc atccaacgcg 5820
atgcaggcgc aggcggctgc tagtgcggca gcggtgcgag aattagagtt gcatcgtgcg 5880
gcgcaattgc aaatgcagat gcagaatgca gctggtggac ggtatcaaga gatggacgac 5940
gatgttgcag gcgatggcga cgaggtgctg ccggagattg catcggaata ctcggactcg 6000
gaagacgaag agacaattca gaagcgagcg gcgatgccgt tgtggacgca gggcgatatg 6060
ctggatgcgg cgctgttggc gcaatccacg gtggatgccg atgagatctt tggtattcca 6120
catggtcctg ttgagctaga aaaaatcttg cctggcgagc gcacagcgaa ccggatccga 6180
cgagcgcgaa cttcgtcggc caactggtcc gggcccgacg gcctcgcgca gtgggagatc 6240

gacagataca acaaacgcat gggcatcaag agtgcaggtg tccagttaca tcgcgaccca 6300

tctcagccgt ctcggccagc cgctatgcca tccctctcga tccaccgaca ctcgatctcg 6360

cacaacccac tcgctcccac ctcgtccacc tcgtccactg ctccacacac agccatcaac 6420

gacgacgctc gtacaaatgc ttag 6444

<210> 14
<211> 568
<212> PRT
<213> Artificial Sequence

<220>
<223> Aurorakinaseaktivator mit MBP-TAG

<400> 14

Met Lys Thr Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg Ile Ser Glu Leu Ser Thr Ser Leu Tyr Lys Lys Ala Gly
385                 390                 395                 400

Ser Ala Ala Ala Val Leu Glu Glu Asn Leu Tyr Phe Gln Gly Ser Phe
                405                 410                 415

Thr Tyr Ser Asp Ser Glu Asp Glu Glu Thr Ile Gln Lys Arg Ala Ala
            420                 425                 430

Met Pro Leu Trp Thr Gln Gly Asp Met Leu Asp Ala Ala Leu Leu Ala
        435                 440                 445

Gln Ser Thr Val Asp Ala Asp Glu Ile Phe Gly Ile Pro His Gly Pro
    450                 455                 460

Val Glu Leu Glu Lys Ile Leu Pro Gly Glu Arg Thr Ala Asn Arg Ile
465                 470                 475                 480

Arg Arg Ala Arg Thr Ser Ser Ala Asn Trp Ser Gly Pro Asp Gly Leu
                485                 490                 495

Ala Gln Trp Glu Ile Asp Arg Tyr Asn Lys Arg Met Gly Ile Lys Ser
            500                 505                 510

Ala Gly Val Gln Leu His Arg Asp Pro Ser Gln Pro Ser Arg Pro Ala
        515                 520                 525

Ala Met Pro Ser Leu Ser Ile His Arg His Ser Ile Ser His Asn Pro
    530                 535                 540

Leu Ala Pro Thr Ser Ser Thr Ser Ser Thr Ala Pro His Thr Ala Ile
545                 550                 555                 560

Asn Asp Asp Ala Arg Thr Asn Ala
                565

<210>  15
<211>  1707
<212>  DNA
<213>  Artificial Sequence

<220>
<223>  Aurorakinaseaktivator (C-Terminus) mit MBP-TAG

<400>  15
atgaaaactg aagaaggtaa actggtaatc tggattaacg gcgataaagg ctataacggt      60

ctcgctgaag tcggtaagaa attcgagaaa gataccggaa ttaaagtcac cgttgagcat     120

ccggataaac tggaagagaa attcccacag gttgcggcaa ctggcgatgg ccctgacatt     180

atcttctggg cacacgaccg ctttggtggc tacgctcaat ctggcctgtt ggctgaaatc     240

accccggaca aagcgttcca ggacaagctg tatccgttta cctgggatgc cgtacgttac     300

aacggcaagc tgattgctta cccgatcgct gttgaagcgt tatcgctgat ttataacaaa     360

gatctgctgc cgaacccgcc aaaaacctgg gaagagatcc cggcgctgga taaagaactg     420

aaagcgaaag gtaagagcgc gctgatgttc aacctgcaag aaccgtactt cacctggccg     480

ctgattgctg ctgacggggg ttatgcgttc aagtatgaaa acggcaagta cgacattaaa     540

gacgtgggcg tggataacgc tggcgcgaaa gcgggtctga ccttcctggt tgacctgatt     600

aaaaacaaac acatgaatgc agacaccgat tactccatcg cagaagctgc ctttaataaa     660

ggcgaaacag cgatgaccat caacggcccg tgggcatggt ccaacatcga caccagcaaa     720

gtgaattatg gtgtaacggt actgccgacc ttcaagggtc aaccatccaa accgttcgtt     780

ggcgtgctga gcgcaggtat taacgccgcc agtccgaaca aagagctggc aaaagagttc     840

ctcgaaaact atctgctgac tgatgaaggt ctggaagcgg ttaataaaga caaaccgctg     900

ggtgccgtag cgctgaagtc ttacgaggaa gagttggcga aagatccacg tattgccgcc     960

accatggaaa acgcccagaa aggtgaaatc atgccgaaca tcccgcagat gtccgctttc    1020

tggtatgccg tgcgtactgc ggtgatcaac gccgccagcg gtcgtcagac tgtcgatgaa    1080

gccctgaaag acgcgcagac taattcgagc tcgaacaaca acaacaataa caataacaac 1140

aacctcggga tcgagggaag gatttcagaa ttatcaacaa gtttgtacaa aaaagcaggc 1200

tccgcggccg ccgtactcga ggaaaacctg tattttcagg gctccttcac ctactcggac 1260

tcggaagacg aagagacaat tcagaagcga gcggcgatgc cgttgtggac gcagggcgat 1320

atgctggatg cggcgctgtt ggcgcaatcc acggtggatg ccgatgagat ctttggtatt 1380

ccacatggtc ctgttgagct agaaaaaatc ttgcctggcg agcgcacagc gaaccggatc 1440

cgacgagcgc gaacttcgtc ggccaactgg tccgggcccg acggcctcgc gcagtgggag 1500

atcgacagat acaacaaacg catgggcatc aagagtgcag gtgtccagtt acatcgcgac 1560

ccatctcagc cgtctcggcc agccgctatg ccatccctct cgatccaccg acactcgatc 1620

tcgcacaacc cactcgctcc cacctcgtcc acctcgtcca ctgctccaca cacagccatc 1680

aacgacgacg ctcgtacaaa tgcttag 1707

<210> 16
<211> 568
<212> PRT
<213> Artificial Sequence

<220>
<223> Aurorakinaseaktivator (C-Terminus) mit MBP-TAG

<400> 16

Met Lys Thr Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg Ile Ser Glu Leu Ser Thr Ser Leu Tyr Lys Lys Ala Gly
385                 390                 395                 400

Ser Ala Ala Ala Val Leu Glu Glu Asn Leu Tyr Phe Gln Gly Ser Phe
                405                 410                 415

Thr Tyr Ser Asp Ser Glu Asp Glu Glu Thr Ile Gln Lys Arg Ala Ala
            420                 425                 430

Met Pro Leu Trp Thr Gln Gly Asp Met Leu Asp Ala Ala Leu Leu Ala
        435                 440                 445

Gln Ser Thr Val Asp Ala Asp Glu Ile Phe Gly Ile Pro His Gly Pro
    450                 455                 460

Val Glu Leu Glu Lys Ile Leu Pro Gly Glu Arg Thr Ala Asn Arg Ile
465                 470                 475                 480

Arg Arg Ala Arg Thr Ser Ser Ala Asn Trp Ser Gly Pro Asp Gly Leu
                485                 490                 495

Ala Gln Trp Glu Ile Asp Arg Tyr Asn Lys Arg Met Gly Ile Lys Ser
            500                 505                 510

Ala Gly Val Gln Leu His Arg Asp Pro Ser Gln Pro Ser Arg Pro Ala
        515                 520                 525

Ala Met Pro Ser Leu Ser Ile His Arg His Ser Ile Ser His Asn Pro
    530                 535                 540

Leu Ala Pro Thr Ser Ser Thr Ser Ser Thr Ala Pro His Thr Ala Ile
545                 550                 555                 560

Asn Asp Asp Ala Arg Thr Asn Ala
                565

## Beschreibung

Die vorliegende Anmeldung bezieht sich unter anderem auf ein Verfahren zum Identifizieren von Fungiziden, umfassend (a) in Kontakt Bringen einer Kombination aus einem Polypeptid mit der biologischen Aktivität einer Aurorakinase (AK) und einem Polypeptid mit der biologischen Funktion eines Aurorakinaseaktivators (AKA) mit einer chemischen Verbindung oder einem Gemisch von chemischen Verbindungen, (b) Vergleichen der biologischen Aktivität der AK aus der Kombination aus (a) in Anwesenheit der chemischen Verbindung oder des Gemisches chemischer Verbindungen mit der biologischen Aktivität der AK aus der Kombination aus (a) in Abwesenheit der chemischen Verbindung oder des Gemisches von Verbindungen, und, optional, (c) Bestimmen der chemischen Verbindung aus einem Gemisch, welche die biologische Aktivität der AK inhibiert; wobei ein Polypeptid mit der biologischen Aktivität einer AK (i) eine Aminosäuresequenz SEQ ID No: 2; oder (ii) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 2 aufweist, umfasst; und wobei ein Polypeptid mit der biologischen Funktion eines AKA (I) eine Aminosäuresequenz SEQ ID No: 6; oder (II) eine Aminosäuresequenz, welche mindestens 70 %, mindestens 75 %, mindestens 80 %, mindestens 85 %, mindestens 90 %, mindestens 95 % und insbesondere mindestens 98 % Identität mit der Aminosäuresequenz SEQ ID No: 6 aufweist, umfasst. Darüber hinaus offenbart diese Anmeldung die Verwendung einer Kombination eines Polypeptids mit der biologischen Aktivität einer AK und eines Polypeptids mit der biologischen Funktion eines AKA wie oben beschrieben zum Auffinden von fungiziden Verbindungen, Nukleinsäuren umfassend eine Sequenz, die für einen AKA wie oben beschrieben codiert; ein dadurch codiertes Polypeptid, eine Kombination umfassend die oben beschriebenen Polypeptide mit der biologischen Funktion eines AKA und der biologischen Aktivität einer AK sowie ein Verfahren zum Herstellen dieser Kombination.

In dieser Beschreibung wird eine Anzahl von Dokumenten einschließlich Patentanmeldungen und Gebrauchsanleitungen zitiert. Obwohl die Offenbarungen dieser Dokumente nicht als relevant für die Patentfähigkeit der vorliegenden Erfindung angesehen werden, sind sie dennoch zur Gänze durch Verweis/Zitat in diese Anmeldung eingeschlossen. Insbesondere sind alle zitierten Dokumente in demselben Ausmaß in diese Anmeldung eingeschlossen, als wenn jedes Dokument spezifisch und einzeln als hier eingeschlossen beschrieben wäre.

Grundlegend sind Prinzipien der Identifizierung von Fungiziden über die Inhibierung eines definierten Targets durch diese Fungizide zum Beispiel aus WO 2005/042734, WO 03/054221, WO 00/77185 und US 5,187,071 bekannt. Im Allgemeinen gibt es einen großen Bedarf daran, Enzyme, die an essentiellen Stoffwechselwegen beteiligt sind, zu detektieren. Diese Enzyme können neue Zielproteine oder Targets für Fungizide darstellen um z. B. Resistenzen bekämpfen zu können und um generell Fungizide mit besserer ökologischer und toxikologischer Unbedenklichkeit und geringerem Aufwand darstellen zu können. Problematisch ist dabei häufig, dass die Auswirkungen der Hemmung eines Enzyms in einem Stoffwechselweg durch alternative Stoffwechselwege umgangen werden können.

Die Detektion neuer Targets ist in der Praxis mit großen Schwierigkeiten verbunden, da die Hemmung eines Enzyms, das Bestandteil eines Stoffwechselweges ist, häufig das Wachstum oder die Infektiosität des pathogenen Pilzes nicht weiter beeinflusst. Dies kann daran liegen, dass der pathogene Pilz auf alternative Stoffwechselwege ausweicht, deren Existenz nicht bekannt ist, oder dass das inhibierte Enzym für den Stoffwechselweg nicht limitierend ist. Die Eignung eines Genproduktes als Target lässt sich daher auch mit Kenntnis der Genfunktion nicht vorhersagen.

N-substituierte Diaminopyrimidine sind eine Klasse niedermolekularer Verbindungen, die als Inhibitoren humaner Kinasen beschrieben wurden (Argiriadi et al., 2010, Bioorg. Med. Chem. Lett., 20(1), 330-333). Zusätzlich wurde gefunden, dass sie fungizide Wirkung aufweisen (WO 2008/107096).

Während beim Menschen das Augenmerk auf der Inhibition deregulierter Kinaseaktivität zur Heilung von Krankheiten liegt, können Pilze wie z.B. *Neurospora crassa* durch Hemmung ihrer normalen Kinaseaktivität bekämpft werden (Pillonel, 2005, Pest Manag. Sci., 61, 1069-1076).

Aurorakinasen spielen beim Zellwachstum eine wichtige Rolle. Im menschlichen Genom finden sich drei verschiedene Aurorakinasen: A, B und C. Alle drei Aurorakinasen sind vor allem an der Zellteilung (Zytokinese) und der Segregation der Chromosomen beteiligt. Auch in anderen Organismen spielen Aurorakinasen eine essentielle Rolle bei diesen Vorgängen. Ein Verlust ihrer Funktion hat schwerwiegende Folgen, die durch ungleichmäßige Verteilung der Chromosomen hervorgerufen werden (Bischoff und Plowman, 1999, Trends Cell Biol., 9, 454-459). Während in *Metazoa* drei verschiedene Isoformen der Aurorakinase vorkommen (A, B und C), gibt es in Hefen wie *S*. *cerevisiae* oder *Schizosaccharomyces. pombe* nur eine Aurorakinase (Honda et al., 2003, Mol. Biol. Cell, 14, 3325-3341).

Es besteht immer ein Bedarf neue Targets und darauf gerichtete Fungizide zu finden um z. B. Resistenzen zu umgehen.

Die Aufgabe wird durch die vorliegenden erfindungsgemäßen Ansprüche gelöst.

In der vorliegenden Anmeldung wird zum ersten Mal ein zusammengehöriges Paar eines Polypeptids mit der biologischen Aktivität einer AK und eines Polypeptids mit der biologischen Funktion eines AKA eines phytopathogenen Pilzes, hier *U. maydis,* vollständig beschrieben, wobei die AK in Abwesenheit des AKA keine messbare Kinaseaktivität aufweist.

Ein Homologievergleich der Sequenz SEQ ID No: 1 aus *U. maydis* zeigte eine große Ähnlichkeit mit bekannten für humane AK kodierenden Sequenzen bzw. für AK kodierende Sequenzen aus anderen Pilzen. Eine Sequenz aus *Puccinia graminis* zeigte dabei die größte Homologie zur Sequenz SEQ ID No: 1 aus *U. maydis* (siehe Tabelle 1).

**Tab. 1:**

| | |
|---|---|
| *P. graminis* | Homolog: Aurora Proteinkinase, E-Value 3e-114, 65% Identität, 79% ähnliche Aminosäuren |
| *S. pombe* | Homolog: Aurora B / Ark1, E-Value 6e-99, 59% Identität, 77% ähnliche Aminosäuren |
| Mensch | Homolog: Aurora A, E-Value 3e-89, 60% Identität, 75% ähnliche Aminosäuren |

Alle bekannten AK des Typs A und B zeigen Aurorakinaseaktivität auch ohne Zugabe eines AKA, jedoch kann ein AKA die Aktivität einer entsprechenden AK um einen Faktor 7 bis 15 steigern (Honda et al., 2003, Mol. Biol. Cell, 14, 3325-3341; Kang et al., 2001, J. Cell Biol., 155, 763-774).

Im Gegensatz zu allen bisher bekannten AK zeigt das Translationsprodukt von SEQ ID No: 1 (SEQ ID No: 2) jedoch keine Kinaseaktivität. Auch in Anwesenheit des spezifischen, bekannten AKA (INCENP-Sequenz) aus *S*. *pombe* (Leverson et al., Mol. Biol. Cell., 2002, 13(4), 1132-43) dessen für eine AK kodierende Aminosäuresequenz eine hohe Ähnlichkeit mit der SEQ ID No: 1 aufzeigt, konnte keine Aurorakinaseaktivität eines Proteins mit der SEQ ID No: 2 gemessen werden.

Überraschend wurde jedoch gefunden, dass ein Protein mit der SEQ ID No: 2 in Anwesenheit eines Proteins mit der SEQ ID No: 6 oder eines Proteins, das eine Aminosäuresequenz SEQ ID No: 6 umfasst, wie z. B. SEQ ID No: 4, SEQ ID No: 14, oder SEQ ID No: 16, Kinaseaktivität aufweist.

Die SEQ ID No: 6 wird in MIPS (Munich Information Center for Protein Sequences (© 2003 GSF - Forschungszentrum für Umwelt und Gesundheit, GmbH Ingolstädter Landstraße 1, D-85764 Neuherberg)) lediglich als potentielles Protein aufgeführt.

### Sequenzen und Abbildungen

- SEQ ID No: 1: Nukleinsäuresequenz AK (*U. maydis*) ohne TAG
- SEQ ID No: 2: Aminosäuresequenz AK *(U. maydis*) ohne TAG
- SEQ ID No: 3: Nukleinsäuresequenz AKA (*U. maydis*) ohne TAG
- SEQ ID No: 4: Aminosäuresequenz AKA (*U. maydis*) ohne TAG
- SEQ ID No: 5: Nukleinsäuresequenz AKA (C-Terminus (*U. maydis*)) ohne TAG
- SEQ ID No: 6: Aminosäuresequenz AKA (C-Terminus (*U. maydis*)) ohne TAG
- SEQ ID No: 7: Nukleinsäuresequenz Primer AK forward
- SEQ ID No: 8: Nukleinsäuresequenz Primer AKA forward
- SEQ ID No: 9: Nukleinsäuresequenz Primer AK reverse
- SEQ ID No: 10: Nukleinsäuresequenz Primer AKA reverse
- SEQ ID No: 11: Nukleinsäuresequenz AK (*U. maydis*) mit TAG
- SEQ ID No: 12: Aminosäuresequenz AK (*U. maydis*) mit TAG
- SEQ ID No: 13: Nukleinsäuresequenz AKA (*U. maydis*) mit TAG
- SEQ ID No: 14: Aminosäuresequenz AKA (*U. maydis)* mit TAG
- SEQ ID No: 15: Nukleinsäuresequenz AKA (C-Terminus (*U. maydis*)) mit TAG
- SEQ ID No: 16: Aminosäuresequenz AKA (C-Terminus (*U. maydis*)) mit TAG

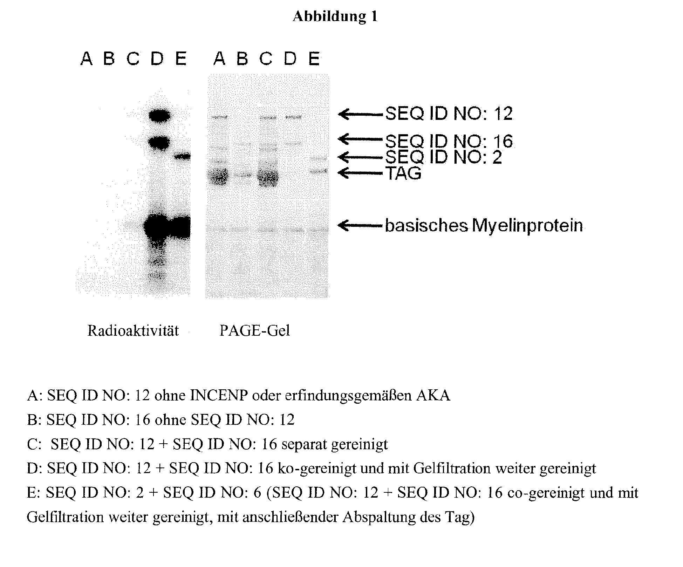

Abb. 1: Radioaktiver Aktivitätstest von (A) gereinigter SEQ ID No: 12, (B) gereinigter SEQ ID No: 16, (C) einer Kombination aus separat gereinigter SEQ ID No: 12 und SEQ ID No: 16, (D) einer Kombination aus co-gereinigter SEQ ID No: 12 und SEQ ID No: 16, (E) einer Kombination aus co-gereinigter SEQ ID No: 2 und SEQ ID No: 6 (co-gereinigter SEQ ID No: 12 und SEQ ID No: 16 mit anschließender Tag-Abspaltung)

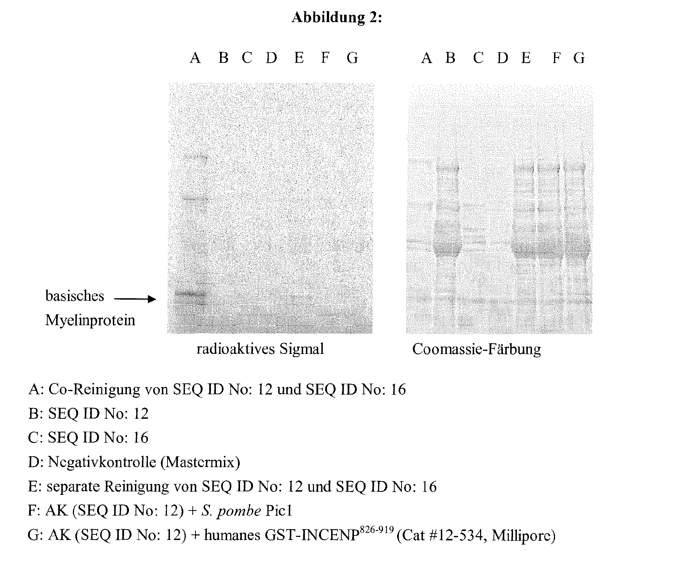

Abb. 2: Radioaktiver Aktivitätstest von (A) einer Kombination aus co-gereinigter SEQ ID No: 12 und SEQ ID No: 16, (B) SEQ ID No: 12, (C) SEQ ID No: 16, (D) Negativkontrolle (Mastermix), (E) einer Kombination aus separat gereinigter SEQ ID No: 12 und SEQ ID No: 16, (F) einer Kombination aus SEQ ID No: 12 und Pic1 aus *S*. *pombe,* (G) einer Kombination aus SEQ ID No: 12 und humanes GST-INCENP (Cat # 12-534, Millipore)

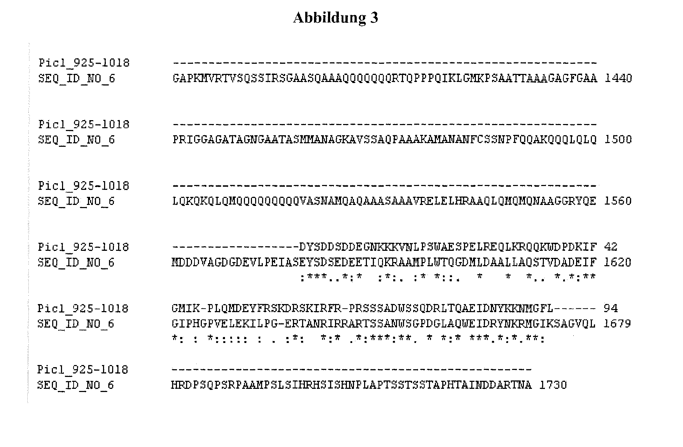

Abb 3.: Sequenzabgleich von Pic1 aus *S*. *pombe* und um03367 (SEQ ID NO: 6 = C-Terminus der SEQ ID NO: 4) ergab, dass auf einem kleinen Teil bestehend aus den Aminosäuren 925-1018 des INCENP von *S*. *pombe* und Aminosäuren 1424-1575 der SEQ ID NO: 6 eine 39%ige Identität (bezogen auf Aminosäurepositionen 925-1019 aus *S*. *pombe*) gefunden wurde.

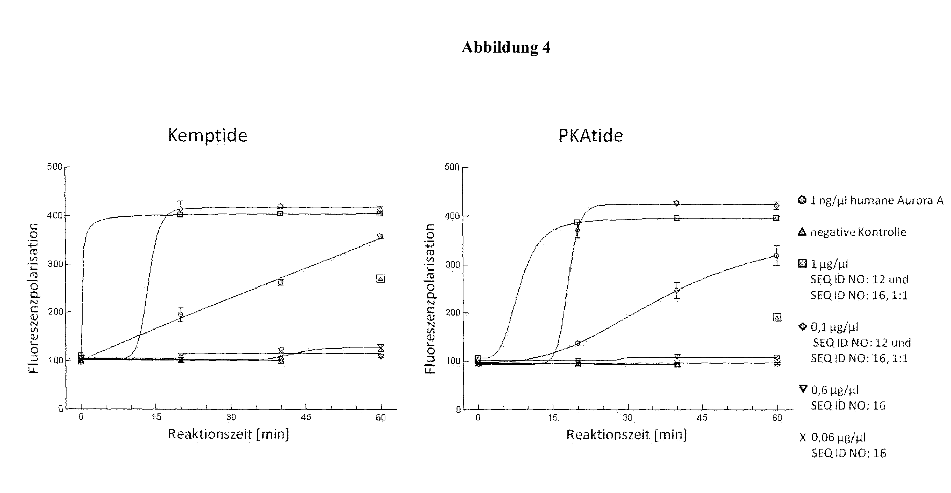

Abb 4.: Kinetik der Phosphorylierung von Substraten für humane Aurorakinase A im IMAP durch eine Kombination aus co-gereinigter SEQ ID No: 12 und SEQ ID No: 16. SEQ ID No: 16 allein zeigt keine Aktivität. Humane Aurorakinase A + humanes INCENP wurden als Positivkontrolle eingesetzt.

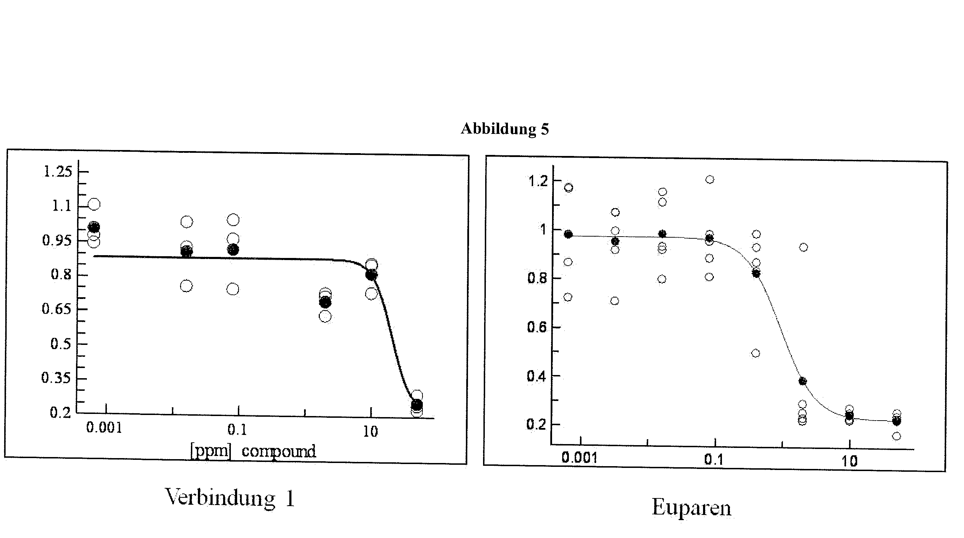

Abb. 5: Wachstumshemmung (ED₅₀-Bestimmung) von *U. maydis* mit Euparen und Verbindung 1

Der Fachmann ist sich bewusst, dass der Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann. Im Zusammenhang mit der hier offenbarten Aurorakinase sowie dem erfindungsgemäßen Aurorakinaseaktivator beziehen sich "ein" oder "eine" nicht auf die Anzahl Moleküle sondern auf die Anzahl von Molekülspezies.

Dem Fachmann ist klar, das in dieser Anmeldung genannte Beispiele nicht als beschränkend anzusehen sind sondern lediglich einige Ausführungsformen näher beschreiben.

Dem Fachmann ist offenbar, dass alle Ausführungsformen alleine oder in Kombination vorliegen können.

Nicht umfasst sind solche Kombinationen, die den Naturgesetzen widersprechen und die der Fachmann daher aufgrund seines Fachwissens ausgeschlossen hätte.

Der Begriff "umfassend" oder "umfassen" bezogen auf die vorliegende Anmeldung, spezifiziert das Vorliegen der aufgelisteten Merkmale, Integer, Schritte oder Komponenten, aber schließt das Vorhandensein eines oder mehrerer zusätzlicher Merkmale nicht aus. Demnach kann eine Nukleinsäure- oder Aminosäuresequenz mehr Nukleotide oder Aminosäuren umfassen als genannt sind, d. h. in eine längere Nukleinsäure- oder Aminosäuresequenz eingeschlossen sein. Eine Expressionskassette oder ein chimäres Gen wie an anderer Stelle beschrieben, welche(s) funktionell oder strukturell defmiert ist, kann weitere Nukleinsäuresequenzen enthalten usw. Dabei kann z. B. die Nukleinsäuresequenz am 3' oder am 5' Ende zusätzliche Nukleinsäuresequenzen enthalten, wobei die Länge der zusätzlichen Nukleinsäuresequenzen 6000 bp, zum Beispiel 3500 bp, 1500 bp, 1000 bp, 500 bp oder 250 bp am 5' und/oder 3' Ende nicht überschreitet. Solche zusätzliche Nukleinsäuresequenzen sind z. B. für ein Tag codierende Nukleinsäuresequenzen oder in Organismen vorkommende Nukleinsäuresequenzen. Im Hinblick auf eine Aminosäuresequenz kann diese zusätzliche Aminosäuresequenzen am C- und/oder N-Terminus enthalten, wobei die Länge der zusätzlichen Aminosäuresequenzen 2000, zum Beispiel 1000, 500, 250 oder100 Aminosäuren nicht überschreitet. Entsprechend sind solche zusätzlichen Aminosäuresequenzen z. B. ein Tag oder in Organismen vorkommende Aminosäuresequenz Im Zusammenhang mit der vorliegenden Anmeldung schließt der Begriff "umfassend" oder "umfassen" den Begriff "bestehend aus" oder "bestehen aus" ein.

Der Ausdruck "Fungizid" bzw. "fungizid" wirksame Verbindung wie er hierin verwendet wird, bezieht sich auf chemische Verbindungen, die zur Bekämpfung von Pilzen, insbesondere solchen, die Pflanzen, Pflanzenteile oder Pflanzenprodukte befallen und schädigen oder deren Ertrag bzw. Wert mindern, geeignet sind. Zu den genannten Pflanzenteilen gehören beispielsweise Blätter, Samen und Früchte (wie z.B. Beeren, Obst, Getreidekörner). Zu den genannten Pflanzenprodukten gehören aus den Pflanzen gewonnene Rohstoffe oder Substanzen wie beispielsweise Hölzer oder Fasern.

Der Begriff "in Kontakt Bringen" umfasst einen Kontakt zwischen der hier offenbarten, Kombination und einer oder mehreren zu untersuchenden chemischen Verbindungen in jeglicher Form unter allen Bedingungen, die eine Aktivität der in der Kombination enthaltenen erfindungsgemäßen AK gewährleisten. Für *in vitro*-Versuche mit einer Kombination in flüssiger Form (in anderen Worten, eine Lösung umfassend die erfindungsgemäße Kombination) wird/werden die zu untersuchende(n) chemische(n) Verbindung(en) der erfindungsgemäßen Kombination zugesetzt oder umgekehrt. Zum Beispiel wird/werden die zu untersuchende(n) chemische(n) Verbindung(en) einer Lösung umfassend die erfindungsgemäße Kombination in Form einer Lösung zugesetzt. Für Versuche mit der Kombination, wobei sowohl erfindungsgemäße AK als auch erfindungsgemäßer AKA in einer Wirtszelle exprimiert werden, kann die chemische Verbindung zu der Wirtszelle gegeben werden.

Eine Kombination ist jede mögliche Mischung umfassend eine erfmdungsgemäße AK und einen erfindungsgemäßen AKA. Die Kombination kann flüssig, fest oder eine Kombination daraus sein. In anderen Worten, eine erfindungsgemäße Kombination kann z. B. eine Lösung, aus Feststoffen bestehend, eine Emulsion oder Dispersion oder ein Aerosol sein. Im Zusammenhang mit dem erfindungsgemäßen Verfahren liegt die erfindungsgemäße Kombination üblicherweise in flüssiger Form vor. Zur Lagerung kann sie sowohl in flüssiger als auch in fester Form vorliegen. Eine Kombination in flüssiger Form kann z. B. nur Wasser und/oder ein organisches Lösungsmittel enthalten, jedoch auch zusätzlich dazu eines oder mehrerer Salze in unterschiedlichen Konzentrationen und/oder eine Puffersubstanz sowie weitere Hilfsstoffe wie Löslichkeitsvermittler oder Stabilisatoren.

Geeignete Salze sind dem Fachmann hinlänglich bekannt und umfassen z. B. Na-Salze wie NaCl, oder zweiwertige Metallsalze wie Mg²⁺ oder Mn²⁺ Salze (z. B. MgCl₂ oder MnCl₂).

Geeignete Puffer sind z. B. Tris(hydroxymethyl)aminomethan (TRIS), 4-(2-Hydroxyethyl)-1-piperazinethanesulfonsäure (HEPES), 4-(2-Hydroxyethyl)-piperazin-1-propansulfonsäure (HEPPS), 3(N-Morpholino)propansulfonsäure MOPS oder 2(N-Morpholino)ethansulfonsäure (MES).

Im Zusammenhang mit der vorliegenden Anmeldung beschreibt der Ausdruck "Polypeptid" (austauschbar mit dem Begriff "Protein") eine Gruppe von Molekülen, die aus mehr als 30 Aminosäuren besteht, während der Begriff "Peptid" Moleküle beschreibt, die aus bis zu 30 Aminosäuren bestehen. Polypeptide und Peptide können Dimere, Trimere oder höhere Oligomere bilden, d. h. dass die resultierenden Strukturen aus mehr als einem Polypeptid-/Peptid-Molekül bestehen. Die solche Dimere, Trimere etc. bildenden Polypeptide oder Peptide können identisch oder nicht identisch sein. Die entsprechenden Strukturen höherer Ordnung heißen somit Homo- oder Heterodimere, Homo- oder Heterotrimere usw.. Die Begriffe "Polypeptid" und "Peptid" lesen sich auch auf natürliche Weise modifizierte Polypeptide oder Peptide, z. B. durch Glycosylierung, Acetylierung, Phosphorylierung usw. Solche Modifikationen sind hinlänglich bekannt.

Die erfindungsgemäßen Polypeptide können in der Form "reifer" Proteine, z. B. mit den oben beschriebenen Modifikationen versehen, oder als Teile größerer Proteine, z.B. als Fusionsproteine, vorliegen. Fusionsproteine sind im besonderen AK oder AKA Polypeptidsequenzen, die an einen Tag gebunden sind, wie z. B. ein His-Tag, GST-Tag (Glutathion-S-Transferase) oder MBP-Tag (Maltosebindeprotein). Weiterhin können sie Sezernierungs- oder "Leader"-Sequenzen, Pro-Sequenzen, Sequenzen, die eine einfache Reinigung ermöglichen, wie mehrfache Histidin-Reste oder einen MBP-Tag, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können.

"Tag" bezeichnet ein Peptid oder Polypeptid, dessen kodierende Nukleinsäuresequenz mit der Nukleinsäuresequenz eines Polypeptids mit der Aktivität einer AK oder der Funktion eines AKA direkt oder mittels eines Linkers über gängige Klonierungstechniken fusioniert werden kann. Ein Tag kann zur Isolierung, Anreicherung, und/oder gezielten Aufreinigung eines rekombinanten (Ziel)proteins mittels Affinitätschromatographie aus, z. B. Gesamtzellextraktionen dienen und/oder zur besseren Solubilisierung von rekombinanten (Ziel)proteinen. Der oben erwähnte Linker kann vorteilhaft eine Protease Schnittstelle (z. B. für Thrombin oder Faktor Xa) enthalten, wodurch das Tag bei Bedarf vom Zielprotein abgespalten werden kann. Beispiele für gängige Tags sind "HisTag", z. B. von Qiagen, "GSTTag", z. B. von Clontech, "MBPTag", z. B. von Promega, "StrepTag", z. B. von IBA Biotagnologies, "MycTag" oder "CBDTag". Ein Tag kann dabei am 5' oder 3' Ende einer kodierenden Nukleinsäuresequenz mit der für das Zielprotein kodierenden Sequenz angebracht sein. In einer Ausführungsform der Erfindung ist eine AK und/oder ein AKA ein rekombinantes Protein (Fusionsprotein) mit einem MBPTag.

Eine AK katalysiert die Phosphorylierung eines spezifischen Proteinsubstrates, wie z. B. basisches Myelinprotein, an Serin und/oder Threonin Resten, wobei ATP als Donorsubstrat fungiert (auch als "AK-Aktivität" bezeichnet). Die enzymatische Aktivität einer AK, wie z. B. der in dieser Anmeldung beschriebenen AK, kann in einem Aktivitätstest über die Zunahme des Produktes, die (signifikante) Abnahme des Substrates (oder Eduktes) oder die Abnahme eines spezifischen Cofaktors oder über eine Kombination aus mindestens zwei der vorstehend genannten Parameter in Abhängigkeit einer definierten Zeitspanne, auch Reaktionszeit, bestimmt werden. Dem Fachmann ist bekannt, dass Proteine für ihre Aktivität/Funktion z. T. essentielle Ionen wie zweiwertige Metallionen (z. B. Mg²⁺ oder Mn²⁺) benötigen. In so einem Fall gibt der Fachmann verständlicherweise diese Ionen z. B. in Form ihrer Salze (wie MgCl₂) und in einer entsprechenden Konzentration (z. B. 5 mM, 10 mM, 20 mM, 50 mM) einer Lösung, die das entsprechende Protein, z. B. eine erfindungsgemäße AK oder AKA, enthält, vor Aktivitätsmessungen zu. Bevorzugt sind solche essentiellen Ionen bereits während der Aufreinigung und/oder während der Produktion der Proteine in den entsprechenden Medien enthalten. Entsprechend enthalten alle Lösungen in der vorliegenden Anmeldung mindestens eine zweiwertige Metallionenart wie z. B. Mg²⁺.

Dabei bezeichnet die "Reaktionszeit" die Zeit, die man für die Durchführung eines Aktivitätstests bis zum Erhalt einer signifikanten Aussage über eine Aktivität benötigt und hängt sowohl von der spezifischen Aktivität des im Test eingesetzten Proteins als auch von der verwendeten Methode und der Empfindlichkeit der verwendeten Geräte ab. Dem Fachmann ist die Ermittlung der Reaktionszeiten bekannt. Bei auf photometrischen Methoden basierenden Verfahren für die Identifizierung von Substanzen mit fungizider Wirkung liegen die Reaktionszeiten im Allgemeinen zwischen > 0 bis 600 Minuten.

Eine "signifikante Abnahme" bezogen auf die Aktivität einer AK ist eine Aktivitätsabnahme der mit einer Testverbindung versetzten AK samt AKA im Vergleich zur Aktivität der nicht mit der Testverbindung inkubierten AK, die außerhalb eines Messfehlers liegt. Zum Beispiel kann eine signifikante Abnahme eine Abnahme um mindestens 10%, mindestens 20%, mindestens 30%, mindestens 40%, mindestens 50%, mindestens 70%, mindestens 90% oder sogar um mindestens 95%, mindestens 98% oder mindestens 99% bedeuten.

Mögliche Assays zur Bestimmung einer AK-Aktivität sind z. B. die radioaktive Bestimmung nach Müller, P., et al. (2003, Eukaryot Cell, 2(6), 1187-99) wobei eine anschließende Quantifizierung z. B. durch ein AIDA-Image Analyzer erfolgen kann, kommerziell erwerbbare HTRF® Kinease Assays™ für Serin/Threonin-Kinasen (Cat #62ST0PEB. Cisbio Bioassays) oder kommerziell erhältliche IMAP Assays (Molecular Devices).

Ein Aurorakinaseaktivator (AKA) ist ein Polypeptid, welches als biologische Funktion ein Polypeptid mit der biologischen Aktivität einer AK, z. B. einer erfindungsgemäßen AK, aktivieren kann. Es ist verständlich, dass eine AKA in Kombination mit einer erfindungsgemäßen AK diese erfindungsgemäße AK aktivieren können muss. Für erfindungsgemäße AKs mit geringer Aktivität in Abwesenheit eines erfindungsgemäßen AKA kann zum Beispiel die Aktivität der erfindungsgemäßen AK bei einem Verhältnis erfindungsgemäße AK zu erfindungsgemäßen AKA von 1:1 durch den erfindungsgemäßen AKA im Vergleich zu der Aktivität derselben erfindungsgemäßen AK in Abwesenheit des erfindungsgemäßen AKA mindestens um den Faktor 2, 3, 4, 5, 10 oder 15 verbessert werden. Erfindungsgemäße AKs ohne Aurorakinaseaktivität in Abwesenheit eines korrespondierenden AKA, was sich durch das Fehlen einer radioaktiven Produktbande auf einem PAGE-Gel eines radioaktiven Assays zeigen lässt, können durch die Anwesenheit eines erfindungsgemäßen AKA aktiviert werden, d. h., sie erzeugen eine radioaktiv markierte Produktbande auf einem PAGE-Gel (Polyacrylamid-Gelelektrophorese) desselben radioaktiven Assays. Zum Beispiel kann basisches Myelinprotein (ca. 19 kDa) durch eine erfindungsgemäße AK in Anwesenheit eines erfindungsgemäßen AKA radioaktiv phosphoryliert werden, so dass ein entsprechendes PAGE-Gel, auf dass max. 500 ng pro Slot basisches Myelinprotein (Substrat des radioaktiven Assays) geladen wurde, eine radioaktive Bande bei ca. 19 kDa aufweist. Der Nachweis einer radioaktiven Bande lässt sich einfach durch die Schwarzfärbung eines Photofilm nach z. B. 50 min an der entsprechenden Stelle nachweisen. Zum Vergleich kann jederzeit eine gleiche Menge basisches Myelinprotein auf einen weiteren Slot des entsprechenden PAGE-Gel aufgetragen werden, das nicht einer Phosphorylierung unterworfen wurde, um mögliche Verfärbungen des Films im Bereich einer 19 kDa-Bande durch größere Konzentrationen des basischen Myelinproteins ausschließen zu können. Eine durch einen erfindungsgemäßen AKA aktivierte erfindungsgemäße AK kann beispielsweise eine Kinaseaktivität ungleich 0 aufweisen, d.h., es kann eine spezifische Aktivität gemessen werden, die außerhalb des Meßfehlerbereichs einer entsprechenden Methode liegt, wie z. B. mindestens 0,02 U/mg, 0,2 U/mg, mindestens 0,5 U/mg, mindestens 1 U/mg, mindestens 5 U/mg, oder sogar mindestens 50 U/mg, wobei U/mg definiert ist als diejenige Enzymmenge, die unter Standardbedingungen je min ein µmol Substrat umsetzt.

Der Ausdruck "inhibieren", wie er hierin gebraucht wird, bezeichnet die direkte oder indirekte Hemmung oder Inhibition der enzymatischen AK-Aktivität einer AK, z. B. einer mittels eines AKA aktivierten AK, durch eine chemische Verbindung. Eine solche Inhibition kann spezifisch sein, d.h. die Inhibition der Aurorakinase-Aktivität findet bei einer Konzentration einer chemischen Verbindung (eines Inhibitors) statt, die niedriger ist als die Konzentration eines Inhibitors, die benötigt wird, um ein anderes Polypeptid ohne Bezug zur AK in seiner Aktivität zu hemmen. Die zur Inhibition der AK benötigte Konzentration an Inhibitor kann beispielsweise mindestens zweifach niedriger, mindestens fünffach niedriger oder mindestens zehnfach oder mindestens 20fach niedriger sein als die Konzentration eines Inhibitors, die zum Hervorrufen eines unspezifischen Effekts benötigt wird. Eine Inhibition einer erfindungsgemäßen AK bedeutet, dass die spezifische Aktivität dieser AK bei ansonsten gleichen Bedingungen wie pH, Substrat, Substratmenge, Enzymmenge etc. in Anwesenheit eines Inhibitors mindestens um 10%, 20%, 30%, 50%, 80%, 90%, 95% geringer ist als bei Abwesenheit des Inhibitors. Der Fachmann versteht, dass eine inhibitorische Substanz z. B. im millimolaren, µmolaren oder nmolaren Bereich anwesend sein sollte.

Chemische Verbindungen beinhalten sämtliche chemisch herstellbare oder natürlich vorkommenden Moleküle, beispielsweise kleine organisch-chemische Moleküle, Peptide oder Antikörper, die an die hier beschriebene AK binden bzw. die deren Aktivität beeinflussen. Weiter umfasst sind kleine organisch-chemische Moleküle, Peptide oder Antikörper, die an ein Molekül binden, welches wiederum an die hier beschriebene AK bindet, und dadurch deren biologische Aktivität beeinflusst. Chemische Verbindungen können natürliche Substrate und Liganden darstellen oder strukturelle oder funktionelle Mimetika davon. Chemische Verbindungen schließen kleine Moleküle eine, die ein Molekulargewicht von bis zu 250 Da, bis zu 500 Da, bis zu 800 Da oder bis zu 1000 Da haben. Beispielhafte chemische Verbindungen sind N-substituierte Diaminopyrimidine wie in WO 2008/107096 beschrieben, Pyridyl-Azinylaminoderivate wie in WO 2010/055114 beschrieben, Heterocyclyl-Triazinylaminoderivate wie in WO 2010/055078 beschrieben, oder andere, potentiell fungizide chemische Verbindungsklassen.

Gemische chemischer Verbindungen sind Mischungen aus mindestens zwei unterschiedlichen chemischen Verbindungen. Im Allgemeinen kann ein Gemisch in fester Form, z. B. mindestens zwei gemischte Pulver, flüssiger Form, z. B. Lösung oder Emulsion oder einer Mischung aus beiden, z. B. Suspension, von mindestens zwei chemischen Verbindungen vorliegen. Liegt ein Gemisch als Lösung vor so ist es für das erfindungsgemäße Verfahren zur Identifizierung von Fungiziden vorzugsweise in dem gleichen Lösungsmittel / Puffersystem gelöst, wie eine Lösung, Emulsion oder Suspension aus erfindungsgemäßer AK und erfindungsgemäßem AKA

Das Vergleichen der biologischen Aktivität von erfindungsgemäßer AK in einem erfindungsgemäße Verfahren zur Identifizierung von Fungiziden kann zum Beispiel durch den direkten Vergleich von Daten, die nach einer der Methoden wie in Paragraph [0032] gewonnen wurden, bestimmt werden. Dies kann z. B. durch das Vorhandensein einer radioaktiven Bande in einem Gel erfolgen, wobei diese radioaktive Bande für ein radioaktiv markiertes Produkt einer durch AK katalysierten Reaktion steht (z. B. basisches Myelinprotein), oder durch die Auswertung von quantitativen Daten, die durch einen AIDA-Image Analyzer erhalten werden, erfolgen.

Das Bestimmen einer chemischen Verbindung aus einem Gemisch kann, z. B. durch Affinitätschromatographie und anschließende Elution und MS/MS Analyse erfolgen, wobei die erfindungsgemäße AK als stationäre Phase dient. Dem Fachmann sind Verfahren geläufig um Proteine zu immobilisieren und MS/MS Analysen durchzuführen. (Knoth et al., Angw. Chem. 2009 48, 7240-7245, Supporting Information © Wiley-VCH 2009).

Die Aminosäuresequenz der hier beschriebenen erfindungsgemäßen AK weist mindestens 70%, mindestens 80%, mindestens 90%, mindestens 95% oder mindestens 98% Sequenzidentität zu SEQ ID No: 2 auf oder zu einer Aminosäuresequenz umfassend eine SEQ ID No: 2, wie z. B. eine SEQ ID No: 2 an deren N- oder C-Terminus ein Tag fusioniert ist. Solche Aminosäuresequenzen schließen auch solche ein, die durch künstliche Mutation der codierenden Nukleinsäure, z. B. der SEQ ID No: 1, SEQ ID No 7 oder SEQ ID No: 11, entstanden sind. Im allgemeinen können die hier beschriebenen Sequenzalternativen für die erfindungsgemäße AK mindestens 70%, z. B. 72%, 74%, 76%, 78%, mindestens 80%, z. B. 81% bis 84%, mindestens 85%, z. B. 86%, 87%, 88%, 89%, 90%, 91%, 92%, 93%, 94%, 95%, 96%, 97%, 98% und 99% Sequenzidentität zur Aminosäuresequenz der SEQ ID No: 2 oder zu einer Aminosäuresequenz umfassend eine SEQ ID No: 2, wie z. B. eine SEQ ID No: 2 an deren N- oder C-Terminus ein Tag fusioniert ist, aufweisen. Die hier beschriebenen Aminosäuresequenzen können z. B. eine oder mehr als eine, z. B. 2, 3, 4, 5, oder 6 Deletionen aufweisen. Diese Deletionen können an einer oder mehrerer wie bis zu 2, bis zu 3, bis zu 4, bis zu 5, bis zu 10, bis zu 20, bis zu 50 aufeinanderfolgenden Aminosäuren an mindestens einer Stelle, z. B. je zwei, je drei, je vier, je fünf, je zehn Stellen erfolgen. Alternativ oder zusätzlich können eine oder mehr als eine, z. B. 2, 3, 4, 5, oder 6 Substitution erfolgen. Diese Substitutionen können an einer einzelnen oder mehreren Steller, wie z. B. 2, 3, 4, 5, 6, 7, 8, 9, 10 oder 20 Stellen erfolgen, wobei an mindestens einer Stelle, wie z. B. 1, 2, 3, 4, 5, oder 6, eine oder mehrere, wie z. B. 1 bis 50, 1 bis 20, 1 bis 10 oder 1, 2, 3, 4, 5, 6, 7, 8, 9, 10, 20 oder 50 aufeinanderfolgende Aminosäuren substituiert werden. Alternativ oder zusätzlich können eine oder mehr als eine, z. B. 2, 3, 4, 5, oder 6 Einfügungen einer oder mehrerer wie bis zu 2, bis zu 3, bis zu 4, bis zu 5, bis zu 10 aufeinanderfolgender Aminosäuren an mindestens einer Stelle, wie z. B. 2, 3, 4, 5, 6, Stellen enthalten. Zum Beispiel können einzelne Elemente in einer erfindungsgemäßen AK, die nicht für ihre enzymatische Aktivität und für die Bindung an den erfindungsgemäßen AKA notwendig sind, entfernt werden. Alle diese Änderungen sind so gewählt, dass die enzymatische Aktivität der erfindungsgemäßen AK im Wesentlichen nicht beeinträchtigt wird. Die Natur dieser Änderung auf Nukleinsäureebene wird an anderer Stelle beschrieben.

Unter "im Wesentlichen nicht beeinträchtigt" ist in diesem Zusammenhang zu verstehen, dass die spezifische AK-Aktivität der erfindungsgemäßen AK nicht weniger als 60%, nicht weniger als 70%, nicht weniger als 80% beträgt, bezogen auf die spezifische AK-Aktivität eines Polypeptids mit der SEQ ID No: 2.

Die Aminosäuresequenz der erfindungsgemäßen AKA weist mindestens 60%, mindestens 70%, mindestens 80%, mindestens 90%, mindestens 95% oder mindestens 98% Sequenzidentität zu SEQ ID No: 6 oder zu einer Aminosäuresequenz umfassend eine SEQ ID No: 6, wie z. B. SEQ ID No: 4 (vollständige Sequenz aus *U. maydis*), SEQ ID No: 14 (vollständige Sequenz aus *U. maydis* an deren N-Terminus ein MBPTag fusioniert ist), oder SEQ ID No: 16 (C-Terminus der Sequenz aus *U. maydis* an deren N-Terminus ein MBPTag fusioniert ist). Naturgemäß kann eine erfmdungsgemäße Aminosäuresequenz sowohl an ihrem C- und/oder an ihrem N-Terminus mit einen Tag fusioniert sein. Solche Aminosäuresequenzen schließen auch solche ein, die durch künstliche Mutation der codierenden Nukleinsäure, z. B. der SEQ ID No: 3, SEQ ID No: 5, SEQ ID No: 13 oder SEQ ID No 15, entstanden sind. Im allgemeinen können die hier beschriebenen Sequenzalternativen für den AKA mindestens 60%, z. B. 62%, 64%, 66%, 68%, mindestens 70%, z. B. 72%, 74%, 76%, 78%, mindestens 80%, z. B. 81% bis 84%, mindestens 85%, z. B. 86%, 87%, 88%, 89%, 90%, 91%, 92%, 93%, 94%, 95%, 96%, 97%, 98% und 99% Sequenzidentität zur Aminosäuresequenz der SEQ ID No: 4 oder SEQ ID No: 10 aufweisen. Die hier beschriebenen Aminosäuresequenzen können z. B. eine oder mehr als eine, z. B. 2, 3, 4, 5, oder 6 Deletionen aufweisen. Diese Deletionen können an einer oder mehrerer wie bis zu 2, bis zu 3, bis zu 4, bis zu 5, bis zu 10, bis zu 20, bis zu 50 aufeinanderfolgenden Aminosäuren an mindestens einer Stelle, z. B. je zwei, je drei, je vier, je fünf, je zehn Stellen erfolgen. Alternativ oder zusätzlich können eine oder mehr als eine, z. B. 2, 3, 4, 5, oder 6 Substitution erfolgen. Diese Substitutionen können an einer einzelnen oder mehreren Steller, wie z. B. 2, 3, 4, 5, 6, 7, 8, 9, 10 oder 20 Stellen erfolgen, wobei an mindestens einer Stelle, wie z. B. 2, 3, 4, 5, oder 6, eine oder mehrere, wie z. B. 1 bis 50, 1 bis 20, 1 bis 10 oder 2, 3, 4, 5, 6, 7, 8, 9, 10, 20 oder 50 aufeinanderfolgende Aminosäuren an substituiert werden. Alternativ oder zusätzlich können eine oder mehr als eine, z. B. 2, 3, 4, 5, oder 6 Einfügungen einer oder mehrerer wie bis zu 2, bis zu 3, bis zu 4, bis zu 5, bis zu 10 aufeinanderfolgender Aminosäuren an mindestens einer Stelle, wie z. B. 2, 3, 4, 5, 6, Stellen enthalten. Z. B. können einzelne Elemente in einem erfindungsgemäßen AKA, die nicht für eine Interaktion mit einer erfindungsgemäßen AK notwendig sind, entfernt werden. Alle diese Änderungen sind so gewählt, dass die Interaktion mit einer AK im Wesentlichen nicht beeinträchtigt wird, d.h. die spezifische AK-Aktivität einer erfindungsgemäßen AK während der Interaktion mit der erfindungsgemäßen AKA nicht weniger als 60%, nicht weniger als 70%, nicht weniger als 80% beträgt, bezogen auf die spezifische AK-Aktivität derselben erfmdungsgemäßen AK in Anwesenheit eines AKA mit der SEQ ID No: 6. Die Natur dieser Änderung auf Nukleinsäureebene wird an anderer Stelle beschrieben.

Im Zusammenhang mit der vorliegenden Anmeldung bezieht sich der Begriff "% Sequenzidentität" auf den Anteil identischer Nukleotide bzw. Aminosäuren zwischen zwei Abschnitten eines Fensters optimal alignter Nukleinsäure bzw. Aminosäuren. Optimales Alignment von Sequenzen zum Alignen eines Vergleichsfensters sind hinreichend bekannt und können mittels Hilfsmitteln wie dem lokalen Homologie-Algorithmus von Smith and Waterman (Waterman, M. S., Chapman & Hall. London, 1995), dem Homologie Alignment Algorithmus von Needleman und Wunsch (1970), der Ähnlichkeitssuche von Pearson und Lipman (1988), und durch Computer-Implementationen dieser Algorithmen wie GAP, BESTFIT, FASTA, und TFASTA durchgeführt werden, die als Bestandteil des GCG (eingetragene Marke), Wisconsin Package (eingetragene Marke von Accelrys Inc., San Diego, Calif.) erhältlich sind.

Für die Zwecke der vorliegenden Anmeldung wurde die Identität zwischen zwei Polypeptidsequenzen oder auch Nukleinsäuresequenzen durch Vergleich mit Hilfe von NCBI-Blast vom The National Center for Biotechnology Information (National Library of Medicine Building 38A Bethesda, MD 20894, USA) festgestellt.

Wie oben im einzelnen beschrieben können die erfmdungsgemäßen AKs und AKAs im Vergleich zu den entsprechenden Regionen von natürlich vorkommenden AKs bzw. AKAs Deletionen oder Aminosäuresubstitutionen aufweisen, solange sie zumindest noch die oben beschriebene Funktionalität einer AK mit der SEQ ID No: 2 bzw. AKA mit der SEQ ID No: 6 zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Ein Aspekt der vorliegenden Erfindung bezieht sich auf ein Verfahren zum Identifizieren von Fungiziden, umfassend (a) in Kontakt Bringen einer Kombination aus einem Polypeptid mit der biologischen Aktivität einer Aurorakinase (AK) und einem Polypeptid mit der biologischen Funktion eines Aurorakinaseaktivators (AKA) mit einer chemischen Verbindung oder einem Gemisch von chemischen Verbindungen, (b) Vergleichen der biologischen Aktivität der AK aus der Kombination aus (a) in Anwesenheit der chemischen Verbindung oder des Gemisches chemischer Verbindungen mit der biologischen Aktivität der AK aus der Kombination aus (a) in Abwesenheit der chemischen Verbindung oder des Gemisches von Verbindungen, und, optional, (c) Bestimmen der chemischen Verbindung aus einem Gemisch, welche die biologische Aktivität der AK inhibiert; wobei dieses Polypeptid mit der biologischen Aktivität einer AK (i) eine Aminosäuresequenz SEQ ID No: 2; oder (ii) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 2 aufweist, umfasst; und wobei ein Polypeptid mit der biologischen Funktion eines AKA (I) eine Aminosäuresequenz SEQ ID No: 6; oder (II) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 6 aufweist, umfasst.

In einer Ausführungsform liegt das Verhältnis von AK zu AKA in Schritt a) des erfindungsgemäßen Verfahrens zur Identifizierung von Fungiziden zwischen 2:1 und 1:10, zwischen 2:1 und 1:5 und bevorzugt zwischen 1:1 und 1:5 bezogen auf die Konzentration der AK und der AKA in der Kombination.

In einer weiteren Ausführungsform beträgt die Konzentration der AK im erfindungsgemäßen Verfahren mindestens 0,5 ng/µl, mindestens 1 ng/µl, mindestens 2 ng/µl.

In einer weiteren Ausführungsform liegt die Kombination aus AK und AKA in Schritt a) des erfindungsgemäßen Verfahrens in Lösung, bevorzugt in wässriger Lösung, vor, d. h. die erfindungsgemäße Kombination ist in diesem Beispiel eine Lösung umfassend eine erfindungsgemäße AK und einen erfindungsgemäßen AKA

In einer weiteren Ausführungsform umfasst ein Polypeptid mit der biologischen Aktivität einer AK eine Aminosäuresequenz SEQ ID No: 12, oder eine Aminosäuresequenz, welche mindestens 80 %, mindestens 85 %, mindestens 90 %, mindestens 95 % und insbesondere mindestens 98 % Identität mit der Aminosäuresequenz, SEQ ID No: 12 aufweist. In einer weiteren Ausführungsform besteht ein Polypeptid mit der biologischen Aktivität einer AK aus einer Aminosäuresequenz SEQ ID No: 12.

In einer weiteren Ausführungsform umfasst ein Polypeptid mit der biologischen Funktion eines AKA eine Aminosäuresequenz SEQ ID No: 4, SEQ ID No: 14 oder SEQ ID No: 16, oder eine Aminosäuresequenz, welche mindestens 80 %, mindestens 85 %, mindestens 90 %, mindestens 95 % und insbesondere mindestens 98 % Identität mit einer der Aminosäuresequenzen SEQ ID No: 4, SEQ ID No: 14 oder SEQ ID No: 16 aufweist. In einer weiteren Ausführungsform besteht ein Polypeptid mit der biologischen Aktivität eines AKA aus einer Aminosäuresequenz SEQ ID No: 4, SEQ ID No: 14 oder SEQ ID No: 16.

In einer weiteren Ausführungsform erfolgt der Vergleich der biologischen Aktivität der AK in An- bzw. Abwesenheit einer chemischen Verbindung oder des Gemisches chemischer Verbindungen mit Hilfe eines radioaktiven Assays wie z. B. in Müller, P., et al. (2003, Eukaryot Cell, 2(6), 1187-99) beschrieben, optional gefolgt von einer Quantifizierung z. B. durch ein AIDA-Image Analyzer, kommerziell erwerbbare HTRF® Kinease Assays™ für Serin/Threonin-Kinasen (Cat #62ST0PEB. Cisbio Bioassays) oder kommerziell erhältliche IMAP Assays (Molecular Devices).

Im oben beschriebenen Verfahren kann die fungizide Wirkung der in Schritt (c) bestimmten Verbindung überprüft werden, indem die Verbindung mit einem oder mehreren Pilzen in Kontakt gebracht wird. So kann zum Beispiel eine Pilzzelle, die sowohl eine AK als auch einen AKA exprimiert, auf einer Platte mit einer Lösung enthaltend die potentielle fungizide Substanz in Kontakt gebracht werden, oder eine potentiell fungizide Substanz wird in einem Gewächshaus-Test oder Feldtests untersucht, wobei Pflanzen zunächst mit einem Pilz infiziert werden und anschließend mit der potentiell fungizide Substanz behandelt werden. Diese Behandlung kann z. B. in Form einer Sprühlösung erfolgen, die auf die Pflanzen aufgesprüht wird, oder in Form von Granulat, das auf die Bodenfläche aufgetragen wird. Eine weitere Möglichkeit ist ein Blattscheibentest. Hierbei wird ein Stück aus einem Blatt ausstanzt und die Blattscheiben werden auf einer Nährlösung schwimmend mit einem entsprechenden Pilz infiziert und anschließend mit der potentiell fungizide Substanz behandelt.

Das Fungizid bzw. die als AK inhibierend identifizierte Verbindung ist gegen mindestens einen Pilz ausgewählt aus Blumeria-Arten; Podosphaera-Arten; Sphaerotheca-Arten; Uncinula-Arten; Gymnosporangium-Arten; Hemileia-Arten; Phakopsora-Arten, z. B. P. pachyrhizi und P. meibomiae; Puccinia-Arten, z. B. P. recondita, P. graminis, P. striiformis oder P. triticina; Uromyces-Arten; Albugo-Arten; Bremia-Arten; Peronospora-Arten; Phytophthora-Arten; Plasmopara-Arten; Pseudoperonospora-Arten, z. B. P. humuli oder P. cubensis; Pythium-Arten; Alternaria-Arten; Cercospora-Arten; Cladiosporum-Arten; Cochliobolus-Arten, z. B. C. sativus (Konidienform: Drechslera, Syn: Helminthosporium) oder C. miyabeanus; Colletotrichum-Arten; Cycloconium-Arten; Diaporthe-Arten; Elsinoe-Arten; Gloeosporium-Arten; Glomerella-Arten; Guignardia-Arten; Leptosphaeria-Arten; Magnaporthe-Arten; Mycosphaerella-Arten; Phaeosphaeria-Arten; Pyrenophora-Arten; Ramularia-Arten; Rhynchosporium-Arten; Septoria-Arten; Typhula-Arten; Venturia-Arten; Corticium-Arten; Fusarium-Arten; Gaeumannomyces-Arten; Plasmodiophora-Arten; Rhizoctonia-Arten; Sarocladium-Arten, z. B. Sarocladium oryzae; Sclerotium-Arten, z. B. S. oryzae oder S. rolfsii; Tapesia-Arten; Thielaviopsis-Arten; Alternaria-Arten; Aspergillus-Arten; Cladosporium-Arten; Claviceps-Arten; Fusarium-Arten; Gibberella-Arten; Monographella-Arten; Sphacelotheca-Arten; Tilletia-Arten; Urocystis-Arte; Ustilago-Arten, wie beispielsweise U. nuda, U. nuda tritici, U.maydis; Aspergillus-Arten, wie beispielsweise Aspergillus flavus; Botrytis-Arten; Penicillium-Arten, wie beispielsweise P. expansum oder P. purpurogenum; Sclerotinia-Arten; Verticilium-Arten; Alternaria-Arten; Aphanomyces-Arten; Ascochyta-Arten; Macrophomina-Arten; Microdochium-Arten; Phoma-Arten; Phomopsis-Arten; Phytophthora Arten; Typhula-Arten; Nectria-Arten; Monilinia-Arten,; Exobasidium-Arten,; Taphrina-Arten,; Esca-Arten, z. B. Phaemoniella chlamydospora, Phaeoacremonium aleophilum oder Fomitiporia mediterranea; Ganoderma-Arten; oder Helminthosporium-Arten wirksam.

Ein weiterer Aspekt der vorliegenden Erfindung bezieht sich auf die Verwendung einer Kombination eines Polypeptids mit der biologischen Aktivität einer AK und eines Polypeptids mit der biologischen Funktion eines AKA zum Auffinden von fungiziden Verbindungen wobei das Polypeptid mit der biologischen Aktivität einer AK (i) eine Aminosäuresequenz SEQ ID No: 2; oder (ii) eine Aminosäuresequenz, welche mindestens 70%, mindestens 80 %, mindestens 85 %, mindestens 90 %, mindestens 95 % und insbesondere mindestens 98 % Identität mit der Aminosäuresequenz aus (i) aufweist, umfasst; und wobei das Polypeptid mit der biologischen Funktion eines AKA '(I) eine Aminosäuresequenz SEQ ID No: 6; oder (II) eine Aminosäuresequenz, welche mindestens 60%, mindestens 70 %, mindestens 75 %, mindestens 80 %, mindestens 85 %, mindestens 90 %, mindestens 95 % und insbesondere mindestens 98 % Identität mit der Aminosäuresequenz aus I) aufweist, umfasst.

Ein weiterer Aspekt der vorliegenden Erfindung bezieht sich auf eine Nukleinsäure, die für einen AKA kodiert, wobei dieser AKA (I) eine Aminosäuresequenz SEQ ID No: 6; oder (II) eine Aminosäuresequenz, welche mindestens 60%, mindestens 65 %, mindestens 70 %, mindestens 75 %, mindestens 80 %, mindestens 85 %, mindestens 90 %, mindestens 95 % und insbesondere mindestens 98 % Identität mit der Aminosäuresequenz aus (I) aufweist, umfasst. In einer Ausführungsform besteht eine Nukleinsäure aus einer Nukleinsäure, die für einen AKA mit der SEQ ID No: 6 codiert.

Überdies bezieht sich die Erfindung auf eine Nukleinsäure umfassend eine Nukleotidsequenz, die für eine AK codiert, wobei diese AK (i) eine Aminosäuresequenz SEQ ID No: 2; oder (ii) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 2 aufweist, umfasst. In einer Ausführungsform besteht eine Nukleinsäure aus einer Nukleinsäure, die für eine AK mit der SEQ ID No: 2 codiert.

In einer Ausführungsform dieses Aspektes ist die Nukleinsäure eine Nukleinsäure aus einem pflanzenpatogenen Pilz wie z. B. *U. maydis,* d.h. in dem Beispielfall wurde die Nukleinsäure aus *U*. *maydis* isoliert.

In einer weiteren Ausführungsform ist die Nukleinsäure eine Nukleinsäure, die für einen AKA kodiert, wobei dieser AKA eine Aminosäuresequenz SEQ ID No: 4, SEQ ID No: 14 oder SEQ ID No: 16 ist, oder eine Aminosäuresequenz, welche mindestens 80 %, mindestens 85 %, mindestens 90 %, mindestens 95 % und insbesondere mindestens 98 % Identität mit einer der Aminosäuresequenzen SEQ ID No: 4, SEQ ID No: 14 oder SEQ ID No: 16 aufweist.

In einer weiteren Ausführungsform codiert die für die AK codierende Nukleinsäure eine Aminosäuresequenz umfassend die SEQ ID No: 12, in einer Ausführungsform bestehend aus SEQ ID No: 12. Eine solche codierende Nukleinsäuresequenz kann die Sequenz der SEQ ID No: 11 umfassen oder aus SEQ ID No: 11 bestehen. Weitere Ausführungsformen dieser erfindungsgemäßen Nukleinsäure sind an anderer Stelle, z. B. im Zusammenhang mit dem erfindungsgemäßen Verfahren zum Identifizieren von Fungiziden beschrieben.

Im Sinne der vorliegenden Anmeldung können Nukleinsäuren DNA oder RNA, einzel- oder doppelsträngig sein. Nukleinsäuren können chemisch synthetisiert oder durch biologische Expression in vitro oder auch in vivo hergestellt werden.

Nukleinsäuren können mittels auf geeignete Weise geschützter Ribonukleosid-Phosphoramidite und eines konventionellen DNA/RNA Synthesizers chemisch synthetisiert werden. Anbieter von RNA Synthese Reagenzien sind Proligo (Hamburg, Deutschland), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).

Im Sinn der vorliegenden Anmeldung schließt der Begriff "DNA" cDNA und genomische DNA ein.

Weiter eingeschlossen sind Nukleinsäuren imitierende Moleküle, die auch als synthetische oder semi-synthetische Derivate von DNA oder RNA und gemischte Polymere bekannt sind. Solche Nukleinsäuren imitierende Moleküle oder Nukleinsäurederivate schließen Phosphorothioat-Nukleinsäure, Phosphoramidat-Nukleinsäure, 2'-O-methoxyethyl Ribonukleinsäure, Morpholino-Nukleinsäure, Hexitol-Nukleinsäure (HNA) and "locked" Nukleinsäure (LNA) (Braasch und Corey, 2001). LNA ist einRNA Derivat, in dem der Ribosering durch eine Methylenbindung zwischen den 2'-Sauerstoff und dem 4'-Kohlenstoff gehemmt ist. Im Zusammenhang mit der vorliegenden Anmeldung kann auch eine Peptidnukleinsäure (PNA) verwendet werden. Peptidnukleinsäuren weisen ein aus sich wiederholenden Einheiten aus N-(2- aminoethy-O-glycin auf, die durch Peptidbindungen verbunden sind. Die Purin- und Pyrimidinbasen sind durch Methylen-Carbonyl-bindungen mit dem Rückgrat verbunden.

Die hier beschriebenen für einen AKA codierenden Nukleinsäuren codieren ausschließlich einen AKA, der in der Lage ist, eine AK, z. B. die hier beschriebene erfindungsgemäße AK, zu aktivieren. Dies trifft z. B. sowohl auf die durch die SEQ ID No: 5, die SEQ ID No: 3, die SEQ ID No: 13 und die SEQ ID No: 15 codierten AKAs zu als auch auf sämtliche an anderer Stelle sowie im Folgenden beschriebenen Abwandlungen der durch diese Nukleotidsequenzen codierten AKAs. Die hier beschriebenen Nukleinsäuren schließen synthetische Nukleotidsequenzen ein, z. B. solche die durch site-spezifische Mutagenese der SEQ ID No: 5, SEQ ID No: 3, SEQ ID No: 13 oder SEQ ID No: 15 hergestellt worden sind. Im allgemeinen können die hier beschriebenen Nukleotidsequenzen mindestens 70%, z. B. 72%, 74%, 76%, 78%, mindestens 80%, z. B. 81% to 84%, mindestens 85%, z. B. 86%, 87%, 88%, 89%, 90%, 91%, 92%, 93%, 94%, 95%, 96%, 97%, 98% und 99% Sequenzidentität zur Nukleotidsequenz der SEQ ID No: 5, SEQ ID No: 3, SEQ ID No: 13 oder SEQ ID No: 15 aufweisen. Sequenzidentität wird grundsätzlich in ähnlicher Weise wie oben beschrieben berechnet. Modifikationen an den hier offenbarten Nukleotidsequenzen wurden bereits an anderer Stelle beschrieben und schließen auch eine oder mehr als eine, z. B. 2, 3, 4, 5, oder 6 Deletionen, z. B. von einem Vielfachen von 3, aufeinanderfolgenden Nukleotiden an mindestens einer Stelle, eine oder mehr als eine, z. B. 2, 3, 4, 5, oder 6, Substitutionen einzelner oder mehrerer, wie z. B. 1 bis 150, 1 bis 60, 1 bis 30, aufeinanderfolgender Nukleotide an mindestens einer Stelle oder eine oder mehr als eine, z. B. 2, 3, 4, 5, oder 6, Einfügungen, z. B. von einem Vielfachen von 3 aufeinanderfolgenden Nukleotiden an mindestens einer Stelle ein. Zum Beispiel können für einzelne Elemente in einem AKA kodierende Nukleinsäureabschnitte, die einen nicht für seine Bindung an die AK notwendigen Teil der Sequenz darstellen, entfernt werden. Alle diese Änderungen sind so gewählt, dass die Bindung des AKA an die erfindungsgemäße AK im Wesentlichen nicht beeinträchtigt wird. Weitere Veränderungen schließen Veränderungen an oder Einfügen einer bestimmten Restriktionsenzymschnittstelle, die Entfernung von DNA zur Verkürzung der Sequenz, den Austausch von Nukleotiden zur Codon Optimierung oder das Hinzufügen weiterer Sequenzen oder funktioneller Elemente ein. Zur Herstellung solcher Varianten benötigte Technik sind allgemein bekannt (siehe, z. B., J. F. Sambrook, D. W. Russell, and N. Irwin, 2000).

Beispielhaft können Modifikationen an Nukleinsäuren durch dem Fachmann geläufige Techniken, wie "Site Directed Mutagenesis", "Error Prone PCR", "DNA shuffling" (Nature 370,1994, pp. 389391) oder "Staggered Extension Process" (Nature Biotechnol. 16,1998, pp. 258261) erzeugt werden..

Die hier beschriebene Nukleinsäure kann eine Reihe von Nukleotidsequenzen umfassen, unter anderem eine Nukleotidsequenz ausgewählt aus (a) der Nukleotidsequenz gemäß SEQ ID No: 5, wie z. B. SEQ ID No: 3, SEQ ID No: 13 oder SEQ ID No: 15; (b) einem Fragment bestehend aus mindestens 15 bis 90 aufeinanderfolgenden Nukleotiden der Nukleotidsequenz aus (a) codierend für ein Peptid oder mindestens 91 aufeinanderfolgenden Nukleotiden der Nukleotidsequenz aus (a) codierend für ein Polypeptid; (c) Nukleotidsequenzen, welche an die unter a) oder b) defmierten Sequenzen bei einer Hybridisierungstemperatur von 20 °C bis 65 °C hybridisieren; (d) Nukleotidsequenzen, welche mindestens 60 %, mindestens 65 %, mindestens 70 %, mindestens 75 %, mindestens 80 %, mindestens 85 %, mindestens 90 %, mindestens 95 % und insbesondere mindestens 98 % Identität zu den unter a) bis c) definierten Sequenzen aufweisen, wobei die durch die Nukleinsäure kodierten Polypeptide AKA Funktion aufweisen (e) Nukleotidsequenzen, welche zu den unter a) bis d) definierten Sequenzen komplementär sind; und (f) Nukleotidsequenzen, welche aufgrund der Degeneriertheit des genetischen Codes für dieselbe Aminosäuresequenz codieren wie die unter a) bis e) defmierten Sequenzen.

Beispielhafte Polynukleotide sind Nukleotidsequenzen der SEQ ID No: 3 oder SEQ ID No: 5. In anderen Beispielen besteht das Polypeptid aus der Aminosäuresequenz der SEQ ID No: 13 oder SEQ ID No:15.

Nukleinsäurefragmente mit mindestens 15, aufeinanderfolgenden Nukleotiden der Nukleotidsequenz aus SEQ ID No: 5, wie z. B. SEQ ID No: 3, SEQ ID No: 13 oder SEQ ID No: 15, bevorzugt SEQ ID No: 3 oder SEQ ID No: 5, sind so ausgewählt, dass sie als spezifische Primer oder Proben in der Amplifikation oder dem Nachweis von Sequenzen der hier beschriebenen AKA verwendet werden können, wie z. B. Fragmente von 15 bis 40, von 18 bis 40, von 20 bis 40 aufeinanderfolgenden Nukleotiden. Die Nukleinsäurefragmente können auch mindestens 91, mindestens 100, mindestens 120, mindestens 150, mindestens 200, mindestens 300, mindestens 400, mindestens 500 aufeinanderfolgende Nukleotide aufweisen. Nukleinsäurefragmente, die Polypeptide codieren, sind so gewählt, dass der durch sie codierte AKA seine Fähigkeit an eine AK, z. b. die hier beschriebene AK zu aktivieren behält.

Der Begriff Hybridisierung bezieht sich auf die Fähigkeit eines ersten Nukleinsäurestrangs, über Basenpaarung über Wasserstoffbrückenbindungen an einem zweiten Nukleinsäurestrang zu binden, wenn beide Nukleinsäurestränge ausreichend komplementär sind. Hybridisierung findet statt, wenn die beiden Nukleinsäurestränge unter geeigneten Bedingungen aneinander binden (annealing). Nukleinsäurehybridisierung ist dem Fachmann in Nukleinsäuermanipulationstechniken hinreichend bekannt. Die Hybridisierungseigenschaften eines bestimmten Paares von Nukleinsäuresträngen ist ein Hinweis auf ihre Komplementarität. Ein anderer Hinweis, dass zwei Nukleinsäuresequenzen überwiegend komplementär sind, ist ihre Fähigkeit, unter stringenten Bedingungen aneinander zu hybridisieren. Stringente Hybridisierungsbedingungen und stringente Hybridiserungs-Waschbedingungen im Zusammenhang mit Nukleinsäurehybridisierungsexperimenten wie Southern und Northern Hyridisierung sind sequenzabhängig und unterscheiden sich je nach den gewählten Bedingungen, sind jedoch auch abhängig von den beteiligten Nukleinsäuren. So liegen beispielsweise die Schmelztemperaturen für DNA : DNA-Hybride ca. 10 °C niedriger als die von DNA : RNA-Hybriden gleicher Länge.

Unter stringenten Hybridisierungsbedingungen sind beispielsweise je nach Nukleinsäure Temperaturen zwischen 20 °C und 65 °C, in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50 % Formamid zu verstehen. Vorteilhafterweise liegen die Hybridisierungsbedingungen für DNA : DNA-Hybride bei 0,1 x SSC und Temperaturen zwischen etwa 20 °C bis 65 °C, bevorzugt zwischen etwa 30 °C bis 45 °C.

Für DNA : RNA-Hybride liegen die Hybridisierungsbedingungen vorteilhaft bei 0,1 x SSC und Temperaturen zwischen etwa 30 °C bis 65 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA Hybridisierung sind in einschlägigen Lehrbüchern der Genetik wie beispielsweise Sambrook et al.,"Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen : Ausubel et al. (eds), 1985, Current Protocols in Molecular Biology, John Wiley & Sons, New York ; Hames and Higgins (eds), 1985, Nucleic Acids Hybridization : A Practical Approach, IRL Press at Oxford University Press, Oxford ; Brown (ed), 1991, Essential Molecular Biology : A Practical Approach, IRL Press at Oxford University Press, Oxford.

Ein Beispiel für hoch stringente Wasch-Bedingungen beinhaltet Waschen mit 0.15 M NaCl bei 72°C für ca. 15 Minuten. Ein Beispiel für stringente Waschbedingungen umfasst Waschen bei 0.2 X SSC bei 65°C für 15 Minuten. Oftmals geht einer Waschung unter hoch stringenten Bedingungen ein Waschung unter niedrigen Stringenzbedingungen voraus, um Hintergrundsignal durch die Probe zu entfernen. Für kurze Proben (z. B. 10 bis 50 Nukleotide) beinhalten stringente Bedingungen typischerweise Salzkonzentrationen von weniger als 1.5 M, z. B. ca. 0,01 bis 1,0 M, eine Natrium Ionenkonzentration (oder andere Salze) bei pH 7,0 bis 8,3, die Temperatur liegt typischerweise bei mindestens 30°C und bei mindestens 60°C bei langen Proben (z. B. > 50 Nukleotide). Stringente Bedingungen können auch durch Zusatz destabilisierender Agenzien wie Formamid erhalten werden. Im allgemeinen deutet ein Signal-Rauschen Verhältnis von 2X (oder höher) als das mit einer Probe ohne Bezug erhaltene in einem bestimmten Hybridisierungsassay auf spezifische Hybridisierung hin. Als sehr stringente Bedingungen wird eine Temperatur gewählt, die der Schmelztemperatur der Probe entspricht.

Demnach umfassen erfmdungsgemäße Nukleinsäuresequenzen auch solche, die unter Standardbedingungen mit der SEQ ID No: 1, SEQ ID No: 3, SEQ ID No: 5, SEQ ID No: 11, SEQ ID No: 13, SEQ ID No: 15, oder jeweils Fragmenten der SEQ ID Nos mit mindestens 15 aufeinanderfolgenden Nukleotiden hybridisieren und befähigt sind die Expression eines Polypeptides mit Aktivität einer AK bzw. Funktion eines AKA zu bewirken. Wird die Aktivität einer AK ausgeschaltet, so sind die resultierenden Transformanden mindestens nicht wachstumsfähig.

Die Degeneriertheit des genetischen Codes beruht auf der mehrfachen Abdeckung einiger der 20 in Tier- und Pflanzenzellen synthetisierten Aminosäuren durch zwei oder mehr der 64 möglichen Basentripletts. Für eine bestimmte Aminosäure codierende Tripletts unterscheiden sich häufig nur in der dritten, ab und zu (auch) in der zweiten Base. Daher können diese Positionen bei Kenntnis der Alternativen Tripletts ausgetauscht werden, z. B. auch um eine Codon-Optimisierung der resultierenden Nukleinsäure zur Expression in bestimmten Organismen zu erreichen.

Die vorliegende Anmeldung bezieht sich in einem weiteres Aspekt außerdem auf eine Expressionskassette umfassend eine oben beschriebene Nukleinsäure und einen Promoter.

Der Promoter wird ausgewählt abhängig vom Organismus, in dem die Nukleinsäure exprimiert werden soll und umfasst in Bakterien, Pilzen, Viren, Pflanzen oder Tieren exprimierbare Promoter. Hierzu kann jede als Promoter in den o. g. Organismen verwendbare Nukleinsäuresequenz dienen, einschließlich der in diesen Organismen natürlich vorkommenden Promoter. Der Promoter kann konstitutiv aktiv oder induzierbar sein, z. B. kann die Expression unter der Kontrolle eines lac-Promotors durch IPTG (Isopropyl-β-D-thiogalactopyranosid) induziert werden. Bevorzugt sind Promotoren aus Bakterien, Bakteriophagen oder Viren wie lac, insbesondere lacZ, trp, recA, nar, T7, VHb, T3-lac, CaMV, RSV-Promotoren.

Die Expressionskassette kann noch weitere regulatorische Elemente enthalten, welche zwischen dem Promoter und der codierenden Nukleinsäure liegen, wie z. B. Transkriptionsaktivatoren ("enhancers"), wie z. B. der Transkriptionsaktivator des Tabakmosaikvirus (TMV) (beschrieben in WO 87/07644), oder des "tobacco etch virus" (TEV) (Carrington und Freed, 1990, J. Virol. 64: 1590-1597), oder Introns wie das adh1 Intron aus Mais or Intron 1 des Aktin-Gens aus Reis.

Die Expressionskassette kann weiterhin eine Transkriptionsterminations- oder Polyadenylierungssequenz enthalten. Hierbei kann jede entsprechende Sequenz aus Bakterien, wie z. B. der nos Terminator aus Agrobacterium tumefaciens, Viren, wie z. B. der CaMV 35S Terminator, oder Pflanzen, wie z. B. ein in EP 0 633 317 beschriebener Histon Terminator verwendet werden.

Die Aktivität oder Stärke eines Promoters kann in Form der von ihm produzierten RNA-Menge oder des produzierten Proteins in einer Zelle oder einem Gewebe gemessen und mit einem anderen Promoter verglichen werden, dessen Aktivität zuvor bestimmt wurde.

Ein weiterer Aspekt ist ein Vektor umfassend eine oben beschriebene Nukleinsäure oder eine oben beschriebene Expressionskassette.

Ein Vektor schließt jedes Nukleinsäure-basierte Agens ein, das in der Lage ist, genetische Information zu tragen und zu übertragen, wie z. B. ein Plasmid, Cosmid, virus, autonom replizierende Sequenz, Phage, oder linear einzelstängige, zirkular einzelsträngige, linear doppelsträngige oder zirkular doppelsträngige DNA oder RNA Nukleotidsequenz. Ein (rekombinanter) Vektor kann aus einer beliebigen Quelle abgeleitet sein und ist in der Lage, ins Genom zu integrieren oder autonom zu replizieren.

Ein rekombinanter Vektor enthält typischerweise, in 5'-3'-Richtung: einen Promoter zur Vermittlung der Transkription einer Nukleinsäuresequenz und eine zu transkribierende Nukleinsäuresequenz. Diese Elemente entsprechen der hier offenbarten Expressionskassette. Der rekombinante Vektor kann weiterhin einen 3' Transkriptionsterminator, ein 3' Polyadenylierungssignal, andere nicht translatierte Nukleinsäuresequenzen, Transit- und Targeting-Nukleinsäure, selektierbare Marker, Enhancer und Operatoren nach Wunsch umfassen. Der Begriff 5' UTR bezieht sich auf die untranslatierte DNA stromaufwärts, oder 5' der codierenden Region eines Gens, 3' UTR beschreibt die untranslatierte DNA stromabwärts, oder 3' der codierenden Region eines Gens. Mittel zur Herstellung rekombinanter Vektoren sind hinlänglich bekannt. Mögliche Vektoren sind z. B. pENTRTM/TEV/D-Topo® (Invitrogen, Cat #45-0228, Lot #792341), pENTRTM/SD/D-Topo®, pRU1 (Brachmann et al. Mol. Microbiol., 2001, 42,(4), 1047-1063), pDEST15, pDEST17, pDEST43 (Invitrogen).

Ebenfalls von dieser Anmeldung umfasst ist eine Wirtszelle enthaltend eine erfindungsgemäße Nukleinsäure, eine oben beschriebene Expressionskassette oder den oben beschriebenen Vektor.

Eine Wirtszelle kann jegliche prokaryotische oder eukaryotische Zelle sein, einschließlich Bakterienzellen, Pilzzellen, Pflanzenzellen (inklusive Gewebe oder ganze Pflanzen) oder tierische Zellen. Neben der hier offenbarten für einen AKA codierenden Nukleinsäure oder der entsprechenden Expressionskassette oder dem Vektor kann die Wirtszelle noch eine weitere, für die hier offenbarte AK codierende Nukleinsäure enthalten, entweder in einem separaten Vektor oder in demselben Vektor, der auch die für den AKA codierende Nukleinsäure enthält.

Wirtszellen aus Säugetieren schließen die menschlichen Zelllinien Hela, 293, H9, SH-EP1 Jurkat, Maus-Zelllinien NIH3T3 und C2C12, Cos 1, Cos 7 and CV1, quail QC1-3, L Zellen, Syrian golden baby hamster kidney (BHK) Zellen und Chinese hamster ovary (CHO) Zellen ein. Als Wirtszellen verwendbare Bakterienzellen schließen E. coli-Stämme, wie z. B. von BL21 (wie BL21(DE3), BL21(DE3)PlysS, BL21(DE3)RIL, BL21 (DE3)PRARE) oder Rosetta® abgeleitete Zellen, Streptomyces Stämme und Salmonella typhimurium Zellen ein. Auch Pilzzellen wie Hefen oder Insektenzellen z. B. aus Drosophila S2 and Spodoptera Sf9 Zellen, oder Pflanzenzellen können verwendet werden. Entsprechende Kulturmedien und -bedingungen für diese Wirtszellen sind dem Fachmann bekannt.

Ein weiterer Aspekt der vorliegenden Anmeldung bezieht sich überdies auf ein Polypeptid, welches durch die oben beschriebene Nukleinsäure codiert wird.

Beispielhafte Polypeptide umfassen die Aminosäuresequenz der SEQ ID No: 4 oder SEQ ID No: 6. In anderen Beispielen besteht das Polypeptid aus der Aminosäuresequenz der SEQ ID No: 14 oder SEQ ID No:16.

In einer Ausführungsform besteht ein Polypeptid aus einer Aminosäuresequenz der SEQ ID No: 4, SEQ ID No: 6, SEQ ID No: 14 oder SEQ ID No:16. Analog dazu besteht in einer Ausführungsform eine erfindungsgemäße Nukleinsäure aus einer Nukleotidsequenz der SEQ ID No: 3, SEQ ID No: 5, SEQ ID No: 13 oder SEQ ID No:15. Auch die an anderer Stelle beschriebenen Varianten werden vom erfindungsgemäßen Polypeptid umfasst.

Beispielhafte Polypeptide können durch eine Vielzahl dem Fachmann bekannte Verfahren überexprimiert und aufgereinigt werden. Dem Fachmann sind Aufreinigungsverfahren wie z. B. chromatographische Verfahren, Gelfiltration, Kristallisation oder Dialyse wohl bekannt. Eine einfache Aufreinigung von erfindungsgemäßen Fusionspolypeptiden, die einen Tag wie einen MBPTag besitzen, kann durch Affinitätschromatographie erfolgen. Nach erfolgter Aufreinigung kann ein Tag optional abgespalten werden. Z. B. kann ein entsprechender Tag, sofern er durch eine Linkersequenz mit entsprechender Erkennungssequenz mit dem AKA Polypeptid verbunden ist, mit AcTEV-Protease (Invitrogen, Cat. No. 12575-015) abgespalten werden. Der Ausdruck "aufgereinigt" bzw. "gereinigt" bezieht sich auf eine Verringerung von anderen Verbindungen wie anderen Proteinen oder Zellteilen aus einer Lösung enthaltend ein erfindungsgemäßes Polypeptid mit der biologischen Funktion eines AKA, ein erfindungsgemäßes Polypeptid mit der biologischen Aktivität einer AK oder eine Kombination dieser beiden Polypeptide, so dass die Menge an anderen Verbindungen im Verhältnis zu der Menge an erfindungsgemäßen Polypeptiden in einer Lösung oder einem Feststoff durch einen oder mehrere Aufreinigungsschritte, mindestens um einen Faktor 2, um einen Faktor 5, um einen Faktor 10, um einen Faktor 50 verringert wird. Bevorzugt liegt die Menge an anderen Verbindungen nach einem oder mehreren Aufreinigungsschritten unter 10%, unter 1%, unter 0,1 %, unter 0,01 % bezogen auf die Menge an erfindungsgemäßen Polypeptiden.

Ein weitere Aspekt der vorliegenden Anmeldung bezieht sich auf eine Kombination aus einem gereinigten erfindungsgemäßen Polypeptid mit der biologischen Aktivität einer AK und einem gereinigten erfindungsgemäßen Polypeptid mit der biologischen Funktion eines AKA

Entsprechend bezieht sich ein Aspekt auf eine Kombination umfassend
a) ein gereinigtes Polypeptid mit der biologischen Funktion eines AKA, welche von einer Nukleinsäure gemäß einem der Ansprüche 7 bis 9 kodiert wird, und
b) ein gereinigtes Polypeptid mit der biologischen Aktivität einer AK, wobei die AK
   i) eine Aminosäuresequenz SEQ ID No: 2 umfasst; oder
   ii) eine Aminosäuresequenz, welche mindestens 70%, mindestens 80 %, mindestens 85 %, mindestens 90 %, mindestens 95 % und insbesondere mindestens 98 % Identität mit der Aminosäuresequenz ais i) aufweist, umfasst.

Die für andere Aspekte der vorliegenden Erfindung, insbesondere das Verfahren zum Identifizieren von Fungiziden, beschriebenen Ausführungsformen sind auch auf die erfindungsgemäße Kombination anwendbar.

In einer Ausführungsform umfasst ein gereinigtes Polypeptid mit der biologischen Funktion eines AKA eine Aminosäuresequenz ausgewählt aus
I) einer Aminosäuresequenz SEQ ID No: 6; oder
II) einer Aminosäuresequenz, welche mindestens 60%, mindestens 70 %, mindestens 75 %, mindestens 80 %, mindestens 85 %, mindestens 90 %, mindestens 95 % und insbesondere mindestens 98 % Identität mit der Aminosäuresequenz aus I) aufweist.

Beispiele für eine AKA umfassend eine Aminosäuresequenz ausgewählt aus SEQ ID No: 6 sind Aminosäuresequenzen SEQ ID No: 4, SEQ ID No: 14 und SEQ ID No: 16.

In einer weiteren Ausführungsform besteht die Kombination aus erfindungsgemäßen AK und AKA aus Feststoffen. Sie kann z. B. in Form von lyophilisierten AK und AKA vorliegen.

Weitere Ausführungsformen beziehen sich auf Kombinationen aus AK und AKA in Form von Lösungen, Emulsionen oder Suspensionen.

In einer weiteren Ausführungsform liegt die Kombination aus AK und AKA als Lösung, bevorzugt als wässrige Lösung, vor.

In einer weiteren Ausführungsform beträgt die Konzentration der AK in einer Kombination in Form einer, bevorzugt wässrigen, Lösung mindestens 0,5 ng/µl, mindestens 1 ng/µl, mindestens 2 ng/µl.

In einer weiteren Ausführungsform liegt das Verhältnis von AK zu AKA zwischen 2:1 und 1:10, zwischen 2:1 und 1:5 und bevorzugt zwischen 1:1 und 1:5 bezogen auf die Konzentration der AK und der AKA in der Kombination liegen.

Eine Kombination aus AK und AKA kann zusätzlich Salze wie NaCl, zweiwertige Metallsalze wie Mg-Salze (z. B. MgCl₂) oder zweiwertige Mn-Salze (z. B. MnCl₂) und/oder Puffersubstanzen wie TRIS, HEPES, HEPPS, MOPS oder MES enthalten.

Ein weiterer Aspekt der vorliegenden Anmeldung bezieht sich auf Antikörper, welche spezifisch an einen erfindungsgemäßen AKA binden können. Dem Fachmann ist bewusst, wie Antikörper nach allgemein bekannten Verfahren hergestellt werden können.

Der Begriff "Antikörper" umfasst z. b. polyklonale oder monoklonale Antikörper. Weiter umfasst sind deren Derivate oder Fragmente, die noch ihre Bindespezifität beibehalten haben. Derivate oder Fragment umfassen unter anderem Fab or Fab' Fragmente, Fd, F(ab')2, Fv oder scFv Fragmente (siehe z. B. Harlow und Lane "Antibodies, A Laboratory Manual", Cold Spring Harbor Laboratory Press, 1988 und Harlow and Lane "Using Antibodies: A Laboratory Manual" Cold Spring Harbor Laboratory Press, 1999). Der Begriff "Antikörper" umfasst ebenfalls Ausführungsformen wie Chimäre (humane konstante Domäne, nicht humane variable Domäne), einkettige Antikörper und humanisierte Antikörper (humaner Antikörper, der nicht-humande CDRs enthält).

Techniken zur Herstellung von Antikörper sind wohl bekannt und z. B. beschrieben in Harlow und Lane (1988) und (1999), loc. cit. Dementsprechend können Antikörper über Peptidomimetika hergestellt werden. Weiterhin können für die Herstellung von einkettigen Antikörpers beschriebene Techniken (siehe z. B. US Patent 4,946,778) zur Herstellung von einkettigen Antikörpern, die die erfindungsgemäßen Polypeptide spezifisch binden, abgewandelt werden. Auch transgene Tiere oder Pflanzen (siehe z. B. US Patent 6,080,560) können zur Expression der erfindungsgemäßen Antikörper verwendet werden. Zur Herstellung monoklonaler Antikörper kann jegliche Technik, die antikörper durch kontinuierliche Zell-Kulturen zur Verfügung stellt, verwendet werden (siehe z. B. Harlow und Lane (1988) und (1999), loc. cit.). Dies schließe die Hybridom-Technik ( Köhler and Milstein Nature 256 (1975), 495-497) und die Triom-Technik ein.

Ein weiterer Aspekt bezeiht sich auf ein Verfahren zum Herstellen einer erfindungsgemäßen Kombination aus AK und AKA umfassend
a) Exprimieren eines AKA von einer Nukleinsäure, die für einen erfindungsgemäßen AKA kodiert;
b) Exprimieren einer AK von einer Nukleinsäure, die für eine erfindungsgemäße AK kodiert; und
c) Gewinnung der AK- und AKA-Polypeptide aus a) und b).

In einer Ausführungsform liegen die Nukleinsäuren aus a) und b) in derselben Wirtszelle vor, d.h., eine Wirtszelle enthält sowohl mindestens eine Nukleinsäure, die für einen erfindungsgemäßen AKA kodiert als auch mindestens eine Nukleinsäure, die für eine erfindungsgemäße AK kodiert. Dabei kann eine Wirtszelle z. B. entweder einen Vektor umfassend beide Nukleinsäuren, wahlweise integriert in ein an anderer Stelle beschriebenes Expressionskonstrukt oder zwei Vektoren umfassend je eine Nukleinsäure oder je ein Expressionskonstrukt enthalten.

Die Gewinnung von AK- und/oder AKA-Polypeptiden erfolgt im Fall von der Benutzung von Wirtszellen unter a) und b) durch Lyse derselben. Der Begriff Lyse umfasst sowohl chemische (z. B. durch Lysozym) als auch mechanische Zerstörung (z. B. Ultraschall) von Zellen. Wahlweise können die erfindungsgemäßen Polypeptide nach erfolgter Lyse weiter durch Standardverfahren wie z. B. Affinitätschromatographie aufgereinigt werden. Dabei werden die erfindungsgemäßen Polypeptide in dieser Situation Co-Aufgereinigt. Das Co-Aufreinigen führt überraschenderweise zu einer höheren Aktivität der AK im Vergleich zu der gleichen AK, zu der der AKA erst nach erfolgter Reinigung zugegeben wurde. Die Erhöhung der Aktivität liegt, z. B. bei einem Faktor 3, 6, 8, 10.

In einer weiteren Ausführungsform werden die Nukleinsäuren aus a) und b) separat in Wirtszellen exprimiert, d. h. eine Wirtszelle enthält einen Vektor umfassend eine Nukleinsäure, die für eine erfindungsgemäße AK kodiert, oder ein eine solche Nukleinsäure enthaltendes Expressionskonstrukt, und eine andere Wirtszelle enthält einen Vektor umfassend eine Nukleinsäure, die für einen erfindungsgemäßen AKA kodiert, oder ein eine solche Nukleinsäure enthaltendes Expressionskonstrukt. In einer Ausführungsform können die beiden AK und AKA exprimierenden Wirtszellen während der Expression (Schritte a) und b)) bereits in einer Mischung vorliegen, d.h. die Gewinnung von AK und AKA erfolgt aus zuvor vereinigten Wirtszellen. Alternativ können die Wirtszellen auch erst nach der Expression und vor Beginn der Gewinnung, z. b. durch Aufreinigung, vereinigt werden. Die Wirtszellen können nach erfolgter Expression der erfindungsgemäßen Polypeptide zusammen in einem Medium z. B. lysiert werden (Schritt c)) um eine wahlweise Aufreinigung der erfindungsgemäßen Polypeptide als Co-Aufreinigung durchzuführen, oder die Lyse und spätere wahlweise Aufreinigung der erfindungsgemäßen AK-Polypeptide und AKA-Polypeptide kann getrennt erfolgen und die beiden Polypeptide werden erst danach vereinigt.

Dem Fachmann ist klar, dass Schritt a) und b) das Kultivieren von Wirtszellen unter Bedingungen, die die Expression der erfindungsgemäßen Nukleinsäure gewährleisten, umfasst.

In einer weiteren Ausführungsform erfolgt die Expression in einem *in vitro* System.

Die Gewinnung der AK- und AKA-Polypeptide kann z. B. in einem *in vitro* System von Applied Biosystems (Cat#AM1200; Product Name: Retic Lysate IVT™ Kit) (Kozak; Nuc. Acid Res. (1990); 18; 2828) oder einem in vitro System von Invitrogen (Cat#K9901-00; Product Name: Expressway™ Mini Cell-Free Expression System (Savasaki et al.; PNAS (2002); 99; 14652-14657) erfolgen.

Die Aufreinigung der erfindungsgemäßen Polypeptide kann nach Standardmethoden wie z. B. Affinitätschromatographie oder Gelelektrophorese, erfolgen, wie in dieser Anmeldung beschrieben.

Die nach diesem Verfahren hergestellte Kombination aus AK und AKA kann z. B. als Kombination in einem erfindungsgemäßen Verfahren zur Identifizierung von Fungiziden eingesetzt werden.

Ein weiterer Aspekt der vorliegenden Erfindung ist die Bereitstellung eines Kits.

Ein Kit enthält ein erfindungsgemäßes Polypeptid mit der biologischen Aktivität einer AK und ein erfindungsgemäßes Polypeptid mit der biologischen Funktion eines AKA

Die erfindungsgemäßen Polypeptide können in einem Kit getrennt voneinander in isolierten Containern oder in Kombination in einem Container vorliegen. Bevorzugt liegen die erfindungsgemäßen Polypeptide in Kombination vor. Die Kombination kann wie bereits in der Anmeldung beschrieben in Lösung oder als Feststoffe vorliegen. Ferner kann sie in Form einer "ready for use"-Lösung oder einer "Stock"-Lösung, die 2x, 5x, 10x, 20x, 50x, oder 100x konzentriert ist, vorliegen. Ferner kann ein Kit eine Gebrauchsanweisung zum Durchführen eines erfindungsgemäßen Verfahrens zur Identifizierung von Fungiziden enthalten.

### Allgemeine Methoden

### Assays zur Aktivitätsbestimmung von Aurorakinasen:

Assay 1: Radioaktive Aktivitätsbestimmung nach Müller, P. et al. (2003), Eukaryot Cell, 2(6), 1187-1199). Substrat: Myelinprotein. Eine anschließende Quantifizierung kann durch ein AIDA-Image Analyzer (Version 4.13.023) erfolgen.

Zum Start des Aktivitätstests wurden 2x Mastermix und Enzymlösung in 1X Kinasepuffer + 1 mM DTT im Verhältnis 1:1 gemischt. Die Messung erfolgte über 45 min. bei RT. Danach wurde der Ansatz mit dem gleichen Volumen SDS-Probepuffer versetzt, 5 min bei 95°C inkubiert.
2X Mastermix
100 ng/µl basisches Myelinprotein
100 µM ATP
0.08 µCi/µl [γ-²²P]ATP
1 mM DTT
in 1X Kinasepuffer

10X Kinasepuffer
500 mM Hepes pH 7.5
100 mM MgCl₂
10 mM EGTA
0,1% Brij-35

Assay 2: Die Aktivität von Kinasen kann ebenfalls an 3 verschiedenen Substraten des kommerziell erhältlichen HTRF® Kinease Assays™ für Serin/Threonin-Kinasen (Cat #62ST0PEB. Cisbio Bioassays) bestimmt werden.

Assay 3: Für den kommerziell erhältlichen IMAP Assay (Molecular Devices) sind 2 Substrate für humane Aurorakinasen (Kemptide, Cat #R7331, Sequenz 5TAMRA-LRRASLG-OH und PKAtide, Cat #R7250, Lot #130188, Sequenz 5FAM-GRTGRRNSI-NH2) kommerziell erwerbbar. Der Assay wurde nach Herstellerangaben durchgeführt.
IMAP Progressive Binding Reagent: Cat #R7284, Lot #143859
IMAP Progressive Binding Buffer A: Cat #R7285, Lot #42898
IMAP 5x Reaction Buffer: Cat #R7206, Lot #33060

### IMAP Fluoreszenz-Polarisations-Assay (Assay 3)

Der IMAP Fluoreszenz-Polarisations-Assay (Molecular Devices) beruht auf der spezifschen, hochafmen Interaktion zwischen Phosphatgruppen und trivalenten Metallionen. Durch die Kinasereaktion wird ein fluoreszentes Substrat phosphoryliert, welches daraufhin von den in der Stop-Lösung enthaltenen Metall-Kügelchen spezifisch gebunden wird. Durch diese Interaktion erhöht sich die Fluoreszenzpolarisation, was die Bestimmung der Kinaseaktivität ermöglicht. Der Assay wurde nach Angaben des Herstellers wie folgt ausgeführt:
1 Vol. Inhibitor in 4% DMSO bzw. 4% DMSO
1 Vol. Enzymlösung
2 Vol. ATP/Substrat
12 Vol. Stoplösung
Inkubation bei RT 30 min.

Enzymlösung: z. B. SEQ ID NO: 12 und SEQ ID NO: 16 (1:1) in Complete Reaction Buffer ATP / Substrat: 70 µM ATP / 200 nM FAM-PKAtide oder TAMRA-Kemptide in Complete Reaction Buffer
Complete Reaction Buffer
1 Vol 5x IMAP Reaction Buffer (Molecular Devices)
4 Vol H₂O
1 mM DTT
0,01 % Tween-20

Stoplösung
1/400 Vol Progressive Binding Reagent (Molecular Devices) in Progressive Binding Buffer A
(Molecular Devices)

Die Messung der Fluoreszenzpolarisation erfolgte in einem Tecan Ultra Mikrotiterplattenfluorimeter mit den folgenden Messparametern:
Anregungswellenlänge 485 nm
Emissionswellenlänge 535 nm
Integrationszeit 40 µs
Anzahl Messungen 3
Verstärkung (gain) 82
z-Position 8800 µm

Ein weiterer möglicher Assay zur Bestimmung von Kinaseaktivität ist zum Beispiel in Bhat et al. (2003, JBC Paper, 278, 45937-45945) beschrieben.

### Affinitätschromatographie zur Aufreinigung von erfindungsgemäßer AK bzw. AKA

Eine Probe mit aufzureinigenden Fusionsproteinen, z. B. SEQ ID NO: 16, SEQ ID NO: 14 oder SEQ ID NO: 12 wird in einer geeigneten Bindepufferlösung, z. B. TRIS, HEPES oder HEPPS Puffer mit einem pH von etwa 6,5 bis etwa 8,5, der optional Salze wie NaCl und/oder Komplexbildner wie EDTA und/oder Reduktionsmittel wie DTT und/oder weitere Hilfssubstanzen zugesetzt sein können, mit einer stationären Phase in Kontakt gebracht. Dem Fachmann ist bekannt, dass je nach verwendetem Tag unterschiedliche Puffer mit unterschiedlichen Hilfssubstanzen sinnvoll sind. So wird z. B. bei einer Aufreinigung von Fusionsproteinen mit einem HisTag der Pufferlösung Imidazol zugesetzt. Bei einer Aufreinigung basierend auf einem MBPTag wird hingegen bevorzugt TRIS Puffer mit NaCl, EDTA und DTT eingesetzt. In einer Ausführungsform wird ein 10 bis 100 mM TRIS Puffer mit 100 bis 300 mM NaCl, 0,5 bis 2 mM EDTA und 0,5 bis 2 mM DTT eingesetzt. Stationäre Phasen für die Affinitätschromatographie sind weithin kommerziell erwerbbar wie z. B. MBPTrap (GE) zur Aufreinigung von MBP-Fusionsproteine oder Ni-NTA-Spin-Columns (Qiagen) zur Aufreinigung von HisTag-Fusionsproteinen oder GSTrap-Columns (GE) zur Aufreinigung von GST-Fusionsproteinen.

Die aufzureinigenden Fusionsproteine binden an die stationäre Phase, nicht spezifisch bindende Verunreinigungen werden durch einen Waschpuffer, der in einigen Fällen auch aus einem Überschuß an Bindepuffer bestehen kann, von der stationären Phase abgewaschen. Bei einer MBP-Affinitätschromatographie kann der Waschpuffer identisch mit dem Bindepuffer sein. Die aufgereingten Fusionsproteine werden anschließend mit einem Elutionspuffer, der einen geeigneten Eluant (z. B. Maltose bei MBPTags, Imidazol bei HisTag, Glutation bei GSTTag) enthält, von der stationären Phase abgelöst und, optional, umgepuffert.

### Beispiele

### Identifikation SEQ ID No: 2 und SEQ ID NO: 1

Mögliche Bindungsproteine an einen Kinaseinhibitor wurden mittels Affinitätschromatographie aus einem Zellextrakt aus *Ustilago maydis* isoliert. Als stationäre Phase wurde eine an NHS (N-Hydroxysuccinimid)-aktivierte Sepharose immobilisierte Verbindung 2 (ein Kinaseinhibitor, der unter die Offenbarung von WO 2008/107096 fällt) eingesetzt. Standard-Immobilisierungsreaktion sind dem Fachmann bekannt. Die Immobilisierung der Verbindung 2 kann z. B. nach Lolli et al. (2003, Proteomics, 3, 1287-1298) erfolgen.

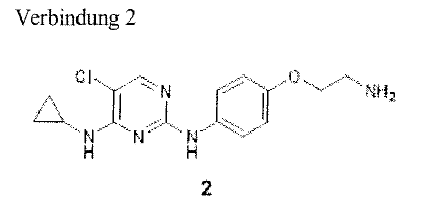

Zur Identifizierung möglicher Zielproteine für Verbindung 2 wurden 20g eines Zellpellets von Ustilago maydis in 50 ml Hepes-Lysepuffer 1 + 0.2 mM DTT + Complete Protease Inhibitor Cocktail Tablette (Roche Art. 11873580001) + PhosStop Phosphatase Inhibitor Tablette (Roche Art. 04906837001) resuspendiert und in einem Hochdruck-Homogenisator EmulsiFlex C 50 (5-10 Durchgänge) aufgeschlossen und zentrifugiert. 25 µl Überstand wurde mit NaCl auf eine Endkonzentration von 1.15 M NaCl eingestellt.

Inhibitormatrix wurde mit Hepes-Lysepuffer 1 mit 1 M NaCl äquilibriert (Matrix), 60 mg Lysat zu 25 µl Matrix zugegeben und 2 h bei 4°C inkubiert. Anschließend wurde der Überstand (=Durchfluss) abgenommen, die Matrix zweimal mit Hepes-Lysepuffer 1 mit 1M NaCl, einmal mit Hepes-Lysepuffer 2 gewaschen und anschließend potentielle Zielproteine mit 100 µl Elutionspuffer (Hepes-Lysepuffer 2 + 0.5 mM freier Inhibitor (Verbindung 2) + 2.5% DMSO + 10 mM ATP + 20 mM MgCl₂ eluiert.

| Hepes Lysepuffer 1 | Hepes-Lysepuffer 2 |
|---|---|
| 50 mM Hepes pH 7.5 | 20 mM Hepes pH 7.5 |
| 150 mM NaCl | 150 mM NaCl |
| 0.5% Triton X-100 | 0.25% Triton X-100 |
| 10% Glycerin | 1 mM EDTA |
| 1 mM EDTA | 1 mM EGTA |
| 10 mM Na₂P₂O₇ | |

Die Analyse der potentiellen Targetproteine erfolgte mittels nano HPLC/MS/MS. Dem Fachmann bekannte Verfahren sind z. B. in Knoth et al. (2009, Angewandte Chemie, 48, 7240-7245 + Supplementary) beschrieben. Die ermittelten Daten wurden zur Proteinidentifikation mittels der NCBInr-Datenbank (Version 20090127) analysiert.

Ein mögliches Targetprotein wurde als um10119 (SEQ ID NO: 2) identifiziert. Die so erhaltene Sequenz wurde in die *U. maydis* Datenbank des MIPS (Munich Information Center for Protein Sequences (© 2003 GSF - Forschungszentrum für Umwelt und Gesundheit, GmbH Ingolstädter Landstraße 1, D-85764 Neuherberg)) eingegeben. Nach MIPS ist um10119 eine potentielle IPL1 Ser/Thr Proteinkinase (MIPS/NCBI: um10119/XP_756618.1). Für die Nukleinsäuresequenz (SEQ ID NO: 1) von SEQ ID NO: 2 gibt es von MIPS und NCBI unterschiedliche Aussagen über das Vorhandensein eines Introns und über die sich daraus ergebende Gesamtlänge des Proteins. Das Protein, welches nach NCBI um10119 darstellt, ist laut MIPS ungültig und wurde durch eine andere, etwas kürzere Version ersetzt.

Die Sequenz der um10119 aus MIPS wurde in der Protein-Datenbank von NCBI geblastet. Das identifizierte Protein um10119 wurde auf Basis von hoher Sequenzhomologie zu anderen IPL1/Aurorakinasen, beispielsweise aus *Puccinia graminis* (Homolog: Aurora Proteinkinase, E-Value 3e-114, 65% Identität, 79% ähnliche Aminosäuren), *S. pombe* (Homolog: Aurora B / Ark1, E-Value 6e-99, 59% Identität, 77% ähnliche Aminosäuren) und Mensch (Homolog: Aurora A, E-Value 3e-89, 60% Identität, 75% ähnliche Aminosöuren) als Aurorakinase klassifiziert.

Es ist bekannt, dass Aurorakinasen vom Typ B mit INCENP (inneres Centromer-Protein) interagieren, welches auch für volle Aktivität *in vitro* benötigt wird. Dennoch sind Aurorakinasen (AK) aus *S*. *cerevisiae* als auch aus *S*. *pombe* auch ohne Anwesenheit des INCENP-Aktivators (AKA) aktiv (Leverson et al., 2002, Mol. Biol. Cell, 13, 1132-1143; Kang et al., 2001, J. Cell Biol., 155, 763-774).

### Expression und Aktivität von SEQ ID. NO: 2

### Klonieren von SEQ ID NO: 1

DNA aus *Ustilago maydis* wurde nach Standardmethoden isoliert (z. B. Hoffmann und Winston, 1987, Gene, 57, 267-272). Zur Auswahl von Oligonukleotiden für PCR-Reaktionen und zur Berechnung der Anlagerungstemperatur wurde DNAStar Lasergene PrimerSelect genutzt. Zu expremierende SEQ ID NO: 1 wurden mittels des Gateway®-Systems (Invitrogen) nach Herstellerangaben zunächst in pENTRTM/TEV/D-Topo® (Cat #45-0228, Lot #792341) kloniert.

Die folgenden PCR Bedingungen und Primer wurden verwendet:
1. 94°C-5min
2. 94°C - 30 sec
3. 56°C-10sec
4. 72°C-3min
5. 72°C-6min
Schritt 2 bis 4 wurde 33 mal wiederholt.

Primer für SEQ ID NO: 1:
AK forward (SEQ ID No: 7)
AK reverse (SEQ ID No: 9)

Korrekt sequenzierte Gene in pENTRTM/TEV/D-Topo wurden mittels homologer Rekombination in pDESTMC2 mit Hilfe des Gateway® LR Clonase® Enzym-Mixes (Invitrogen, Cat #11791019, Lot #1143296) nach Angaben des Herstellers kloniert. Daraus ergab sich der Expressionsvektor pDESTMC2_ SEQ ID NO: 11. SEQ ID NO 11 kodiert für ein Fusionsprotein aus SEQ ID NO: 1 und einem MBP-TAG.

PDESTMC2_ SEQ ID NO: 11 wurde in Expressionszellen OneShot® BL21(DE3)pLysE, chemisch kompetente *E. coli* Invitrogen (Cat # C656503, Carlsbad, CA, USA), nach Herstellerangaben transformiert.

### Expression und Reinigung von SEQ ID NO: 12 (MBP-AK-Fusionsprotein)

Aus einer Vorkultur aus BL12(DE3)pLysE mit MBP-Fusionsprotein wurde 11 LB-Flüssigmedium (+ 0,2%Glukose, 100 µg/ml Ampicillin, 34 µg/ml Chloramphenicol) angeimp. und bis zu einer OD600 von etwa 0,4 bei 37°C kultiviert. Danach wurde bei 16°C bis zu einer OD600 von etwa 0,7 weiter geschüttelt, worauf die Expression mit 0,4 mM IPTG induziert wurde. Die Expression erfolgte über 20 h bei 16°C.

Zur Aufarbeitung wurden die Zellen in 10ml/l Expressionskultur Puffer A + 87,5 U/ml DNAse I, 95 µg/ml Lysozym, 21 mM MgCl₂ und Complete Protease Inhibitor Cocktail (Roche Art. 11873580001) resuspendiert und durch Sonifikation aufgeschlossen. Das Lysat wurde abzentrifugiert und der Überstand filtriert.

Der so geklärte Überstand wurde mit 1 ml/min auf eine MBP Trap HP Affinitätssäule geladen. Anschließend wurde die Säule mit Puffer A gewaschen, bis kein Protein mehr durch UV-Messung detektiert werden konnte. Gebundenes Protein wurde in 0,75 ml Fraktionen mit Puffer B eluiert. Protein enthaltende Elutionsfraktionen wurden aufkonzentriert. Die konzentrierte Proteinlösung wurde auf eine Gelfiltrationssäule (HiLoad 16/60 Superdex 200, GEHealthcare Art. 17-1069-01) geladen und die Proteine mit Puffer A + 5% Glyzerin in 2 ml Fraktionen eluiert, um sie so weiter aufzureinigen. Elutionsfraktionen, die im Elutionsprofil ein singuläres Proteinsignal für monomeres Fusionsprotein ergaben, wurden vereinigt, auf etwa 1-2 ml aufkonzentriert, mit 20% Glyzerin (finale Konzentration) versetzt, aliquotiert, in flüssigem Stickstoff schockgefroren und bei -80°C gelagert.

| **Puffer A** | **Puffer B** |
|---|---|
| 20 mM Tris-Cl pH 7.5 | Puffer A + 10 mM Maltose |
| 200 mM NaCl | |
| 1 mM EDTA | |
| 1 mM DTT | |

### Aktivitätsbestimmung von SEQ ID NO: 12 ohne AKA

Die Aktivität von SEQ ID NO: 12 kann auf verschiedene Weisen wie unter allgemeine Methoden beschrieben, bestimmt werden. Es wurde der radioaktive Assay nach Müller wie unter allgemeine Methoden beschrieben durchgeführt.

Es konnte keine AK-Aktivität festgestellt werden. Weder das basische Myelinprotein (Substrat) noch HistonH3 (Subtrat; nicht gezeigt) wurden von SEQ ID NO: 12 phosphoryliert (siehe Abb. 1 A).

Assay 2: Die Aktivität der AK wurde mit allen drei 3 verschiedenen Substraten des kommerziell erhältlichen HTRF® Kinease Assays™ für Serin/Threonin-Kinasen (Cat #62ST0PEB. Cisbio Bioassays) gemessen. Keines der 3 Substrate wurde von AK phosphoryliert.

Assay 3: Keines der beiden kommerziell erhältlichen Substrate (Kemptide, Cat #R7331, Sequenz 5TAMRA-LRRASLG-OH und PKAtide, Cat #R7250, Lot #130188, Sequenz 5FAM-GRTGRRNSI-NH2) wurde von der AK phosphoryliert.

Daraufhin wurde ein radioaktiver Assay 1 nach Müller (2003, Eukaryot. Cell, 2, 1187-1199) mit der SEQ ID NO: 12 und der INCENP Sequenz aus *S*. *pombe* (Leverson et al., 2002, Mol. Biol. Cell, 13, 1132-1143) als auch mit dem humanen GST-INCENP (CAT#12-534, Millipore) durchgeführt

Es konnte ebenfalls keine Aurorakinaseaktivität festgestellt werden (siehe Abb. 2 C und 2 D).

### Identifikation SEQ ID No: 3 (AKA)

Obwohl Aktivitätstests von Proteinen mit der SEQ ID NO: 12 in Anwesenheit des INCENP-Aktivators aus *S*. *pombe* keine Aurorakinaseaktivität zeigten, wurde die Sequenz dieses Aktivators gegen das Genom von *U*. *maydis* mittels MIPS (MIPS Ustilago Maydis Datenbank) geblasted. Es wurde keine Treffer mit hohen Wahrscheinlichkeiten gefunden. Die Sequenz um03367 (vermutetes Protein) mit einen E-Wert von 2*10⁻¹⁴ zeigte lediglich in einem kleinen Bereich am C-Terminus eine Identität von 38% (Abb. 3).

### Klonieren von SEQ ID NO: 5

DNA aus *U. maydis* wurde nach Standardmethoden isoliert (z. B. Hoffman und Winston, 1987, Gene, 57, 267-272). Zur Auswahl von Oligonukleotiden für PCR-Reaktionen und zur Berechnung der Anlagerungstemperatur wurde DNAStar Lasergene PrimerSelect genutzt. Kloniert wurde die Nukleinsäuresequenz (SEQ ID NO: 5) entsprechend den Aminosäuren 1424 bis 1575 aus (SEQ ID NO: 6) mittels des Gateway®-Systems (Invitrogen) nach Herstellerangaben zunächst in pENTRTM/TEV/D-Topo® (Cat #45-0228, Lot #792341) kloniert.

Die folgenden PCR Bedingungen und Primer wurden verwendet:
1) 95°C - 4 min
2) 95°C - 30 sec
3) 58°C - 30 sec
4) 72°C - 1 min
5) 72°C - 10min
6) Schritt 7 bis 9 wurde 30 mal wiederholt.

Primer für SEQ ID NO: 5:
AKA forward (SEQ ID NO 8):
AKA reverse (SEQ ID NO 10):

Korrekt sequenzierte Gene in pENTRTM/TEV/D-Topo wurden mittels homologer Rekombination in pDESTMC2 mit Hilfe des Gateway® LR Clonase® Enzym-Mixes (Invitrogen, Cat #11791019, Lot #1143296) nach Angaben des Herstellers kloniert. Daraus ergab sich der Expressionsvektor pDESTMC2_ SEQ ID NO: 15.

PDESTMC2_ SEQ ID NO: 15 wurde in Expressionszellen OneShot® BL21(DE3)pLysE, chemisch kompetente *E. coli* Invitrogen (Cat # C656503, Carlsbad, CA, USA), nach Herstellerangaben transformiert.

### Expression und Aufreinigung von SEQ ID NO: 16 (MBP-AKA-Fusionsprotein)

Die Expression und Reinigung erfolgte analog zu der Aufreinigung von SEQ ID NO: 12 wie in den Paragraphen [0140] bis [0142] beschrieben.

### Expression und Co-Aufreinigung von SEQ ID NO: 14 und SEQ ID NO: 16

Die Expression der beiden Polypeptide erfolgte jeweils in OneShot® BL21 (DE3)pLysE- mit MBP-AK- Fusionsprotein bzw. in OneShot® BL21(DE3)pLysE- mit MBP-AKA-Fusionsprotein wie oben beschrieben. Vor der Lyse wurden die beiden Zellkulturen vereinigt und dann wie in Paragraphen [0141] und [0142] beschrieben aufgereinigt.

### Aurorakinaseaktivität von SEQ ID NO: 12 in Anwesenheit von SEQ ID NO: 16

Wie bereits in Abb. 1 B gezeigt, konnte keine AK-Aktivität von SEQ ID NO: 16 in Abwesenheit von SEQ ID NO: 12 festgestellt werden. Wie ebenfalls gezeigt (Abb. 1 A) zeigt auch SEQ ID NO: 12 keine AK-Aktivität in Abwesenheit von SEQ ID NO: 16. Auch die Anwesenheit von Pic1 aus *S*. *pombe* führte zu keiner AK-Aktivität von SEQ ID NO: 12.

Zum Start des Aktivitätstests wurden 2x Mastermix und Enzymlösung in 1X Kinasepuffer + 1 mM DTT im Verhältnis 1:1 gemischt. Die Messung erfolgte über 45 min. bei RT. Danach wurde der Ansatz mit dem gleichen Volumen SDS-Probepuffer versetzt, 5 min bei 95°C inkubiert. Die Auftrennung auf einem 4-12% Bis-Tris-Gel (Invitrogen) erfolgte für 35 min bei 200 V in 1X MES-Puffer. Das Gel wurde mit Coomassie gefärbt und 50 min gegen einen Röntgenfilm exponiert. Die Detektion der Signale erfolgte mit Hilfe eines Typhoon Trio 9500 Scanners (Amersham Biosciences).

Es konnte jedoch überraschend gezeigt werden, dass SEQ ID NO: 12 in Anwesenheit von SEQ ID NO: 16 AK-Aktivität aufweist. Es stellte sich zudem heraus, dass überraschend das Co-Aufreinigen der beiden Proteine zu einer höheren AK-Aktivität der SEQ ID NO: 12 führt als das isolierte Aufreinigen beider Proteine und spätere Zusammenführen dieser beiden Proteine (siehe Abb. 1 C und D und Abb. 2 B und A).

### Abspaltung des MBP-TAG von SEQ ID NO: 12 und SEQ ID NO: 16

Die Abspaltung des MBP-Tags erfolgte z.B. mittels der AcTEV-Protease (Invitrogen, Cat #12575-015) nach Angaben des Herstellers bei 4°C. Dies resultierte in SEQ ID NO: 2 und SEQ ID NO: 6.

### Aurorakinaseaktivität von SEQ ID NO: 2 in Anwesenheit von SEQ ID NO: 6

Zusätzlich konnte gezeigt werden, dass auch SEQ ID NO: 2 in Anwesenheit von SEQ ID NO: 6 (AK bzw AKA ohne TAG) AK-Aktivität aufweist (siehe Abb. 1 E).

### Aurorakinaseaktivität von SEQ ID NO: 12 in Anwesenheit von SEQ ID NO: 16

Der gereinigte Komplex aus MBP-SEQ ID NO: 12 und MBP-SEQ ID NO 16 war in der Lage, sowohl basisches Myelinprotein als auch die Substrate für humane Aurora Kinasen, PKAtide und Kemptide, in IMAP® Fluoreszenzpolarisations-Assays zu phosphorylieren (Abbildung 4).

Wenn nicht anders beschrieben, wurde die Aktivität der AK nach der IMAP Methode wie unter allgemeine Methoden beschrieben durchgeführt

Finale Enzymkonzentration: 2 ng/µl, finale ATP-Konzentration 35 µM, Reaktionsdauer 50 min.

Beide Substrate für humane Aurorakinase A wurden von SEQ ID NO: 12 in Anwesenheit von SEQ ID NO: 16 phosphoryliert (Siehe Abbildung 4).

### Inhibitor-Messung

Anhand von Staurosporin (Karaman et al., 2008, Nat. Biotechnol., 26, 127-132) und Verbindung 1

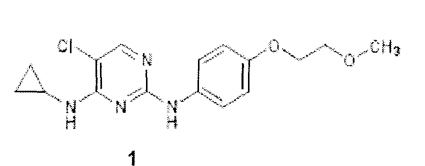

die genau wie Verbindung 2 unter die Offenbarung der WO 2008/107096 fällt, wurde überprüft, ob eine Inhibierung des Komplexes aus SEQ ID NO: 12 und SEQ ID NO: 16 auch durch andere Verbindungen als durch Verbindung 2 möglich ist. Die Versuche wurden mit dem IMAP Assay wie oben beschrieben durchgeführt, wobei zusätzlich Staurosporin bzw. Verbindung 1 zu der Reaktionslösung zugegeben wurde.

Der IC₅₀ von Staurosporin lag bei 2,1 nM und der IC₅₀ von Verbindung 1 lag bei 8,8 nM.

### ED₅₀-Bestimmung bei U. maydis

Der ED₅₀ Wert ist die Konzentration einer Verbindung, bei der das Wachstum von *U*. maydis in Flüssigkultur zu 50% gehemmt wird. Verbindungen für ED₅₀-Bestimmungen wurden in Methanol + 4 g/l Emulgator (z. B. PS 16 von Bayer AG) zu 666,67facher Endkonzentration gelöst. 15 µl jeder Verbindung werden in 7 Konzentrationen bei getrocknet. Flüssigkulturen von *U. maydis* in Difco Potato Dextrose Broth (BD, Cat No #254920) wurden auf eine OD₆₀₀ ~ 0,1 eingestellt und je 200 µl dieser Suspension zugegeben. Die Kulturen wurden 3 Tage bei 20°C, 600 Upm und 80% Luftfeuchte inkubiert. Anschließend wurde die OD bei 600 nm bestimmt und gegen die Inhibitor-Konzentration der Verbindung 1 aufgetragen. Als positive Kontrolle wurde Euparen (Dichlorfluarid; CAS 1085-98-9) verwendet, als negative Kontrolle Methanol + PS 16 ohne Inhibitor. Aus der gemessenen OD₆₀₀ wurde der ED₅₀-Wert bestimmt (Abb. 5). Der ED₅₀ Wert der Verbindung 1 wurde zu 21,8 ppm ermittelt, der ED₅₀ Wert von Dichlorfluarid wurde zu 1 ppm ermittelt.

## Patentansprüche

1. Verfahren zum Identifizieren von Fungiziden, umfassend
(a) In Kontakt Bringen einer Kombination aus einem Polypeptid mit der biologischen Aktivität einer Aurorakinase (AK) und einem Polypeptid mit der biologischen Funktion eines Aurorakinaseaktivators (AKA) mit einer chemischen Verbindung oder einem Gemisch von chemischen Verbindungen,
(b) Vergleichen der biologischen Aktivität der AK aus der Kombination aus (a) in Anwesenheit der chemischen Verbindung oder des Gemisches chemischer Verbindungen mit der biologischen Aktivität der AK aus der Kombination aus (a) in Abwesenheit der chemischen Verbindung oder des Gemisches von Verbindungen, und, optional,
(c) Bestimmen der chemischen Verbindung aus einem Gemisch, welche die biologische Aktivität der AK inhibiert;
wobei dieses Polypeptid mit der biologischen Aktivität einer AK
i) eine Aminosäuresequenz SEQ ID No: 2; oder ii) eine Aminosäuresequenz, welche mindestens 80 %, mindestens 85 %, mindestens 90 %, mindestens 95 % und insbesondere mindestens 98 % Identität mit der
Aminosäuresequenz SEQ ID No: 2 aufweist,
umfasst; und wobei ein Polypeptid mit der biologischen Funktion eines AKA
I) eine Aminosäuresequenz SEQ ID No: 6; oder
II) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 6 aufweist,
umfasst.

2. Verfahren gemäß Anspruch 1, in dem die fungizide Wirkung der in Schritt (c) bestimmten Verbindung überprüft wird, indem sie mit einem oder mehreren Pilzen in Kontakt gebracht wird.

3. Verfahren nach Anspruch 1 oder 2, in dem das Verhältnis von einem Polypeptid mit der biologischen Aktivität einer AK und einem Polypeptid mit der biologischen Funktion eines AKA zwischen 2:1 und 1:10, bevorzugt zwischen 2:1 und 1:5 und mehr bevorzugt zwischen 1:1 1 und 1:5, liegt.

4. Verfahren nach einem der Ansprüche 1 bis 3, wobei die Konzentration eines Polypeptids mit der biologischen Aktivität einer AK mindestens 0,5 ng/µl beträgt.

5. Verfahren nach einem der Ansprüche 1 bis 4, wobei das Polypeptid mit der biologischen Aktivität einer AK eine Aminosäuresequenz SEQ ID No: 12, oder eine Aminosäuresequenz, welche mindestens 80 %, mindestens 85 %, mindestens 90 %, mindestens 95 % und insbesondere mindestens 98% Identität mit einer Aminosäuresequenz SEQ ID No: 12 aufweist, umfasst.

6. Verfahren nach einem der Ansprüche 1 bis 5, wobei das Polypeptid mit der biologischen Funktion eines AKA eine Aminosäuresequenz SEQ ID No: 4, SEQ ID No: 14 oder SEQ ID No: 16, oder eine Aminosäuresequenz, welche mindestens 80 %, mindestens 85 %, mindestens 90 %, mindestens 95 % und insbesondere mindestens 98 % Identität mit einer Aminosäuresequenz SEQ ID No: 4, SEQ ID No: 14 oder SEQ ID No: 16 aufweist, umfasst.

7. Verwendung einer Kombination eines Polypeptids mit der biologischen Aktivität einer AK und eines Polypeptids mit der biologischen Funktion eines AKA zum Auffinden von fungiziden Verbindungen wobei das Polypeptid mit der biologischen Aktivität einer AK
i) eine Aminosäuresequenz SEQ ID No: 2; oder
ii) eine Aminosäuresequenz, welche mindestens 70%, mindestens 80 %, mindestens 85 %, mindestens 90 %, mindestens 95 % und insbesondere mindestens 98 % Identität mit der Aminosäuresequenz aus (i) aufweist,
umfasst; und
wobei das Polypeptid mit der biologischen Funktion eines AKA
I) eine Aminosäuresequenz SEQ ID No: 6; oder
II) eine Aminosäuresequenz, welche mindestens 60%, mindestens 70 %, mindestens 75 %, mindestens 80 %, mindestens 85 %, mindestens 90 %, mindestens 95 % und insbesondere mindestens 98 % Identität mit der Aminosäuresequenz SEQ ID No: 6 aufweist,
umfasst.

8. Nukleinsäure, die für einen AKA kodiert, wobei dieser AKA
I) eine Aminosäuresequenz SEQ ID No: 6; oder
II) eine Aminosäuresequenz, welche mindestens 60%, mindestens 70 %, mindestens 75 %, mindestens 80 %, mindestens 85 %, mindestens 90 %, mindestens 95 % und insbesondere mindestens 98 % Identität mit der Aminosäuresequenz SEQ ID No: 6 aufweist,
umfasst.

9. Die Nukleinsäure nach Anspruch 8, wobei der AKA eine Aminosäuresequenz SEQ ID No: 4, SEQ ID No: 14 oder SEQ ID No: 16, oder eine Aminosäuresequenz, welche mindestens 80 %, mindestens 85 %, mindestens 90 %, mindestens 95% und insbesondere mindestens 98 % Identität mit einer der Aminosäuresequenzen SEQ ID No: 4, SEQ ID No: 14 oder SEQ ID No: 16 aufweist, umfasst.

10. Nukleinsäure gemäß Anspruch 8 oder 9, umfassend eine Nukleotidsequenz ausgewählt aus
a) der Nukleotidsequenz gemäß SEQ ID No: 5;
b) einem Fragment bestehend aus mindestens 15 bis 90 aufeinanderfolgenden Nukleotiden der Nukleotidsequenz aus (a) codierend für ein Peptid oder mindestens 91 aufeinanderfolgenden Nukleotiden der Nukleotidsequenz aus (a) codierend für ein Polypeptid;
c) Nukleotidsequenzen, welche an die unter a) oder b) definierten Sequenzen bei einer Hybridisierungstemperatur von 20-65°C hybridisieren;
d) Nukleotidsequenzen, welche mindestens 70 %, mindestens 75 %, mindestens 80 %, mindestens 85 %, mindestens 90 %, mindestens 95 % und insbesondere mindestens 98 % Identität zu den unter a) bis c) definierten Sequenzen aufweisen, wobei die durch die Nukleinsäure kodierten Polypeptide AKA Funktion aufweisen;
e) Nukleotidsequenzen, welche zu den unter a) bis d) definierten Sequenzen komplementär sind; und
f) Nukleotidsequenzen, welche aufgrund der Degeneriertheit des genetischen Codes für dieselbe Aminosäuresequenz codieren wie die unter a) bis e) definierten Sequenzen.

11. Expressionskassette (DNA-Konstrukt) umfassend eine Nukleinsäure gemäß einem der Ansprüche 8 oder 10 und einen Promotor.

12. Vektor umfassend eine Nukleinsäure gemäß einem der Ansprüche 8 bis 10, oder eine Expressionskassette (DNA-Konstrukt) gemäß Anspruch 11.

13. Wirtszelle enthaltend eine Nukleinsäure gemäß einem der Ansprüche 8 bis 10, eine Expressionskassette (DNA-Konstrukt) gemäß Anspruch 11 oder einen Vektor gemäß Anspruch 12.

14. Polypeptid, welches durch die Nukleinsäure aus einem der Ansprüche 8 bis 10 codiert wird.

15. Polypeptid aus Anspruch 14, umfassend die SEQ ID No: 4, SEQ ID No: 6, SEQ ID No: 14 oder SEQ ID No: 16.

16. Kombination umfassend
a) ein gereinigtes Polypeptid mit der biologischen Funktion eines AKA, welches von einer Nukleinsäure gemäß einem der Ansprüche 7 bis 9 kodiert wird, und
b) ein gereinigtes Polypeptid mit der biologischen Aktivität einer AK, wobei die AK
i) eine Aminosäuresequenz SEQ ID No: 2 umfasst; oder
ii) eine Aminosäuresequenz, welche mindestens 70%, mindestens 80 %, mindestens 85 %, mindestens 90 %, mindestens 95 % und insbesondere mindestens 98 % Identität mit der Aminosäuresequenz ais i) aufweist, umfasst.

17. Kombination nach Anspruch 16, wobei der AKA eine Aminosäuresequenz ausgewählt aus
I) einer Aminosäuresequenz SEQ ID No: 6; oder
II) einer Aminosäuresequenz, welche mindestens 60%, mindestens 70 %, mindestens 75 %, mindestens 80 %, mindestens 85 %, mindestens 90 %, mindestens 95 % und insbesondere mindestens 98 % Identität mit der Aminosäuresequenz aus I) aufweist,
umfasst.

18. Antikörper, welcher spezifisch an eine AKA gemäß einem der Ansprüche 14 oder 15 bindet.

19. Verfahren zum Herstellen einer Kombination aus AK und AKA gemäß einem der Ansprüche 14 oder 15 umfassend
a) Exprimieren eines AKA von einer Nukleinsäure, die für das Polypeptid aus Anspruch 14 oder 15 kodiert;
b) Exprimieren einer AK von einer Nukleinsäure, die für eine wie in Anspruch 16 b) beschriebe AK kodiert; und
c) Gewinnung der AK- und AKA-Polypeptide; bevorzugt durch Aufreinigen, bevorzugt Co-Aufreinigen des AKA aus a) und der AK aus b).

20. Verfahren nach Anspruch 19, wobei die Nukleinsäuren aus a) und b) zusammen in einer Wirtszelle vorliegen.

21. Verfahren nach Anspruch 19, wobei die Nukleinsäuren aus a) und b) in separaten Wirtszellen vorliegen.

22. Verfahren nach Anspruch 21, wobei die AK und AKA exprimierenden Wirtszellen während der Expression in einer Mischung vorliegen.

23. Verfahren nach einem der Ansprüche 19, 21 oder 22, wobei die Gewinnung von AK und AKA aus zuvor vereinigten Wirtszellen erfolgt.

24. Verfahren gemäß einem der Ansprüche 1 bis 4, wobei die Kombination aus AK und AKA gemäß einem der Ansprüche 19 bis 23 hergestellt wird.

25. Kit umfassend ein Polypeptid nach Anspruch 14 oder 15 und ein Polypeptid mit der biologischen Aktivität einer AK umfassend
i) eine Aminosäuresequenz SEQ ID No: 2; oder
ii) eine Aminosäuresequenz, welche mindestens 80 %, mindestens 85 %, mindestens 90 %, mindestens 95 % und insbesondere mindestens 98 % Identität mit der Aminosäuresequenz SEQ ID No: 2 aufweist.

## Geänderte Patentansprüche

### Geänderte Patentansprüche gemäss Regel 137(2) EPÜ.

**1.** Verfahren zum Identifizieren von Fungiziden, umfassend
(a) In Kontakt Bringen einer Kombination aus einem Polypeptid mit der biologischen Aktivität einer Aurorakinase (AK) und einem Polypeptid mit der biologischen Funktion eines Aurorakinaseaktivators (AKA) mit einer chemischen Verbindung oder einem Gemisch von chemischen Verbindungen,
(b) Vergleichen der biologischen Aktivität der AK aus der Kombination aus (a) in Anwesenheit der chemischen Verbindung oder des Gemisches chemischer Verbindungen mit der biologischen Aktivität der AK aus der Kombination aus (a) in Abwesenheit der chemischen Verbindung oder des Gemisches von Verbindungen, und, optional,
(c) Bestimmen der chemischen Verbindung aus einem Gemisch, welche die biologische Aktivität der AK inhibiert;
wobei dieses Polypeptid mit der biologischen Aktivität einer AK
i) eine Aminosäuresequenz SEQ ID No: 2; oder
ii) eine Aminosäuresequenz, welche mindestens 80 % Identität mit der Aminosäuresequenz SEQ ID No: 2 aufweist,
umfasst; und wobei ein Polypeptid mit der biologischen Funktion eines AKA
I) eine Aminosäuresequenz SEQ ID No: 6; oder
II) eine Aminosäuresequenz, welche mindestens 80 % Identität mit der Aminosäuresequenz SEQ ID No: 6 aufweist,
umfasst.

**2.** Verfahren gemäß Anspruch 1, in dem die fungizide Wirkung der in Schritt (c) bestimmten Verbindung überprüft wird, indem sie mit einem oder mehreren Pilzen in Kontakt gebracht wird.

**3.** Verfahren nach Anspruch 1 oder 2, in dem das Verhältnis von einem Polypeptid mit der biologischen Aktivität einer AK und einem Polypeptid mit der biologischen Funktion eines AKA zwischen 2:1 und 1:10 liegt.

**4.** Verfahren nach einem der Ansprüche 1 bis 3, wobei die Konzentration eines Polypeptids mit der biologischen Aktivität einer AK mindestens 0,5 ng/µl beträgt.

**5.** Verfahren nach einem der Ansprüche 1 bis 4, wobei das Polypeptid mit der biologischen Aktivität einer AK eine Aminosäuresequenz SEQ ID No: 12, oder eine Aminosäuresequenz, welche mindestens 80 % Identität mit einer Aminosäuresequenz SEQ ID No: 12 aufweist, umfasst.

**6.** Verfahren nach einem der Ansprüche 1 bis 5, wobei das Polypeptid mit der biologischen Funktion eines AKA eine Aminosäuresequenz SEQ ID No: 4, SEQ ID No: 14 oder SEQ ID No: 16, oder eine Aminosäuresequenz, welche mindestens 80 % Identität mit einer Aminosäuresequenz SEQ ID No: 4, SEQ ID No: 14 oder SEQ ID No: 16 aufweist, umfasst.

**7.** Verwendung einer Kombination eines Polypeptids mit der biologischen Aktivität einer AK und eines Polypeptids mit der biologischen Funktion eines AKA zum Auffinden von fungiziden Verbindungen wobei das Polypeptid mit der biologischen Aktivität einer AK
i) eine Aminosäuresequenz SEQ ID No: 2; oder
ii) eine Aminosäuresequenz, welche mindestens 70% Identität mit der Aminosäuresequenz aus (i) aufweist,
umfasst; und
wobei das Polypeptid mit der biologischen Funktion eines AKA
I) eine Aminosäuresequenz SEQ ID No: 6; oder
II) eine Aminosäuresequenz, welche mindestens 60% Identität mit der Aminosäuresequenz SEQ ID No: 6 aufweist,
umfasst.

**8.** Kombination umfassend
a) ein gereinigtes Polypeptid mit der biologischen Funktion eines AKA, wobei dieses Polypeptid
I) eine Aminosäuresequenz SEQ ID No: 6; oder
II) eine Aminosäuresequenz, welche mindestens 60% Identität mit der Aminosäuresequenz aus I) aufweist,
umfasst und
b) ein gereinigtes Polypeptid mit der biologischen Aktivität einer AK, wobei dieses Polypeptid
i) eine Aminosäuresequenz SEQ ID No: 2; oder
ii) eine Aminosäuresequenz, welche mindestens 70% Identität mit der Aminosäuresequenz aus i) aufweist,
umfasst.

**9.** Kombination nach Anspruch 8, wobei die AKA eine Aminosäuresequenz SEQ ID No: 4, SEQ ID No: 14 oder SEQ ID No: 16, oder eine Aminosäuresequenz, welche mindestens 80 % Identität mit einer der Aminosäuresequenzen SEQ ID No: 4, SEQ ID No: 14 oder SEQ ID No: 16 aufweist, umfasst.

**10.** Antikörper, welcher spezifisch an einen AKA bindet, wobei dieser AKA eine Aminosäuresequenz SEQ ID NO: 6 oder einer Aminosäuresequenz, welche mindestens 60% Identität mit SEQ ID NO: 6 aufweist, umfasst, wobei dieser AKA eine Aminosäuresequenz umfasst, die ausgewählt ist aus SEQ ID No: 4, SEQ ID No: 14 oder SEQ ID No: 16 oder wobei dieser AKA durch eine Nukleinsäuresequenz kodiert ist, die ausgewählt ist aus
a) einer Nukleotidsequenz gemäß SEQ ID No: 5;
b) einem Fragment bestehend aus mindestens 15 bis 90 aufeinanderfolgenden Nukleotiden der Nukleotidsequenz aus (a) codierend für ein Peptid oder mindestens 91 aufeinanderfolgenden Nukleotiden der Nukleotidsequenz aus (a) codierend für ein Polypeptid;
c) Nukleotidsequenzen, welche an die unter a) oder b) definierten Sequenzen bei einer Hybridisierungstemperatur von 20-65°C hybridisieren;
d) Nukleotidsequenzen, welche mindestens 70 % Identität zu den unter a) bis c) definierten Sequenzen aufweisen, wobei die durch die Nukleinsäure kodierten Polypeptide AKA Funktion aufweisen;
e) Nukleotidsequenzen, welche zu den unter a) bis d) definierten Sequenzen komplementär sind; und
f) Nukleotidsequenzen, welche aufgrund der Degeneriertheit des genetischen Codes für dieselbe Aminosäuresequenz codieren wie die unter a) bis e) definierten Sequenzen.

**11.** Verfahren zum Herstellen einer Kombination aus AK und AKA gemäß einem der Ansprüche 8 oder 9 umfassend
a) Exprimieren eines AKA von einer Nukleinsäure, die für das Polypeptid wie in Anspruch 8a) oder 9 definiert kodiert;
b) Exprimieren einer AK von einer Nukleinsäure, die für eine wie in Anspruch 8b) definierte AK kodiert; und
c) Gewinnung der AK- und AKA-Polypeptide; bevorzugt durch Aufreinigen, bevorzugt Co-Aufreinigen des AKA aus a) und der AK aus b).

**12.** Verfahren nach Anspruch 11, wobei die Nukleinsäuren aus a) und b) zusammen in einer Wirtszelle vorliegen.

**13.** Verfahren nach Anspruch 11, wobei die Nukleinsäuren aus a) und b) in separaten Wirtszellen vorliegen.

**14.** Verfahren nach Anspruch 13, wobei die AK und AKA exprimierenden Wirtszellen während der Expression in einer Mischung vorliegen.

**15.** Verfahren nach einem der Ansprüche 11, 13 oder 14, wobei die Gewinnung von AK und AKA aus zuvor vereinigten Wirtszellen erfolgt.

**16.** Verfahren gemäß einem der Ansprüche 1 bis 4, wobei die Kombination aus AK und AKA gemäß einem der Ansprüche 11 bis 15 hergestellt wird.

**17.** Kit umfassend ein Polypeptid mit der biologischen Aktivität eines AKA umfassend
I) eine Aminosäuresequenz SEQ ID No: 6; oder
II) eine Aminosäuresequenz, welche mindestens 60% Identität mit der Aminosäuresequenz aus I) aufweist,
und ein Polypeptid mit der biologischen Aktivität einer AK umfassend
i) eine Aminosäuresequenz SEQ ID No: 2; oder
ii) eine Aminosäuresequenz, welche mindestens 80 % Identität mit der Aminosäuresequenz SEQ ID No: 2 aufweist.
